# EUROPEAN PATENT SPECIFICATION

(11) **EP 3 551 766 B1**
(45) Date of publication and mention of the grant of the patent: **14.09.2022**
(21) Application number: 17829381.7
(22) Date of filing: 11.12.2017
(51) Int. Cl.: C12Q 1/6804, G01N 33/53

(54) **INTEGRATED IMMUNO-PCR AND NUCLEIC ACID ANALYSIS IN AN AUTOMATED REACTION CARTRIDGE**
INTEGRIERTE IMMUNO-PCR UND NUKLEINSÄUREANALYSE IN EINER AUTOMATISIERTEN REAKTIONSKARTUSCHE
IMMUNO-PCR ET ANALYSE D'ACIDE NUCLÉIQUE CO-INTÉGRÉES DANS UNE CARTOUCHE DE RÉACTION AUTOMATISÉE

(30) Priority: 12.12.2016 US 201662433155 P
(43) Date of publication of application: 16.10.2019
(73) Proprietor: Cepheid, Sunnyvale, CA 94089 (US)
(72) Inventor: MCDOWELL-BUCHANAN, Carla Maria, Sunnyvale, California 94089 (US); CLEMENS, Daniel, Sunnyvale, California 94089 (US)
(74) Representative: Patent Boutique LLP
(86) International application number: PCT/US2017/065653
(87) International publication number: WO 2018/111782

(56) References cited:
- WO-A1-2015/175856
- WO-A2-2006/121997
- WO-A2-2008/012550
- CN-A- 102 994 638
- JP-A- 2012 255 664

## Description

### BACKGROUND

The development of a reliable, sensitive, specific and rapid diagnostic test is a major challenge for determining effective treatment of infectious diseases and other pathologies. PCR has become one of the most popular methods for the direct detection of nucleic acids from an infectious agent or characteristic of another pathology *(e.g.,* a cancer). The very high sensitivity of PCR, which is capable of detecting a single molecule of DNA makes it an excellent choice for diagnostic purposes. However, in some cases, the protein target is expressed at higher copy number than the nucleic acid.

Immunoassays have been used for the quantification of proteins since 1960 *(see, e.g.,* Lequin (2005) Clin. Chem. 51: 2415-2418; Brechot et al. (1985) N. Engl. J. Med. 312: 270-276; and the like) and have become a versatile and powerful tool for diagnostic methods. Immunoassays allow a diagnostic method to directly detect target proteins (e.g., the proteins of pathogens) and other analytes, and also indirectly detect antibodies produced against microorganisms or other immunogens. The enzyme-linked immunosorbent assay (ELISA) is a commonly used technique to detect antibodies or antigens in samples using the reaction of antibodies to their antigens (Lequin (2005) Clin. Chem. 51: 2415-2418). ELISA combines the specificity of anti- bodies with the sensitivity of simple enzyme assays and antibodies are coupled to an easily assayed enzyme. Despite its effectiveness and its specificity, ELISA is unable to detect some antigens, particularly when they are present at low concentrations, as can be the case, for example, with antigens of hepatitis B virus (HBV) (Brechot et al. (1985) N. Engl. J. Med. 312: 270-276), which seems to use the low level of transcription of its genes as a persistence mechanism in the host.

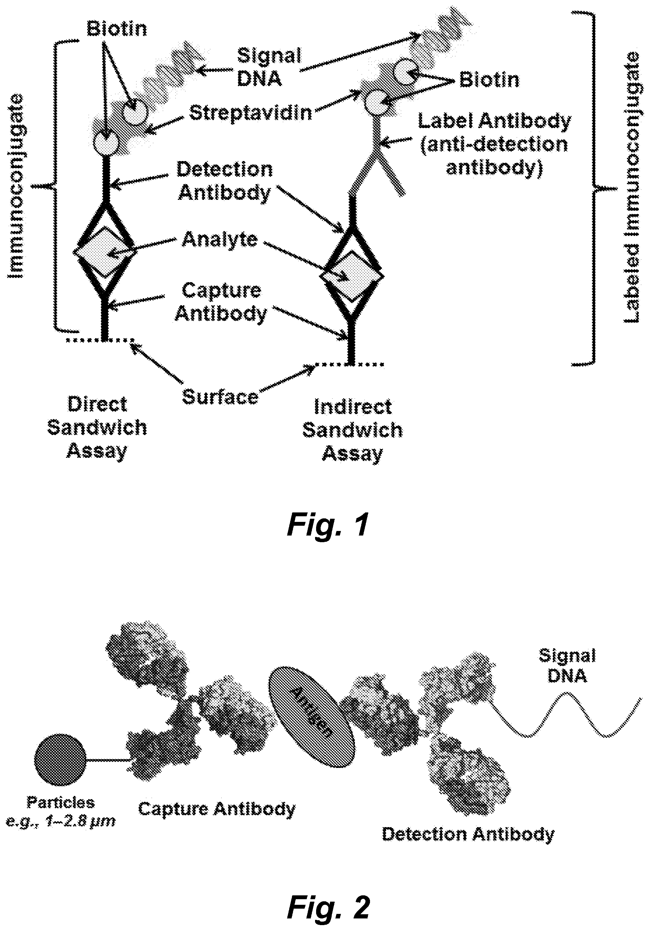

Immuno-PCR (I-PCR) combines the versatility of ELISA with the exponential amplification power and sensitivity of PCR, thus leading to an increase in sensitivity compared with an analogous ELISA. Immuno-PCR basically similar to ELISA (Fig. 1a), which detects an antigen-antibody reaction, but instead of using an enzyme-conjugated antibody, the antibody is labelled with a DNA fragment, which can be amplified by PCR.

Using immuno-PCR, a 100-10 000-fold improvement over the detection limit of the ELISA has routinely been obtained (Brechot et al. (1985) N. Engl. J. Med. 312: 270-276). Despite this gain of sensitivity, for a long time the number of immuno-PCR studies were low and the majority of applications were based on research questions with demanding protocols. The requirement for experimental expertise in both ELISA and PCR, the complexity of protocols, and the high degree of labor required, has limited the use of immuno-PCR for diagnostic purposes. WO 2006/121997 A2 describes a self-contained pouch to perform immuno-PCR in a closed environment. JP 2012/255664 A describes immuno-PCR in a closed environment.

### SUMMARY

In a first aspect the present invention provides a cartridge as defined in claim 1. In a second aspect the present invention provides a method as defined in claim 7. In a third aspect the present invention provides a method as defined in claim 8. In a fourth aspect the present invention provides a system as defined in claim 13. In a fifth aspect the present invention provides a kit as defined in claim 14. Further features are defined in the dependent claims.

### BRIEF DESCRIPTION OF THE DRAWINGS

Figure 1 illustrates a direct Sandwich Assay (left) and an indirect sandwich assay (right). The surface can be, *inter alia,* the surface of a particle, the surface of a chamber or channel wall or floor, or a surface of a matrix material *(e.g.,* a filter).
Figure 2 illustrates a direct sandwich immunocomplex with the capture antibody attached to a particle.
Figure 3 shows some illustrative, but non-limiting immuno-PCR methods as implemented in a cartridge,

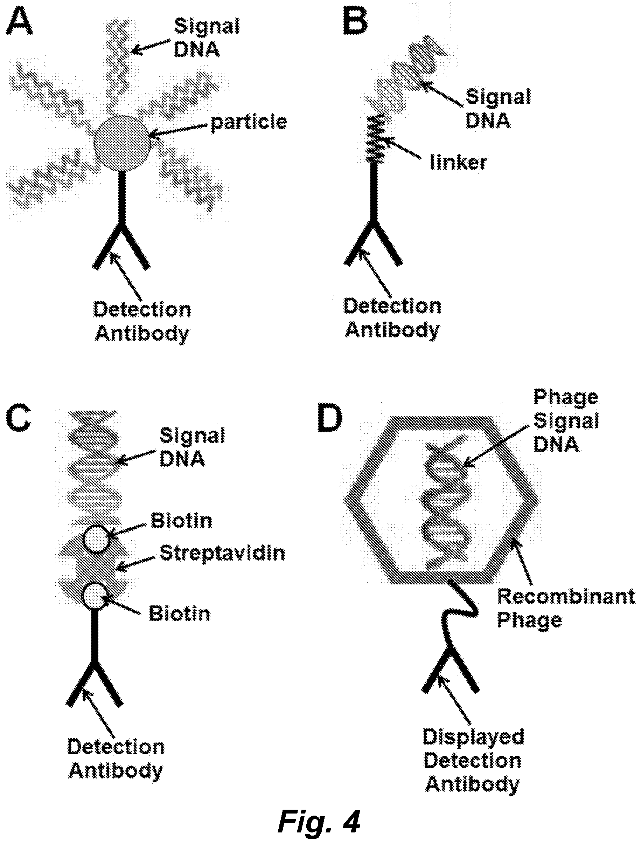

Figure 4, panels A-D, illustrates various schemes for coupling a signal DNA to an antibody. Panel A shows a signal nucleic chemically conjugated to a detection antibody via a linker. Panel B shows a signal DNA attached to a detection antibody via an avidin/biotin linkage. Panel C shows a signal DNA attached to a particle to which is also attached a detection antibody. Panel D illustrates a detection antibody displayed on a phase with the signal DNA contained within the phage. It will be recognized that in an indirect assay the detection antibody in any of these embodiments can be replaced with a label antibody that binds to a detection antibody.
Figure 5A illustrates major components of a cartridge *(e.g.,* a GENEXPERT^{®} cartridge) suitable for use with the methods described herein. Fig. 5B shows a top view of the cartridge illustrating chambers disposed around a central valve.
Figure 6A shows one illustrative, but non-limiting, implementation of a GENEXPERRT^{®} cartridge configured for immuno-PCR, while Figure 6B shows one illustrative, but non-limiting, implementation of a GENEXPERRT^{®} cartridge configured for immuno-PCR and nucleic acid analysis.
Figure 7, panels A-C, illustrates one embodiment of a GENEXPERT^{®} cartridge suitable for immuno-PCR and optional integrated nucleic acid analysis as described herein.
Figures 8A-8C show illustrative, but non-limiting embodiments of the modules, and systems (e.g., processing units) for the immuno-PCR detection and/or quantification of polypeptide(s) and optional integrated nucleic acid analysis. Fig. 8A illustrates a module for operation of a GENEXPERT^{®} cartridge. Fig. 8B illustrates some components of one embodiment of a module for operation of a cartridge for immuno-PCR and, optionally, nucleic acid analysis. Fig. 8C illustrates a system (e.g., processing unit) incorporating a plurality of modules.
Figures 9 illustrates an immunocomplex comprising a capture antibody bound to a surface (e.g., the surface of a bead) where the capture antibody has bound an antigen (analyte) which, in turn, is bound by a detection antibody. The detection antibody is attached to a single DNA via a linker and the detection antibody is bound to FAM which can be bound by an anti-FITC alkaline phosphatase conjugate.

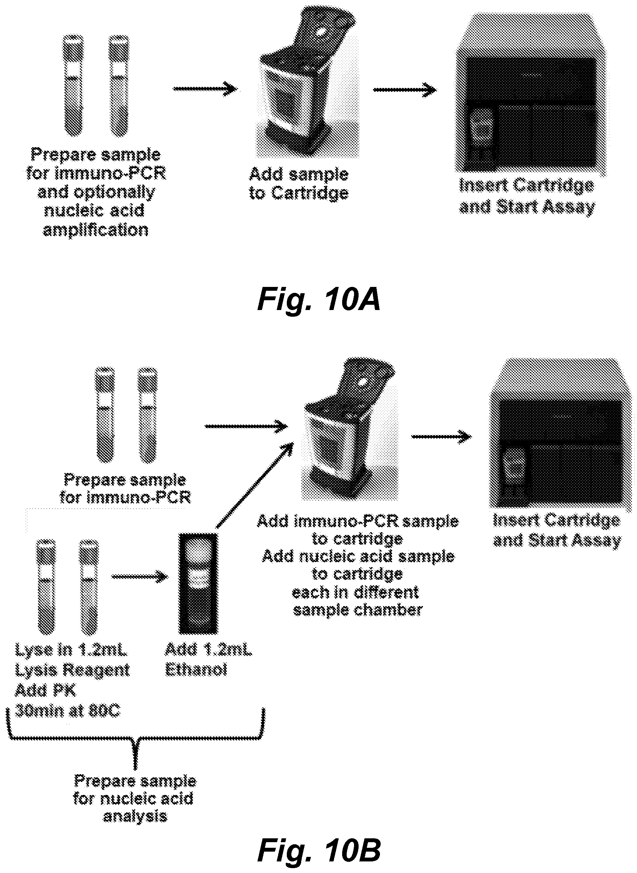

Figures 10A and 10B show illustrative but non-limiting workflows for immuno-PCR and optional nucleic acid analysis. In certain embodiments immuno-PCR and nucleic acid analysis when performed, are both performed on the same sample. Thus a single sample can be introduced into one sample chamber (Fig. 10A). In other embodiments the sample may be processed differently for immuno-PCR and nucleic acid analysis. In such instances the immuno-PCR sample can be introduced into one sample chamber and the nucleic acid analysis sample can be introduced into another sample chamber in the same cartridge (Fig. 10B). The nucleic acid preparation shown is illustrative and not limiting.
Figure 11 is a schematic representation of an automated immuno-PCR assay using the GeneXpert^{®} Cartridge, which is described in Example 2.
Figure 12 shows the cartridge chamber (CH) assignments, reagents, and initial volumes for the automated immuno-PCR assay using the GeneXpert^{®} Cartridge described in Example 2.
Figure 13 is a dose-response curve for detection of human IL-8, as described in Example 2.
Figure 14 is a dose-response curve for detection of C. *difficile,* as described in Example 2.
Figure 15A-15B is a dose-response curve for (A) manual and (B) automated *C. difficile* Toxin B immune-PCR formats (see Example 2).

### DEFINITIONS

To facilitate an understanding of the present invention, a number of terms and phrases are defined below:
As used herein, the terms "detect", "detecting" or "detection" may describe either the general act of discovering or discerning or the specific observation of a detectably labeled composition.
As used herein, the term "detectably different" or "spectrally distinguishable" refers to a set of labels (such as dyes/fluorophores) that can be detected and distinguished simultaneously.
As used herein, the terms "patient" and "subject" are typically used interchangeably to refer to a human. In some embodiments, the methods described herein may be used on samples from non-human animals, e.g., a non-human primate, canine, equine, feline, porcine, bovine, lagomorph, and the like. Additionally the term patient may be used for non-human animals in the veterinary context.

The terms "polypeptide", "peptide" and "protein" are used interchangeably herein to refer to a polymer of amino acid residues. In certain embodiments the polypeptide is at least 2 amino acids in length, or at least 4 amino acids in length, or at least 4 amino acids in length, or at least 6 amino acids in length, or at least 8 amino acids in length, or at least 10 amino acids in length, or at least 15 amino acids in length, or at least 20 amino acids in length, or at least 25 amino acids in length, or at least 30 amino acids in length, or longer. The terms apply to amino acid polymers in which one or more amino acid residue is an artificial chemical analogue of a corresponding naturally occurring amino acid, as well as to naturally occurring amino acid polymers. The term also includes variants on the traditional peptide linkage joining the amino acids making up the polypeptide. Preferred "peptides/polypeptides/proteins" are chains of amino acids whose α carbons are linked through peptide bonds. The terminal amino acid at one end of the chain (amino terminal) therefore has a free amino group, while the terminal amino acid at the other end of the chain (carboxy terminal) has a free carboxyl group. As used herein, the term "amino terminus" (abbreviated N-terminus) refers to the free α-amino group on an amino acid at the amino terminal of a protein or to the α-amino group (imino group when participating in a peptide bond) of an amino acid at any other location within the protein. Similarly, the term "carboxy terminus" refers to the free carboxyl group on the carboxy terminus of a protein or the carboxyl group of an amino acid at any other location within the protein. Proteins also include essentially any polyamino acid including, but not limited to peptide mimetics such as amino acids joined by an ether as opposed to an amide bond. Typically any of the protein sequences provided herein comprise all "L" amino acids. However, in certain embodiments, any of the protein sequences provided herein can comprise a combination of "L" and "D" amino acids. In certain embodiments any of the protein sequences described herein comprise all "D" amino acids thereby providing the D-enantiomer or inverso form of the protein. In certain embodiments any of the protein sequences described herein comprise a retro-protein in which the amino acids are all "L" amino acids, but in a reverse order. In certain embodiments any of the protein sequences described herein comprise a retro-inverso protein composed of all "D" amino acids in a reverse order.

As used herein, the terms "oligonucleotide," "polynucleotide," "nucleic acid molecule," and the like, refer to nucleic acid-containing molecules, including but not limited to, DNA. The terms encompass sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine. In certain embodiments the term "oligonucleotide," refers to a single-stranded polynucleotide typically having fewer than 500 nucleotides. In some embodiments, an oligonucleotide is at least 6 nt, or at least 8 nt, or at least about 10 nt, or at least about 12 nt, or at least about 15nt, up to about 200 nt, or up to about 100 nt, or up to about 50 nt, or up to about 30 nt, or up to about 25 nt. Oligonucleotides may be referred to by their length, for example, a 24 residue oligonucleotide may be referred to as a "24-mer."

As used herein, the term "complementary" to a target gene (or target region thereof), and the percentage of "complementarity" of the probe sequence to the target gene sequence is the percentage "identity" to the sequence of target gene or to the complement of the sequence of the target gene. In determining the degree of "complementarity" between probes used in the compositions described herein (or regions thereof) and a target gene, such as those disclosed herein, the degree of "complementarity" is expressed as the percentage identity between the sequence of the probe (or region thereof) and sequence of the target gene or the complement of the sequence of the target gene that best aligns therewith. The percentage is calculated by counting the number of aligned bases that are identical as between the 2 sequences, dividing by the total number of contiguous nucleotides in the probe, and multiplying by 100. When the term "complementary" is used, the subject oligonucleotide is at least 90% complementary to the target molecule, unless indicated otherwise. In some embodiments, the subject oligonucleotide is at least 91%, at least 92%, at least 93%, at least 94%, at least 95%, at least 96%, at least 97%, at least 98%, at least 99%, or 100% complementary to the target molecule.

The term "primer" refers to an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. In various embodiments primers typically have a length in the range of from about 14 to about 40 nucleotides, or in the range of from about 18 to about 36, or to about 30, or to about 25 nucleotides. In certain embodiments the term "primer" refers to an oligonucleotide that comprises a region that is complementary to a sequence of at least 8 contiguous nucleotides of a target nucleic acid molecule, such as a target gene, a signal DNA, and the like. In some embodiments, a primer or probe comprises a region that is complementary to a sequence of at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 29, at least 30, at least 319, at least 32, at least 33, at least 34, at least 35, at least 36, at least 37, at least 38, at least 39, or at least 40 contiguous nucleotides of a target molecule. When a primer or probe comprises a region that is "complementary to at least x contiguous nucleotides of a target molecule," the primer or probe is at least 95% complementary to at least x contiguous nucleotides of the target molecule. In some embodiments, the primer or probe is at least 96%, at least 97%, at least 98%, at least 99%, or 100% complementary to the target molecule. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references: Dieffenbach, editor, PCR Primer: A Laboratory Manual, 2nd Edition (Cold Spring Harbor Press, New York, 2003).

The term "nucleic acid amplification," encompasses any means by which at least a part of at least one target nucleic acid is reproduced, typically in a template-dependent manner, including without limitation, a broad range of techniques for amplifying nucleic acid sequences, either linearly or exponentially. Exemplary means for performing an amplifying step include polymerase chain reaction (PCR), ligase chain reaction (LCR), ligase detection reaction (LDR), multiplex ligation-dependent probe amplification (MLPA), ligation followed by Q-replicase amplification, primer extension, strand displacement amplification (SDA), hyperbranched strand displacement amplification, multiple displacement amplification (MDA), nucleic acid strand-based amplification (NASBA), two-step multiplexed amplifications, rolling circle amplification (RCA), and the like, including multiplex versions and combinations thereof, for example but not limited to, OLA/PCR, PCR/OLA, LDR/PCR, PCR/PCR/LDR, PCR/LDR, LCR/PCR, PCR/LCR (also known as combined chain reaction -- CCR), digital amplification, and the like. Descriptions of such techniques can be found in, among other sources, Ausbel et al. (1995) PCR Primer: A Laboratory Manual, Diffenbach, Ed., Cold Spring Harbor Press; Msuih et al. (1996) J. Clin. Micro. 34: 501-07; Rapley, ed. (2002) The Nucleic Acid Protocols Handbook, Humana Press, Totowa, N.J.; Abramson et al. (1993) Curr. Opin. Biotechnol. 4(1):41-47; Day et al. (1995) Genomics, 29(1): 152-162; Ehrlich et al.(1991) Science, 252: 1643-1650; Innis et al.(1990) PCR Protocols: A Guide to Methods and Applications, Academic Press; Favis et al. (2000) Nat. Biotechnol., 18: 561-564; Rabenau et al.(2000) Infection, 28: 97-102; Belgrader, Barany, and Lubin, Development of a Multiplex Ligation Detection Reaction DNA Typing Assay, Sixth International Symposium on Human Identification, 1995 (available on the world wide web at: promega.com/geneticidproc/ussymp6proc/blegrad.html); LCR Kit Instruction Manual, Cat. #200520, Rev. #050002, Stratagene, 2002; Barany et al. (1991) Proc. Natl. Acad. Sci. USA, 88: 188-193; Bi and Sambrook (1997) Nucl. Acids Res. 25: 2924-2951; Zirvi et al. (1999) Nucl. AcidRes. 27: e40i-viii; Dean et al. (2002) Proc. Natl. Acad. Sci. USA, 99: 5261-5266; Barany and Gelfand (1991) Gene, 109: 1-11; Walker et al. (1992) Nucl. AcidRes. 20: 1691-1696; Polstra et al. (2002) BMC Inf. Dis. 2: 18; Lage et al.(2003) Genome Res. 13(2): 294-307; Landegren et al. (1988) Science, 241: 1077-1080; Demidov (2002) Expert Rev. Mol. Diagn. 2(6): 542-548; Cook et a/.(2003) J. Microbiol. Meth. 53(2): 165-174, Schweitzer et al. (2001) Curr. Opin. Biotechnol. 12(1): 21-27; U.S. Patent Nos: 6,027,998, 5,830,711, 6,027,889, 5,686,243, and 6,605,451; PCT Publication Nos. WO 97/31256, WO 01/92579, WO/0056927A3, and WO/9803673A1; and the like.

In some embodiments, amplification comprises at least one cycle of the sequential procedures of: annealing at least one primer with complementary or substantially complementary sequences in at least one target nucleic acid; synthesizing at least one strand of nucleotides in a template-dependent manner using a polymerase; and denaturing the newly-formed nucleic acid duplex to separate the strands. The cycle may or may not be repeated. Amplification can comprise thermocycling or, in certain embodiments, can be performed isothermally.

The term "hybridize" is typically used herein refer to "specific hybridization" which is the binding, duplexing, or hybridizing of a nucleic acid molecule preferentially to a particular nucleotide sequence, in some embodiments, under stringent conditions. The term "stringent conditions" refers to conditions under which a probe will hybridize preferentially to its target sequence, and to a lesser extent to, or not at all to, other sequences. A "stringent hybridization" and "stringent hybridization wash conditions" in the context of nucleic acid hybridization are sequence-dependent and are different under different environmental parameters. An extensive guide to the hybridization of nucleic acids is found in, *e.g.,* Tijssen (1993) Laboratory Techniques in Biochemistry and Molecular BiologyHybridization with Nucleic Acid Probes part I, Ch. 2, "Overview of principles of hybridization and the strategy of nucleic acid probe assays," Elsevier, N.Y. ("Tijssen"). Generally, highly stringent hybridization and wash conditions for filter hybridizations are selected to be about 5°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. In certain embodiments very stringent conditions are selected to be equal to the Tₘ for a particular probe. Dependency of hybridization stringency on buffer composition, temperature, and probe length are well known to those of skill in the art (see, *e.g.,* Sambrook and Russell (2001) Molecular Cloning: A Laboratory Manual (3rd ed.) Vol. 1-3, Cold Spring Harbor Laboratory, Cold Spring Harbor Press, NY).

As used herein, an "antibody" refers to a protein consisting of one or more polypeptides substantially encoded by immunoglobulin genes or fragments of immunoglobulin genes from humans or other species or polypeptides derived therefrom that can bind a target molecule *(e.g.,* an antigen). The recognized human immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist as intact immunoglobulins or as a number of well characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region thereby converting the (Fab')₂ dimer into a Fab' monomer. The Fab' monomer is essentially a Fab with part of the hinge region *(see,* Fundamental Immunology, W.E. Paul, ed., Raven Press, N.Y. (1993), for a more detailed description of other antibody fragments). While various antibody fragments are defined in terms of the digestion of an intact antibody, one of skill will appreciate that such Fab' fragments may be synthesized *de novo* either chemically or by utilizing recombinant DNA methodology. Thus, the term antibody, as used herein also includes antibody fragments either produced by the modification of whole antibodies or synthesized *de novo* using recombinant DNA methodologies. Certain preferred antibodies include single chain antibodies (antibodies that exist as a single polypeptide chain), more preferably single chain Fv antibodies (sFv or scFv) in which a variable heavy and a variable light chain are joined together (directly or through a peptide linker) to form a continuous polypeptide. The single chain Fv antibody is a covalently linked V_{H}-V_{L} heterodimer which may be expressed from a nucleic acid including V_{H}- and V_{L}- encoding sequences either joined directly or joined by a peptide-encoding linker. Huston, et al. (1988) Proc. Nat. Acad. Sci. USA, 85: 5879-5883. While the V_{H} and V_{L} are connected to each as a single polypeptide chain, the V_{H} and V_{L} domains associate non-covalently. The first functional antibody molecules to be expressed on the surface of filamentous phage were single-chain Fv's (scFv), however, alternative expression strategies have also been successful. For example Fab molecules can be displayed on phage if one of the chains (heavy or light) is fused to g3 capsid protein and the complementary chain exported to the periplasm as a soluble molecule. The two chains can be encoded on the same or on different replicons; the important point is that the two antibody chains in each Fab molecule assemble post-translationally and the dimer is incorporated into the phage particle via linkage of one of the chains to, *e.g.,* g3p *(see, e.g.,* U.S. Patent No: 5,733,743). The scFv antibodies and a number of other structures converting the naturally aggregated, but chemically separated light and heavy polypeptide chains from an antibody V region into a molecule that folds into a three dimensional structure substantially similar to the structure of an antigen-binding site are known to those of skill in the art (*see e.g.,* U.S. Patent Nos. 5,091,513, 5,132,405, and 4,956,778). Particularly preferred antibodies should include all that have been displayed on phage (*e.g.,* scFv, Fv, Fab and disulfide linked Fv *(see, e.g.,* Reiter et al. (1995) Protein Eng. 8: 1323-1331). Antibodies as used herein also include, but are not limited to nanobodies, *e.g.,* camelid antibodies *(see, e.g.,* Harmsen and Haard (2007) Appl. Microbiol. Biotechnol. 77 (1): 13-22; Moller et al. (2010) J. Biol. Chem. 285(49): 38348-38361; Dolk et al. (2005) Appl. Environ. Microbiol. 71(1): 442-450; Stanfield et al. (2004) Science, 305(5691): 1770-1773; Desmyter et al. (1996) Nat. Struct. Biol. 3(9): 803-811; and the like), unibodies *(see, e.g.,* Kolfschoten et al. (2007) Science 317: 1554-1557; PCT Pub. No: WO2007/059782; and the like), affibodies *(see, e.g.,* Nord et al. (1997) Nat. Biotechnol. 15: 772-777; Ronmark et al. (2002) Eur. J. Biochem., 269: 2647-2655; U.S. Patent No: 5,831,012; and the like).

The phrase "specifically binds" indicates that the molecule binds preferentially to the target of interest or binds with greater affinity to the target (analyte) than to other molecules. For example, an antibody will selectively bind to the antigen against which it was raised. A DNA molecule will bind to a substantially complementary sequence and not to unrelated sequences under stringent conditions. Specific binding can refer to a binding reaction that is determinative of the presence of a target in a heterogeneous population of molecules (e.g., proteins and other biologics). Thus, under designated conditions (e.g., immunoassay conditions in the case of an antibody or stringent hybridization conditions in the case of a nucleic acid), the specific ligand or antibody binds to its particular "target" molecule and does not bind in a significant amount to other molecules present in the sample. Thus, in various embodiments "specific" or "specificity" in reference to the binding of one molecule to another molecule, such as a target sequence for a probe, means the recognition, contact, and formation of a stable complex between the two molecules, together with substantially less recognition, contact, or complex formation of that molecule with other molecules. In one aspect, "specific" in reference to the binding of a first molecule to a second molecule means that to the extent the first molecule recognizes and forms a complex with another molecule in a reaction or sample, it forms the largest number of the complexes with the second molecule. Preferably, this largest number is at least fifty percent, or at least 60%, or at least 70%, or at least 80%, or at least 90%,, or at least 95% of the complexes formed by the either member of the complex. Generally, molecules involved in a specific binding event have areas on their surfaces or in cavities giving rise to specific recognition between the molecules binding to each other. Examples of specific binding include antibody-antigen interactions, enzyme-substrate interactions, formation of duplexes or triplexes among polynucleotides and/or oligonucleotides, receptor-ligand interactions, and the like. As used herein, "contact" in reference to specificity or specific binding means two molecules are close enough that weak noncovalent chemical interactions, such as Van der Waal forces, hydrogen bonding, base-stacking interactions, ionic and hydrophobic interactions, and the like, dominate the interaction of the molecules.

The term "sample" refers to a quantity of material from a biological, environmental, medical, or patient source in which detection or measurement of target analyte(s) *(e.g.,* polypeptides, nucleic acids, *etc.)* is sought. On the one hand the term "sample" includes a specimen or culture (e.g., microbiological cultures). On the other hand, the term sample includes both biological and environmental samples. A sample may include a specimen of synthetic origin. Biological samples may be animal, including human, fluid, solid *(e.g.,* stool), cells, or tissue, as well as liquid and solid food and feed products and ingredients such as dairy items, vegetables, meat and meat by-products, and waste. Biological samples may include materials taken from a subject *(e.g.,* a patient) including, but not limited to cultures, plasma, serum, blood, saliva, amniotic fluid, mucus, urine, pancreatic juice, cerebral spinal fluid, pleural fluid, milk, lymph, sputum, semen, skin biopsies, bronchial and/or tracheal aspirates, needle aspirates, punch biopsies, cryopreserved sections, FFPE sections, and the like. Biological samples may be obtained from human and all of the various families of domestic animals, as well as feral or wild animals, including, but not limited to, such animals as ungulates, bear, fish, rodents, lagomorphs, *etc.* Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention. The terms "sample" and "specimen" are used interchangeably.

The term "analyte" refers to any moiety that is to be detected and/or quantified. Analytes include, but are not limited to particular biomolecules (proteins, antibodies, nucleic acids *(e.g.,* DNA and/or RNA), carbohydrates, lectins, *etc.),* bacteria or components thereof, bacterial toxins or components thereof, viruses or components thereof *(e.g.,* coat proteins), fungi or components thereof, fungal toxins or components thereof, protozoa or components thereof, protozoal toxins or components thereof, drugs, other toxins, food pathogens, and the like.

The terms "polymerase chain reaction," or "PCR," refer to a reaction for the *in vitro* amplification of specific DNA sequences by the simultaneous primer extension of complementary strands of DNA. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors well-known to those of ordinary skill in the art *(see, e.g.,* McPherson et al. eds (1995) PCR: A Practical Approach, 2nd Ed., IRL Press, Oxford; and the like). For example, in a conventional PCR using Taq DNA polymerase, a double stranded target nucleic acid may be denatured at a temperature greater than about 90°C °, primers annealed at a temperature in the range of about 50°C to about 75°C, and primers extended at a temperature in the range of about 72°C to about 78°C. The term "PCR" encompasses derivative forms of the reaction, including but not limited to, RT-PCR, real-time PCR, nested PCR, quantitative PCR, multiplexed PCR, and the like. In various embodiments PCR reaction volumes can range from a few hundred nanoliters, e.g. 200 nL, to a few hundred µL, *e.g.* ~200 µL.

The term "reverse transcription PCR," or "RT-PCR," refers to a PCR that is preceded by a reverse transcription reaction that converts a target RNA to a complementary single stranded DNA, which is then amplified *(see, e.g.,* U.S. Pat. No. 5,168,038).

The term "real-time PCR" refers to a PCR for which the amount of reaction product, *i.e.* amplicon, is monitored as the reaction proceeds. There are many forms of real-time PCR that differ mainly in the detection chemistries used for monitoring the reaction product *(see, e.g.,.* Gelfand et al. U.S. Pat. No. 5,210,015 ("TAQMANTM"); Wittwer et al. U.S. Pat. Nos. 6,174,670 and 6,569,627 (intercalating dyes); Tyagi etal. U.S. Pat. No. 5,925,517 (molecular beacons); and the like). Detection chemistries for real-time PCR are reviewed, *inter alia* in Mackay et al. (2002) Nucl. Acids Res. 30: 1292-1305,

The terms "quantitative PCR" or "qPCR" refer to a PCR designed to measure the abundance of one or more specific target sequences in a sample or specimen. Quantitative PCR includes both absolute quantitation and relative quantitation of such target sequences. Typically, quantitative measurements are made using one or more reference sequences that may be assayed separately or together with a target sequence. The reference sequence can be endogenous or exogenous to a sample or specimen, and in the latter case, may comprise one or more competitor templates. Typical endogenous reference sequences include, but are not limited to segments of transcripts of the following genes: β-actin, GAPDH, β2-microglobulin, ribosomal RNA, and the like. Techniques for quantitative PCR are well-known to those of ordinary skill in the art *(see, e.g.,* Freeman et al. (1999) Biotechniques, 26: 112-126; Becker-Andre et al. (1989) Nucl. Acids Res. 17: 9437-9447; Zimmerman et al. (1996) Biotechniques, 21: 268-279; Diviacco et al. (1992) Gene, 122: 3013-3020; and the like).

The terms "spectrally resolvable" or "different and distinguishable" in reference to a plurality of optical *(e.g.,* fluorescent) labels means that the optical signal *(e.g.,* fluorescent emission) bands of the labels are sufficiently distinct, *i.e.* sufficiently nonoverlapping, that molecular tags to which the respective labels are attached can be distinguished on the basis of the signal (e.g., fluorescent signal) generated by the respective labels by standard photodetection systems, e.g. employing a system of band pass filters and photomultiplier tubes, or the like *(see, e.g.,* the systems described in U.S. Patent Nos: 4,230,558; 4,811,218, and the like, or in Wheeless et al. (1985) pages. 21 -76, in Flow Cytometry: Instrumentation and Data Analysis (Academic Press, New York).

The term "tubefill procedure" refers to a procedure that is run using standard laboratory instrumentation rather than on a cassette (e.g., rather than with a GENEXPERT^{®}, or modified GENEXPERT^{®} cartridge described herein).

### DETAILED DESCRIPTION

In a first aspect the present invention provides a cartridge for performing immuno-PCR to detect and/or quantify one or more target analytes, and/or detecting and/or quantifying a nucleic acid, said cartridge comprising: a sample receiving chamber; a chamber comprising a matrix material that acts as a filter and/or a DNA binding agent; a temperature controlled channel or chamber; and a plurality of chambers containing reagents for performing immuno-PCR, wherein: said plurality of chambers comprises a chamber containing a capture antibody that binds the analyte that is to be detected; said plurality of chambers comprises a chamber containing a detection antibody where said detection antibody is optionally attached directly or indirectly to a signal DNA; said plurality of chambers comprises a chamber containing a PCR master mix; said plurality of chambers comprises a chamber containing primers for amplifying all or a region of said signal DNA; said plurality of chambers comprises a chamber containing a probe for detecting all or a region of said signal DNA, and wherein said sample receiving chamber, said chamber comprising a matrix material, said temperature controlled heating channel or chamber or a port into said temperature controlled channel or chamber, and said plurality of chambers are disposed around a central valve and selectively in fluid communication with a channel in said central valve, wherein said central valve is configured to accommodate a plunger that is capable of drawing fluid into or out of a chamber in fluid communication with said central valve.

Said PCR primers, and/or probes, and/or polymerase in said master mix may be provided as beads. Said cartridge may contain detection antibodies for the detection of a single analyte. Said cartridge may contain capture antibodies for the capture of a single analyte. Said cartridge may contain detection antibodies for the detection of a plurality of analytes. Said cartridge may contain detection antibodies for the detection of at least 2, or at least 3, or at least 4, or at least 5, or at least 6, or at least 7, or at least 8, or at least 9, or at least 10 different analytes. Said cartridge may contain capture antibodies for the capture of a plurality of analytes. Said capture antibody may be attached to the wall of a reaction chamber or to said matrix material. Said capture antibody may be chemically conjugated to the wall of a reaction chamber or to said matrix material. Said capture antibody may be attached to the wall of a reaction chamber or to said matrix material via a biotin/streptavidin interaction. Said capture antibody may be attached to a particle. Said capture antibody may be chemically conjugated to said particle. Said capture antibody may be attached to said particle via a biotin/streptavidin interaction. Said particle may comprise a particle selected from the group consisting of a latex particle (polystyrene), poly(styrene/divinylbenzene) copolymers, polymethylmethacrylate (PMMA), poly(hydroxyethyl methacrylate) (pHEMA), a silica particle, a teflon particle, and noble metal particle, and a magnetic particle. Said particle may comprise a latex particle. Said particle may range in size from about 0.5 µm, or from about 1 µm up to about 10 µm, or up to about 3 µm, or said particle may range in size from about 1 µm up to about 2.8 µm. Said plurality of chamber may comprise a chamber containing one or more blocking agents to reduce non-specific binding. Said blocking agent may be selected from the group consisting of a polymer, a detergent, a carbohydrate, a protein, a surfactant, and a polysaccharide. Said blocking agent may comprise a polymer, detergent, or carbohydrate selected from the group consisting of PEG, Pluronics F68/F 108/F 127 (F68 preferred), PVP, Biolipidure 802 (NOF America), Tween 20 or 80, TEGME (Tre(ethylene glycol) monoethyl ether), TEG (Tegraethylene glycol), and phosphorylcholine containing polymers. Said blocking agent may comprise a protein, a surfactant, or a carbohydrate from the group consisting of Casein, BSA, goat IgG, bovine IgG, Stabilcoat (ThermoFisher), Iota-carrageenan, dextran sulfate, herring or salmon sperm DNA, and mouse serum. Said detection antibody may not be attached to a signal DNA and said cartridge further may comprise a chamber containing a label antibody that binds to said detection antibody where said label antibody is attached to a signal DNA. Said detection antibody may be attached to a signal DNA. Said signal DNA may be chemically conjugated to said detection antibody, or when said label antibody is present, said signal DNA may be chemically conjugated to said label antibody. Said signal DNA may be chemically conjugated to said antibody through a cysteine, through a lysine, or through a carbohydrate. Said signal DNA may be chemically conjugated with a C₆ to C₁₈ linker, or a C₆ to C₁₂ linker. Said signal DNA may be chemically conjugated with a with a heterobifunctional cross-linker. Said signal DNA may be chemically conjugated with succinimidyl 4-hydrazinonicotinate acetone hydrazone (SANH) or succinimidyl 4-(*N*-maleimidomethyl) cyclohexane-1-carboxylate (SMCC). Said signal DNA may be chemically conjugated via an azide modification of the DNA and linkage to the antibody through a dibenzocyclooctyne (DBCO) moiety. Said linker may comprise a DBCO-PEG-NHS linker. Said signal DNA may be chemically conjugated with a cleavable linker. Said cleavable linker may be selected from the group consisting of a linker containing a cleavable disulfide linkage, a base-cleavable linker, and an acid cleavable linker. Said cleavable linker may comprise a disulfide linkage cleavable with DTT. Said cleavable linker may additionally comprise a tetrazine. Said signal DNA may be attached to said detection antibody via a biotin/avidin interaction, or when said label antibody is present, said signal DNA may be attached to said label antibody via a biotin/avidin interaction. Said biotin/avidin interaction may be a biotin/streptavidin interaction or a biotin/neutravidin interaction. Said signal DNA may comprise a *Ter* sequence and said detection antibody or said label antibody may be attached to a Tus protein and said signal nucleic acid may bind to said detection antibody or to said label antibody via a Tus-Ter interaction. Said detection antibody may be attached to a bead and said signal DNA is to the same bead, or when said label antibody is present, said label antibody may be attached to a bead and said signal DNA may be attached to the same bead. Said antibody and/or said signal DNA may be chemically conjugated to said bead. Said antibody and/or said signal DNA may be attached to said bead via a biotin/streptavidin interaction. Said bead may comprise a material selected from the group consisting of latex, silica, ceramic, teflon, a noble metal, a semiconductor, and a magnetic material. Said bead may comprise gold. Said detection antibody may be presented on the surface of a phage and the signal DNA may be contained within said phage, or when said label antibody is present, said label antibody may be presented on the surface of a phage and said signal DNA contained within said phage. Said plurality of chambers further may comprise a chamber containing PCR primers for amplifying a nucleic acid other than said signal nucleic acid. Said plurality of chambers may further comprise a chamber containing a probe for detecting and/or quantifying a nucleic acid other than said signal nucleic acid. Said target analyte may comprise a polypeptide and said nucleic acid other than said signal nucleic acid may comprise a nucleic acid encoding said polypeptide or a fragment thereof. Said target analyte may comprise a polypeptide and said nucleic acid other than said signal nucleic acid comprise a nucleic acid characteristic of a cell, tissue, or organism that produces said polypeptide. Said cartridge may comprise one or more chambers containing reagents for TaqMan PCR reactions. Said cartridge may comprise one or more chambers containing one or more fluorescent probes that are markers for amplified signal DNA, and optionally one or more fluorescent probes that are markers for amplified sequence other than amplified signal DNA. Said probes may comprise a fluorescent reporter dye and a quencher dye, where the probes provides a signal upon cleavage by the 5' to 3' nuclease activity of Taq DNA polymerase. Said primers for amplifying a nucleic acid other than said signal nucleic acid, and/or said probe for detecting a nucleic acid other than said signal nucleic acid may be provided as beads. Said temperature controlled channel or chamber may be a thermocycling channel or chamber. Said matrix material may comprise a material that is selected from the group consisting of glass or silica, an ion exchange resin, and hydroxyapatite. Said sample receiving chamber, said chamber comprising a matrix material, said plurality of chambers containing reagents, and said temperature-controlled heating channel or chamber, may be selectively in fluid communication. Said sample receiving chamber, said chamber comprising a matrix material, said plurality of chambers containing reagents, and said temperature-controlled heating channel or chamber, may be selectively in fluid communication by microfluidic channels and valves. Said cartridge may be configured so that, when in use, said cartridge comprises: a chamber containing a sample; a chamber containing said detection antibody; a chamber containing said capture antibody; a chamber containing a wash buffer; and a chamber containing a PCR master mix for amplifying said signal DNA. Said cartridge may comprise a chamber containing a sample; a chamber containing said detection antibody; a chamber containing said capture antibody; a chamber containing a wash buffer; a chamber containing KOH; a chamber containing HEPES or Tris-HCl; and a chamber to receive waste. Said cartridge may comprise a chamber containing a sample; a chamber containing said detection antibody in PBS at about pH 7.25; a chamber containing said capture antibody in PBS at about pH 7.25; a chamber containing a PBS wash buffer; a chamber containing KOH; a chamber containing HEPES at about pH 8.25 or Trish-HCl at about pH 7.4; and a chamber to receive waste. Said capture antibody and said detection antibody may be antibodies that bind to an analyte selected from the group consisting of a viral antigen, a bacterial antigen, a prion, a parasitic antigen, a mycotoxin, and a cancer marker, and a drug-resistance gene product. Said capture antibody and said detection antibody may be antibodies that bind to a viral antigen selected from the group consisting of Hepatitis B surface antigen, Hepatitis C surface antigen, bovine herpesvirus antigen, norovirus capsid, rotavirus, Angiostrongylus cantonensis worms, Hantavirus protein, avian influenza viral antigen, HIV-1 antigen, and H5N1 antigen. Said capture antibody and said detection antibody may be antibodies that bind to a prion selected from the group consisting or bovine PRP, human brain PRP. Said capture antibody and said detection antibody may be antibodies that bind to a bacterial antigen or bacterial toxin selected from the group consisting of C. *difficile* antigen, C. *difficile* Toxin A, *C. difficile Toxin* B, *P. piscidia, E. coli* antigen, *Bacteroides fragilis,* BoNT/A, *Streptococcus pyogenes* group A, *Staphylococcus aureus, Y. pestis* antigen, and *M. tuberculosis* antigen. Said capture antibody and said detection antibody may be antibodies that bind to a bacterial toxin selected from the group consisting of shiga toxin 2, *Clostridium botulinum* neurotoxin A, Staphylococcal enterotoxin, *Bacillus thurigiensis* toxin, C. *difficile* Toxin A, and C. *difficile* Toxin B. Said capture antibody and said detection antibody may be antibodies that bind to a polypeptide encoded by a drug resistance gene. Said capture antibody and said detection antibody may be antibodies that bind to a drug-resistance polypeptide selected from the group consisting of p-glycoprotein p glycoprotein, OleC polypeptide, mbcF polypeptide, MsrA polypeptide(s), bexA polypeptide, bexB polypeptide, kpsT polypeptide, and kpsM polypeptide. Said capture antibody and said detection antibody may be antibodies that bind to an analyte that is a marker for a host-response inflammation. Said capture antibody and said detection antibody may be antibodies that bind to an analyte selected from the group consisting of IL-8, calprotectin, and lactoferrin. Said capture antibody and said detection antibody may be antibodies that bind to an analyte that is a cancer marker. Said capture antibody and said detection antibody may be antibodies that bind to a cancer marker in Table 5. Said cartridge may contain primers and probes for detecting and/or quantifying a nucleic acid that encodes an analyte or a fragment of an analyte selected from the group consisting of a viral antigen, a bacterial antigen, a prion, a parasitic antigen, a mycotoxin, and an analyte from a cancer cell. Said cartridge may contain primers and probes for detecting and/or quantifying a nucleic acid that encodes an viral antigen or a fragment of thereof where said antigen is selected from the group consisting of Hepatitis B surface antigen, Hepatitis C surface antigen, bovine herpesvirus antigen, norovirus capsid, rotavirus, Angiostrongylus cantonensis worms, Hantavirus protein, avian influenza viral antigen, HIV-1 antigen, and H5N1 antigen. Said cartridge may contain primers and probes for detecting and/or quantifying a nucleic acid that encodes a bacterial antigen or bacterial toxin or fragment thereof where said bacterial antigen or bacterial toxin is selected from the group consisting of C. *difficile* antigen, C. *difficile* Toxin A, *C. difficile* Toxin B, *P. piscidia, E. coli* antigen, *Bacteroides fragilis,* BoNT/A, *Streptococcus pyogenes* group A, *Staphylococcus aureus, Y. pestis* antigen, and *M. tuberculosis* antigen. Said cartridge may contain primers and probes for detecting and/or quantifying a nucleic acid that encodes a bacterial toxin or fragment thereof where said bacterial toxin is selected from the group consisting of shiga toxin 2, *Clostridium botulinum* neurotoxin A, Staphylococcal enterotoxin, *Bacillus thurigiensis* toxin, C. *difficile* Toxin A, and C. *difficile* Toxin B. Said cartridge may contain primers and probes for detecting and/or quantifying a nucleic acid that encodes a polypeptide expressed by a drug resistance gene. Said cartridge may contain primers and probes for detecting and/or quantifying a nucleic acid that encodes a drug-resistance polypeptide selected from the group consisting of MRP, p-glycoprotein p glycoprotein, OleC polypeptide, mbcF polypeptide, MsrA polypeptide(s), bexA polypeptide, bexB polypeptide, kpsT polypeptide, and kpsM polypeptide. Said cartridge may contain primers and probes for detecting and/or quantifying a nucleic acid that encodes a marker or a fragment of a marker for a host-response inflammation. Said cartridge may contain primers and probes for detecting and/or quantifying a nucleic acid that encodes a marker or a fragment of a marker selected from the group consisting of IL-8, calprotectin, and lactoferrin. Said cartridge may contain primers and probes for detecting and/or quantifying a nucleic acid that encodes a cancer marker or a fragment of a cancer marker. Said cartridge may contain primers and probes for detecting and/or quantifying a nucleic acid that encodes a cancer marker or a fragment of a cancer marker in Table 5. Said cartridge may be loaded with a sample comprising a material from a biological, environmental, medical, or patient source. Said sample may comprise an environmental sample selected from the group consisting of surface matter, soil, water, vegetation, and an industrial sample. Said sample may comprise a biological sample selected from the group consisting of a culture, blood, saliva, cerebral spinal fluid, urine, stool, bronchial aspirates, tracheal lavage, pleural fluid, milk, lymph, sputum, semen, needle aspirates, punch biopsies, surgical biopsies, cryopreserved sections, FFPE section. Said sample may comprise an environmental sample selected from the group consisting of surface matter, soil, water, vegetation, and an industrial sample. Said sample may comprise a food or agriculture sample, selected from the group consisting of meat, meat products, avian or avian products, milk or milk products, and farm crops, and organic waste.

In a second aspect the present invention provides a method of performing immuno-PCR to detect and/or quantify one or more target analytes, and/or detecting and/or quantifying a target nucleic acid, said method comprising: providing a sample in a sample receiving chamber of a cartridge configured for performing immuno-PCR; and using said cartridge in an immuno-PCR method comprising: contacting said analyte with a capture antibody under conditions where said capture antibody binds said analyte, forming a capture antibody/analyte complex; contacting said analyte with a detection antibody attached to a signal DNA under conditions where said detection antibody specifically binds to and forms an immunocomplex with said capture antibody/analyte complex; releasing said immunocomplex or a portion thereof comprising said signal DNA and delivering said immunocomplex or a portion thereof into a temperature controlled channel or chamber; and performing a nucleic acid amplification in said temperature controlled channel or chamber to detect and/or quantify said signal nucleic acid thereby detecting and/or quantifying said analyte, wherein said cartridge is a cartridge as described above.

In a third aspect the present invention provides a method of performing immuno-PCR to detect and/or quantify one or more target analytes, and/or detecting and/or quantifying a nucleic acid, said method comprising: providing a sample in a sample receiving chamber of a cartridge configured for performing immuno-PCR; and using said cartridge in an immuno-PCR method comprising: contacting said analyte with a capture antibody under conditions where said capture antibody binds said analyte, forming a capture antibody/analyte complex; contacting said analyte with a detection antibody where said contacting is under conditions where said detection antibody specifically binds to and forms an immunocomplex with said capture antibody/analyte complex; contacting said immunocomplex with a labeled antibody that binds to said detection antibody where said labeled antibody is attached to a signal DNA and said contacting is under conditions where said labeled antibody specifically binds to said detection antibody to form a labeled immunocomplex; releasing said labeled immunocomplex or a portion thereof comprising said signal DNA and delivering said immunocomplex or a portion thereof into a temperature controlled channel or chamber; and performing a nucleic acid amplification in said temperature controlled channel or chamber to detect and/or quantify said signal nucleic acid thereby detecting and/or quantifying said analyte, wherein said cartridge is a cartridge as described above.

Said nucleic acid amplification may comprise a method selected from the group consisting of polymerase chain reaction (PCR), ligase chain reaction (LCR), ligase detection reaction (LDR), multiplex ligation-dependent probe amplification (MLPA), ligation followed by Q-replicase amplification, primer extension, strand displacement amplification (SDA), hyperbranched strand displacement amplification, multiple displacement amplification (MDA), nucleic acid strand-based amplification (NASBA), and rolling circle amplification (RCA). Said nucleic acid amplification may comprise polymerase chain reaction (PCR). Said nucleic acid amplification may comprise qPCR. Said capture antibody may be attached to the wall of a reaction chamber or to said matrix material and binding of said analyte may immobilize said analyte in said reaction chamber or said matrix material. Said capture antibody may be attached to a particle and binding of said analyte may attache said analyte to said particle. Said contacting said analyte with a detection antibody may comprise contacting said analyte attached to said particle with said detection antibody. Said method may comprise trapping said particle in said matrix. When said capture antibody is attached to a bead, releasing said immunocomplex or a portion thereof or releasing said labeled immunocomplex or a portion thereof may comprise reverse-eluting the entire complex and delivering that complex into said temperature controlled channel or chamber; or when said capture antibody is attached to the wall of a reaction chamber or to said matrix material releasing said immunocomplex, or a portion thereof, or releasing said labeled immunocomplex, or a portion thereof, may comprise cleaving the entire complex from said wall or matrix material and delivering that complex into said temperature controlled channel or chamber. Said releasing said immunocomplex or a portion thereof or releasing said labeled immunocomplex or a portion thereof may comprise: releasing said detection antibody attached to a signal DNA from said immunocomplex and delivering said detection antibody with attached signal DNA into said temperature controlled channel or chamber; or releasing said label antibody attached to a signal DNA from said labeled immunocomplex and delivering said label antibody attached to a signal DNA into said temperature controlled channel or chamber. Said releasing may comprise chemically disrupting said detection antibody from said immunocomplex or chemically disrupting said label antibody from said labeled immunocomplex. Said disrupting may comprise contacting said immunocomplex or labeled immunocomplex with a base. Said disrupting may comprise contacting said immunocomplex or labeled immunocomplex with KOH. Said releasing may comprise using heat to disrupt said detection antibody from said immunocomplex or said label antibody from said labeled immunocomplex. Said releasing may comprise using sonication to disrupt said detection antibody from said immunocomplex or said label antibody from said labeled immunocomplex. Said signal DNA may be chemically conjugated to said detection antibody or to said label antibody and releasing said immunocomplex, or a portion thereof, or releasing said labeled immunocomplex, or a portion thereof, may comprise cleaving the linker joining said signal DNA to said detection antibody or to said label antibody, and delivering said signal DNA into said temperature controlled channel or chamber. The linker joining said signal DNA to said detection antibody or to said label antibody may be an acid-labile linker, and said cleaving may comprise contacting said linker with an acid capable of cleaving said linker. The linker joining said signal DNA to said detection antibody or to said label antibody may be a base-labile linker, and said cleaving may comprise contacting said linker with a base capable of cleaving said linker. The linker joining said signal DNA to said detection antibody or to said label antibody may comprise a disulfide bond, and said cleaving may comprise contacting said linker with an agent capable of disrupting said disulfide bond. Said agent capable of disrupting said disulfide bond may comprise dithiothreitol (DTT). Said signal DNA may be chemically conjugated to said detection antibody or to said label antibody or joined to said detection antibody, or to said label antibody, by an avidin/biotin interaction, and releasing said immunocomplex, or a portion thereof, or releasing said labeled immunocomplex, or a portion thereof, may comprise cleaving said signal DNA to release a fragment of said signal DNA that is capable of detection or quantification in a nucleic acid amplification reaction, and delivering said released fragment of said signal DNA into said temperature controlled channel or chamber. Said cleaving said signal DNA may comprise contacting said signal DNA with a restriction endonuclease that recognizes a restriction site in said signal DNA and cleaves said signal DNA at or near said restriction site. Said signal DNA may be attached to a bead and said detection antibody or said label antibody may be attached to the same bead, and releasing said signal DNA, may comprise heating or sonicating said signal DNA and/or bead to release said signal DNA, and delivering said signal DNA into said temperature controlled channel or chamber. Said detection antibody may be presented on the surface of a phage that contains said signal DNA or said label antibody may be presented on the surface of a phage that contains said signal DNA, and releasing said signal DNA may comprise heating or sonicating or lysing said phage to release said signal DNA to release said signal DNA, and delivering said signal DNA into said temperature controlled channel or chamber. Said method may comprise washing said immunocomplex or said labeled immunocomplex to remove unbound materials or non-specifically bound material before releasing said immunocomplex or a portion thereof or said labeled immunocomplex or a portion thereof. Said temperature controlled channel or chamber may comprise a thermocycling channel or chamber and said amplifying may comprise a polymerase chain reaction (PCR). Said PCR may comprise qPCR. Said amplifying may comprise a method selected from the group consisting of a linear polymerase reaction, a ligase chain reaction, a strand displacement reaction, a nucleic acid sequenced based amplification, and a rolling circle amplification reaction. Said amplification may comprise subjecting a reaction mixture containing said signal DNA to amplification conditions, and monitoring an optical signal of an indicator in the reaction mixture. Said optical signal may be selected from the group consisting of a fluorescent signal, a chemiluminescent signal, an electrochemiluminescent signal, and a colorimetric signal. The optical signal may be a fluorescent optical signal generated by a fluorescent indicator. Said fluorescent indicator may be a non-specific intercalating dye that binds to double-stranded DNA products. Said fluorescent indicator may comprise a target sequence specific probe. Said target sequence specific probe may be selected from the group consisting of a TAQMAN probe, a SCORPION probe, and a MOLECULAR BEACON. Said method may be performed on a plurality of different analytes in the same cartridge. Said plurality may comprise at least 2, or at least 3, or at least 4, or at least 5, or at least 6 different analytes. Each analyte comprising said plurality of analytes may be derived from the same sample in said cartridge. The signal DNAs representing each of the analytes comprising said plurality may be amplified sequentially in the same temperature controlled channel or chamber. Between each amplification reaction said method may comprise: washing said temperature controlled channel or chamber with a wash solution; and removing said wash solution from said temperature controlled channel or chamber and fluidly transferring the wash solution to the a waste reservoir. After removing said wash solution from said temperature controlled channel or chamber said method may comprise transferring air into said temperature controlled channel or chamber and heating the channel or chamber to a temperature at or above a DNA denaturation temperature. Said denaturation temperature may range from about 90°C to about 99°C. The signal DNAs representing each of the analytes comprising said plurality may be amplified simultaneously each in a different temperature controlled channel or chamber in said cartridge. The signal DNAs representing each of the analytes comprising said plurality may be amplified simultaneously in the same temperature controlled channel or chamber in said cartridge and amplification of each signal DNA provides a different a distinguishable optical signal. Said method can be completed in about 1 hour or less, or in about 50 minutes or less, or in about 40 minutes or less, or in about 30 minutes or less, or in about 20 minutes or less, or in about 15 minutes or less. Said method may further comprise amplifying a nucleic acid other than said signal DNA. Said amplifying a nucleic acid other than said signal DNA may comprise: binding a nucleic acid from said sample to a matrix material; washing the bound nucleic acid to provide a washed nucleic acid; eluting the washed nucleic acid; and subjecting said washed nucleic acid to an amplification reaction to amplify a target nucleic acid sequence if present in said washed nucleic acid. Said nucleic acid other than said signal DNA may comprise a DNA. Said nucleic acid other than said signal DNA may comprise an RNA. Said amplifying a nucleic acid other than said signal DNA may be performed on nucleic acid(s) obtained from the same sample in the sample chamber used for said immuno-PCR. Said amplifying a nucleic acid other than said signal DNA may be performed on nucleic acid(s) obtained from a sample in a sample chamber different than the sample chamber used for said immuno-PCR. The signal DNAs and the nucleic acid(s) other than a signal DNA may be amplified sequentially in the same temperature controlled channel or chamber The signal DNA may be amplified before the amplification of the nucleic acid other than a signal DNA. The signal DNA may be amplified after the amplification of the nucleic acid other than a signal DNA. Between the amplification of said signal DNA and the amplification of a nucleic acid other than a signal DNA said method may comprise: washing said temperature controlled channel or chamber with a wash solution; and removing said wash solution from said temperature controlled channel or chamber and fluidly transferring the wash solution to the a waste reservoir. After removing said wash solution from said temperature controlled channel or chamber said method may comprise transferring air into said temperature controlled channel or chamber and heating the channel or chamber to a temperature at or above a DNA denaturation temperature. Said denaturation temperature may range from about 90°C to about 99°C. The signal DNA(s) and the nucleic acid(s) other than a signal DNA may be amplified simultaneously each in a different temperature controlled channel or chamber in said cartridge. The signal DNA(s) and the nucleic acid(s) other than a signal DNA may be amplified simultaneously in the same temperature controlled channel or chamber in said cartridge and amplification of the signal DNA(s) and the nucleic acid(s) other than a signal DNA may provide a different a distinguishable optical signal. Said amplification of a nucleic acid other than a signal DNA may comprise subjecting a reaction mixture containing said nucleic acid other than a signal DNA to amplification conditions, and monitoring an optical signal of an indicator in the reaction mixture. Said optical signal may be selected from the group consisting of a fluorescent signal, a chemiluminescent signal, an electrochemiluminescent signal, and a colorimetric signal. The optical signal may be a fluorescent optical signal generated by a fluorescent indicator. Said fluorescent indicator may be a non-specific intercalating dye that binds to double-stranded DNA products. Said fluorescent indicator may comprise a target sequence specific probe. Said target sequence specific probe may be selected from the group consisting of a TAQMAN probe, a SCORPION probe, and a MOLECULAR BEACON. Said sample may comprise a material from a biological, environmental, medical, or patient source. Said sample may comprise an environmental sample selected from the group consisting of surface matter, soil, water, vegetation, and an industrial sample. Said sample may comprise a biological sample selected from the group consisting of a culture, blood, saliva, cerebral spinal fluid, urine, stool, bronchial aspirates, tracheal lavage, pleural fluid, milk, lymph, sputum, semen, needle aspirates, punch biopsies, surgical biopsies, cryopreserved sections, FFPE section. Said sample may comprise an environmental sample selected from the group consisting of surface matter, soil, water, vegetation, and an industrial sample. Said sample may comprise a food or agriculture sample, selected from the group consisting of meat, meat products, avian or avian products, milk or milk products, and farm crops, and organic waste. Said analyte may comprise a moiety selected from the group consisting of a polypeptide, a lectin, a lipid, a carbohydrate, and a small organic molecule. Said analyte may be selected from the group consisting of a viral antigen, a bacterial antigen, a prion, a parasitic antigen, a mycotoxin, and a cancer marker, and a drug-resistance gene product. Said analyte may comprise a viral antigen selected from the group consisting of Hepatitis B surface antigen, Hepatitis C surface antigen, bovine herpesvirus antigen, norovirus capsid, rotavirus, Angiostrongylus cantonensis worms, Hantavirus protein, avian influenza viral antigen, HIV-1 antigen, and H5N1 antigen. Said analyte may comprise a prion selected from the group consisting or bovine PRP, human brain PRP. Said analyte may comprise a bacterial antigen or bacterial toxin selected from the group consisting of C. *difficile* antigen, C. *difficile* Toxin A, *C. difficile Toxin* B, *P. piscidia, E. coli* antigen, *Bacteroides fragilis,* BoNT/A, *Streptococcus pyogenes* group A, *Staphylococcus aureus, Y. pestis* antigen, *and M. tuberculosis* antigen. Said analyte may comprise a bacterial toxin selected from the group consisting of shiga toxin 2, *Clostridium botulinum* neurotoxin A, Staphylococcal enterotoxin, *Bacillus thurigiensis* toxin, *C. difficile* Toxin A, and C. *difficile* Toxin B. Said analyte may comprise a polypeptide encoded by a drug resistance gene. Said analyte may comprise a drug-resistance polypeptide selected from the group consisting of p-glycoprotein p glycoprotien, OleC polypeptide, mbcF polypeptide, MsrA polypeptide(s), bexA polypeptide, bexB polypeptide, kpsT polypeptide, and kpsM polypeptide. Said analyte may comprise a marker for a host-response inflammation. Said marker may be selected from the group consisting of IL-8, calprotectin, and lactoferrin. Said analyte may comprise a cancer marker. Said analyte may comprise a cancer marker in Table 5. Said method may comprise amplifying a nucleic acid that encodes an analyte or a fragment of an analyte selected from the group consisting of a viral antigen, a bacterial antigen, a prion, a parasitic antigen, a mycotoxin, and an analyte from a cancer cell. Said method may comprise amplifying a nucleic acid that encodes a viral antigen or a fragment of thereof where said antigen is selected from the group consisting of Hepatitis B surface antigen, Hepatitis C surface antigen, bovine herpesvirus antigen, norovirus capsid, rotavirus, Angiostrongylus cantonensis worms, Hantavirus protein, avian influenza viral antigen, HIV-1 antigen, and H5N1 antigen. Said method may comprise amplifying a nucleic acid that encodes a bacterial antigen or bacterial toxin or fragment thereof where said bacterial antigen or bacterial toxin is selected from the group consisting of C. *difficile* antigen, *C. difficile* Toxin A, *C. difficile* Toxin B, *P. piscidia, E. coli* antigen, *Bacteroides fragilis,* BoNT/A, *Streptococcus pyogenes* group A, *Staphylococcus aureus, Y. pestis* antigen, and *M*. *tuberculosis* antigen. Said method may comprise amplifying a nucleic acid that encodes a bacterial toxin or fragment thereof where said bacterial toxin is selected from the group consisting of shiga toxin 2, *Clostridium botulinum* neurotoxin A, Staphylococcal enterotoxin, *Bacillus thurigiensis* toxin, C. *difficile* Toxin A, and C. *difficile* Toxin B. Said method may comprise amplifying a nucleic acid that encodes a polypeptide expressed by a drug resistance gene. Said cartridge may contain primers and probes for detecting and/or quantifying a nucleic acid that encodes a drug-resistance polypeptide selected from the group consisting of MRP, p-glycoprotein p glycoprotien, OleC polypeptide, mbcF polypeptide, MsrA polypeptide(s), bexA polypeptide, bexB polypeptide, kpsT polypeptide, and kpsM polypeptide. Said method may comprise amplifying a nucleic acid that encodes a marker for a host-response inflammation. Said cartridge may contain primers and probes for detecting and/or quantifying a nucleic acid that encodes a marker selected from the group consisting of IL-8, calprotectin, and lactoferrin. Said method may comprise amplifying a nucleic acid that encodes a cancer marker. Said method may comprise amplifying a nucleic acid that encodes a cancer marker in Table 5.

In a fourth aspect the present invention provides a system for performing immuno-PCR to detect and/or quantify one or more target analytes, and/or to detect and/or to quantify a nucleic acid, said system comprising: an enclosure configured to contain one or more sample processing modules, each sample processing module configured to hold a removable cartridge as described above, where said system is configured to operate the sample processing modules to perform immuno-PCR to determine the presence and/or quantity of one or more target analytes and/or to determine the level of one or more target DNA sequences within a corresponding removable sample cartridge, wherein said processing on a sample within the corresponding removable sample cartridge performs a method as described above.

Said system may be configured to contain one sample processing module. Said system may be configured to contain at least two sample processing modules, or at least 4 sample processing modules, or at least 8 sample processing modules, or at least 12 sample processing modules, or at least 16 sample processing modules, or at least 20 sample processing modules, or at least 24 sample processing modules, or at least 28 sample processing modules, or at least 32 sample processing modules, or at least 64 sample processing modules, or at least 128 sample processing modules. Said modules may comprise one or more heating plates to heat a temperature controlled chamber or channel in said cartridge. Said modules may comprise a fan configured to cool a temperature controlled channel or chamber in said cartridge. Said modules may comprise circuitry to pass information (e.g., optical information) to a computer for analysis. Said modules may comprise optical blocks to provide excitation and/or detection of one or more optical signals produced by reactions in said cartridge. Said system may be configured to operate said cartridge to perform an immuno-PCR. Said system may be configured to operate said cartridge to perform an amplificaiton of a nucleic acid other than a signal DNA.

In a fifth aspect the present invention provides a kit for performing immuno-PCR to detect and/or to quantify one or more target analytes, and/or to detect and/or to quantify a nucleic acid, said kit comprising: a container containing a cartridge as described above. Said kit may further comprise a container containing one or more reagents for preparing a sample for immuno-PCR.

Said kit may further comprise a container containing one or more reagents for preparing a sample for a nucleic acid amplification. Said one or more reagents for preparing a sample for a nucleic acid amplification may comprise a lysis solution for serum, or plasma, or an FFPE sample. Said one or more reagents for preparing a sample for a nucleic acid amplification may comprise proteinase K. Said kit may contain instructional materials teaching the use of said cartridge for the performance of immuno-PCR. Said kit may contain instructional materials teaching the use of said cartridge for performing a nucleic acid amplification in addition to said immuno-PCR.

In various embodiments devices and methods are provided that facilitate the rapid detection and/or quantification of a polypeptide (or other analyte) using immuno-PCR. In certain embodiments the devices also provide detection and/or quantification of a nucleic acid of interest (other than an immuno-PCR signal DNA) by PCR or other amplification methods. In view of the ability to detect and/or quantify both polypeptides (or other analytes) and nucleic acids, the devices provided herein permit detection and/or quantification of a target analyte (e.g., polypeptide) and, optionally, a reflex assay for a relevant nucleic acid, or conversely detection and/or quantification of a target nucleic acid and a reflex assay for a relevant polypeptide. In certain embodiments automated reaction cartridges are provided that facilitate rapid and accurate immuno-PCR as well as nucleic acid detection (e.g., via qPCR) as are methods that utilize the automated reaction cartridge(s) to facilitate detection and/or quantification of one or more target analytes (e.g., polypeptide(s)) and, optionally, one or more nucleic acid target(s).

In certain embodiments, the cartridges described herein perform all or part of one or more immuno-PCR reaction(s) including, but not limited to both direct and indirect sandwich formats. In certain embodiments the cartridges provide preparation of the target immunoconjugate, and optionally removal the entire immunocomplex, or removal of the antibody that is labeled with the signal DNA, or removal of the signal DNA from the bound immunoconjugate which can subsequently be separately and any of these moieties can be separately analyzed, e.g., in a PCR cartridge or in a benchtop PCR system. In certain embodiments the cartridges provide the full immuno-PCR assay including the amplification and detection and/or quantification of the amplified product. In certain embodiments the cartridges additionally provide for a nucleic acid analysis (e.g., preparation, amplification, and detection and/or quantification) of a nucleic acid *(e.g.,* RNA, DNA) other signal DNA(s) used in the immuno-PCR reaction(s). In certain embodiments the isolation and purification of the target analyte to be analyzed by immuno-PCR and, optionally the additional nucleic acid that is to be analyzed.

There are several advantages to automating the immuno-PCR analysis including for example, reduction in overall processing time, improvements in efficiency, decreased user error and variability, minimization of loss between steps, and an improved ability to use smaller amounts of sample. Use of a cartridge-based process, as described herein, allows for rapid and easy testing of multiple sample types. Additionally the ability to perform nucleic acid analysis (e.g., qPCR of RNA or DNA) on the same sample permits an effective reflex assay to be performed with minimal commitment of time and resources and is believed to provide improved detection thresholds and accuracy.

The immuno-PCR cartridges and methods described herein find use in a wide number of contexts. For example, immuno-PCR can be used to identify the presence of, and/or quantify any of a number of microorganisms including, but not limited to viruses, bacteria, fungi, protozoans including, but not limited to, any pathogenic forms/species/strains of such microorganisms.

In certain embodiments the immuno-PCR can be used to identify toxins produced by any of these microorganism including, but not limited to shiga toxin, *Clostridium difficile* toxin A and toxin B, botulinum neurotoxin A and B, various mycotoxins, and the like. The immuno-PCR devices and methods herein can be used to identify host-response inflammation markers such as IL-8, calprotectin and lactoferrin associated with *Clostridium difficile* infection *(see, e.g.,* Swale et al. (2014) PLoS ONE 9(8): e106118). The immuno-PCR devices and methods described herein can be used to identify various cancers or cancer markers and to identify cancer cells or microorganisms that show drug resistance (e.g., MRSA, drug-resistant tuberculosis, doxorubicin or gentamicin resistant cancer cells, and the like). The immuno-PCR assays find use, inter alia, in the medical field as well as in environmental studies *(e.g.,* to screen water, and soil contaminants), and in the agriculture field *(e.g.,* to screen beef, poultry, various food crops, and the like).

Because, in various embodiments, the cartridges described herein permit both an immuno-PCR analysis (of one or more target analytes) and a nucleic acid analysis (of one or more target nucleic acids) it is possible to first assay for an immuno-PCR target and then perform a reflex assay for a related nucleic acid, or to first assay for a nucleic acid target and then perform a reflex immuno-PCR assay for a related target analyte. The ability to rapidly perform such reflex assays on essentially the same sample improves the sensitivity of the assay (e.g., particularly where transcriptional and translational patterns not always positively correlated) and can reduce the occurrence of false positives.

### Immuno-PCR methods.

Immuno-PCR (I-PCR) combines the versatility of ELISA with the exponential amplification power and sensitivity of PCR, thus leading to an increase in sensitivity compared with an analogous ELISA. Immuno-PCR is similar to ELISA which detects an antigen-antibody interaction, but instead of using an enzyme-conjugated antibody, the antibody is labelled with a DNA fragment (a signal DNA), which can be amplified, e.g., by PCR. Although ELISA is the most commonly used method for the detection of antigens, it often fails when there is a low concentration of target antigen. PCR is widely used as a routine laboratory technique for the detection of nucleic acid molecules, but it cannot detect non-nucleic acid molecules.

Immuno-PCR is a versatile method that allows the detection of protein (or other) antigens and the antibodies that are generated against those antigens. Immuno-PCR has been widely explored for the detection of a variety of biological molecules including, but not limited to, cytokines, tumor markers, T-cell receptors, angiotensinogen, toxins, hormones, and biomarkers for autoimmune and Alzheimer's diseases. It can be adapted as a novel diagnostic tool for the detection of microbial antigens and antibodies *(see, e.g.,* Niemeyer et al. (2005) Trends Biotechnol. 23: 208-216; Niemeyer et al. (2007) Nat. Protoc. 2: 1918-1930; Malou and Raoult (2011) Trends Microbiol. 19: 295-302; Potůčková et al. (2011) J. Immunol. Meth. 371: 38-47; Mehta et al. (2012) Diagn. Microbiol. Infect. Dis. 72: 166-174; and the like). Immuno-PCR has been used to detect a variety of viral and bacterial proteins (e.g. rotavirus antigen VP6 *(see, e.g.,* Adler et al. (2005) Biochem. Biophys. Res. Commun. 333: 1289-1294), HIV p24 antigen *(see, e.g.,* Barletta et al. (2004) Am. J. Clin. Pathol. 122: 20-27), norovirus capsid *(see, e.g.,* Tian and Mandrell (2006) J. Appl. Microbiol. 100: 564-574), and the cell wall protein A of *Staphylococcus aureus (see, e.g.,* Huang and Chang (2004) Clin. Chem. 50: 1673-1674)). Immuno-PCR has also been adapted and applied to antibody detection, such as for the measurement of mumps-specific immunoglobulin G (IgG) in human serum *(see, e.g.,* McKie et al. (2002) J. Immunol. Meth. 270: 135-141; and the like).

Several commonly used formats of immuno-PCR include, but are not limited to direct immuno-PCR, indirect immuno-PCR, sandwich immuno-PCR and indirect sandwich immuno-PCR.

In a traditional sandwich immuno-PCR, the antigen to be detected (target analyte) is bound by a capture antibody attached to the wells of a microtiter plate and sandwiched between that capture antibody and a detection antibody that also binds the antigen *(see, e.g.,* Figure 1, left). The detection antibody has an attached signal DNA (also known as a reporter DNA) that is subsequently amplified to provide a detection signal. Formation of the "sandwich" (capture Ab-analyte-detection-Ab-Signal DNA) provides an immunocomplex (IC) (a.k.a. immunoconjugate). After washing to remove unbound materials and/or non-specifically bound materials, the signal DNA can be removed from the detection antibody, or the immunocomplex can be disrupted to release the detection antibody bearing the signal DNA for subsequent amplification and detection and/or quantification of the signal DNA amplification product which provides an indication of the presence and/or amount of target analyte. In certain novel embodiments described herein the entire immunocomplex can be introduced into the amplification reaction.

In an indirect sandwich immuno-PCR, the antigen (target analyte) is sandwiched between a capture antibody, typically attached to the wells of a microtiter plate and a detection antibody. The detection antibody, in turn, is bound by a label antibody that has an attached signal DNA (also known as a reporter DNA) that is subsequently amplified to provide a detection signal *(see, e.g.,* Figure 1, right). Formation of the "labeled sandwich" (capture Ab-analyte-detection Ab-Label Ab-Signal DNA) provides a "labeled immunoconjugate (a.k.a. labeled immunocomplex). After washing to remove unbound materials and/or non-specifically bound materials, the signal DNA can be removed from the label antibody, or the immunocomplex can be disrupted to release the label antibody bearing the signal DNA for subsequent amplification and detection and/or quantification of the signal DNA amplification product which provides an indication of the presence and/or amount of target analyte. In certain novel embodiments described herein the entire labeled immunocomplex can be introduced into the amplification reaction.

In direct immuno-PCR and indirect immuno-PCR the capture antibody is omitted and the analyte is bound directly to a surface, *e.g.,* a surface of a microtiter plate.

Without being bound to a particular theory, it is believed that sandwich formats (e.g., sandwich immuno-PCR and indirect sandwich immuno-PCR) are advantageous over the direct immuno-PCR and indirect immuno-PCR as the sandwich methods eliminate eliminates the need for the direct coating of analytes on a surface, which may decrease non-specific binding without compromising stability *(see, e.g.,* Niemeyer et al. (1997) Anal. Biochem. 246: 140-145; Mehta et al. (2012) Diagn. Microbiol. Infect. Dis. 72: 166-174; and the like). However, while cartridge-implemented immuno-PCR methods are described herein with respect to sandwich methods, it will be recognized that in various embodiments the formats can be altered to provide non-sandwich formats with direct attachment of the analyte(s) to beads and/or to chamber/channel/matrix sufaces).

As explained below, numerous strategies can be utilized in the cartridge methods described herein for attachment and/or release of the signal DNA.

It will also be appreciated that immuno-PCR as described herein is not limited solely to PCR amplification of the signal DNA. Other amplification methods are also contemplated herein and include, but are not limited to, ligase chain reaction (LCR), ligase detection reaction (LDR), multiplex ligation-dependent probe amplification (MLPA), ligation followed by Q-replicase amplification, primer extension, strand displacement amplification (SDA), hyperbranched strand displacement amplification, multiple displacement amplification (MDA), nucleic acid strand-based amplification (NASBA), rolling circle amplification (RCA), proximity ligase assay (PLA), and the like.

### Methods of Cartridge-Based Immuno-PCR.

In various embodiments the immuno-PCR, and optionally, a nucleic acid analysis (e.g., qPCR) of a nucleic acid other than a signal DNA used for the immuno-PCR, is performed in a cartridge. In certain embodiments where both an immuno-PCR and a nucleic acid analysis are preformed, both assays can be performed in the same cartridge.

In certain embodiments the binding of the target analyte *(e.g.,* a polypeptide), formation of a sandwich immunocomplex (or a labeled sandwich immunocomplex in the case of an indirect assay) the release of a signal DNA and subsequent amplification and detection and/or quantification of the signal DNA (which provides a measure of the presence and/or quantity of the target analyte) is all performed in a single cartridge. Similarly, where a nucleic acid analysis is additionally performed cleanup and amplification and detection and/or quantification of the amplified nucleic acid can all be performed in the same cartridge.

For immuno-PCR the sample, optionally prepared in a buffer/solution compatible with immuno-PCR is introduced into a sample chamber in the cartridge. The cartridge typically contains one or more, or all of the reagents required to perform a direct immuno-PCR reaction or an indirect immuno-PCR reaction. In certain embodiments the cartridge comprise one or more or all of the regents to detect a plurality of analytes using immuno-PCR. Thus, for example, the cartridge may contain capture and/or detection antibodies for 2, or 3, or 4, or 5, or 6, or 7, or 8, or 9, or 10 or more different target analytes.

In certain embodiments the cartridge is placed into a processing module and the immuno-PCR assay is initiated by clicking through a set of selections within the software controlling the processing module *(see, e.g.,* Figs. 10A and 10B). The cartridge then performs the immuno-PCR assay(s). In certain embodiments nucleic acid analysis *(e.g.,* DNA or RNA detection and/or quantitation) is also performed. While in certain embodiments, all of the operations are performed in the cartridge, in other embodiments, subsets of the various operations are performed in the cartridge, e.g., as described below.

In various embodiments illustrative ,but non-limiting embodiments the sample can comprise any material that is suspected to contain the analyte(s) that are to be detected. In certain embodiments the sample can comprise a quantity of material from a biological, environmental, medical, or patient source in which detection or measurement of target analyte(s) (e.g., polypeptides, small organic molecules, sugars, lectins, carbohydrates, nucleic acids, *etc.)* is sought. In various embodiments the "sample" comprises a specimen or culture *(e.g.,* a microbiological cultures). In certain embodiments the comprises a biological sample from a human or a non-human animal such as a biological fluid, a biological solid *(e.g.,* stool), cells, or tissue. In certain embodiments the sample comprise a biological material taken from a subject *(e.g.,* a human patient or an animal) such as stool, plasma, serum, blood, saliva, amniotic fluid, mucus, urine, pancreatic juice, cerebral spinal fluid, pleural fluid, milk, lymph, sputum, semen, skin biopsies, bronchial and/or tracheal aspirates, needle aspirates, punch biopsies, cryopreserved sections, FFPE sections, and the like. In certain embodiments the sample comprises a liquid or solid food and feed product *(e.g.,* dairy items, vegetables, meat and meat by-products, *etc.).* In certain embodiments the sample comprises an environmental sample such as surface matter, soil, water, and/or vegetation. In certain embodiments the sample can comprise a material suspected of containing a bacterium, virus, protozoan, or other pathogen.

In various embodiments illustrative cartridge-based immuno-PCR using a direct assay involves providing a sample in a sample receiving chamber of a cartridge configured for performing immuno-PCR (e.g., as described below) and using the cartridge to:
1) contact the analyte (if present in the sample) with a capture antibody under conditions where the capture antibody binds the analyte forming a capture antibody/analyte complex;
2) contact the analyte with a detection antibody attached to a signal DNA under conditions where the detection antibody specifically binds to and forms an immunocomplex with the capture antibody/analyte complex;
3) release the immunocomplex or a portion thereof comprising the signal DNA and delivering the immunocomplex or a portion thereof into a temperature controlled channel or chamber; and
4) perform a nucleic acid amplification in the temperature controlled channel or chamber to detect and/or quantify the signal nucleic acid thereby detecting and/or quantifying the analyte.

In certain embodiments illustrative cartridge-based immuno-PCR using an indirect assay involves providing a sample in a sample receiving chamber of a cartridge configured for performing immuno-PCR (e.g., as described below) and using the cartridge to:
1) provide a sample in a sample receiving chamber of a cartridge configured for performing immuno-PCR; and using the cartridge:
2) contact the analyte (if present in the sample) with a capture antibody under conditions where the capture antibody binds the analyte forming a capture antibody/analyte complex;
3) contact the analyte with a detection antibody where the contacting is under conditions where the detection antibody specifically binds to and forms an immunocomplex with the capture antibody/analyte complex;
4) contact the immunocomplex with a label antibody that binds to the detection antibody where the label antibody is attached to a signal DNA and the contacting is under conditions where the label antibody specifically binds to the detection antibody to form a labeled immunocomplex;
5) release the labeled immunocomplex or a portion thereof comprising the signal DNA and delivering the immunocomplex or a portion thereof into a temperature controlled channel or chamber; and
6) perform a nucleic acid amplification in the temperature controlled channel or chamber to detect and/or quantify the signal nucleic acid thereby detecting and/or quantifying the analyte.

In various embodiments the nucleic acid amplification comprises a method selected from the group consisting of polymerase chain reaction (PCR), ligase chain reaction (LCR), ligase detection reaction (LDR), multiplex ligation-dependent probe amplification (MLPA), ligation followed by Q-replicase amplification, primer extension, strand displacement amplification (SDA), hyperbranched strand displacement amplification, multiple displacement amplification (MDA), nucleic acid strand-based amplification (NASBA), and rolling circle amplification (RCA).

The immuno-PCR can be implemented in the cartridge using any of a number of formats. For example, in certain embodimnts, the capture antibody is attached to the wall of a reaction chamber or to a matrix material (e.g., silica) in a chamber in the cartridge and binding of the analyte immobilizes the analyte in said reaction chamber or matrix material. The detection antibody is bound and, in the case of an indirect assay a label antibody is bound, the resulting immunoconjugate or labeled immunoconjugate is washed to remove unbound materials and/or non-specifically bound material and the signal DNA (attached to the detection antibody in a direct assay or attached to a label antibody in an indirect assay) is released, *e.g.,* as described below, for subsequent amplification.

In various embodiments, however, the capture antibody is attached to a particle *(e.g.,* a latex particle, a silica particle, a teflon particle, a noble metal particle *(e.g.,* Au), a magnetic particle, *etc*.)*, e.g.,* as illustrated in Figure 2. In certain embodiments the capture antibody is attached directly to the particle (*e*.*g*., via reaction between functional groups on the particle and the antibody, or the capture antibody is chemically conjugated to the antibody *(e.g.,* through a bifunctional linker *(e.g.,* a heterobifunctional linker)), or the antibody is joined to the particle by a biotin/avidin reaction.

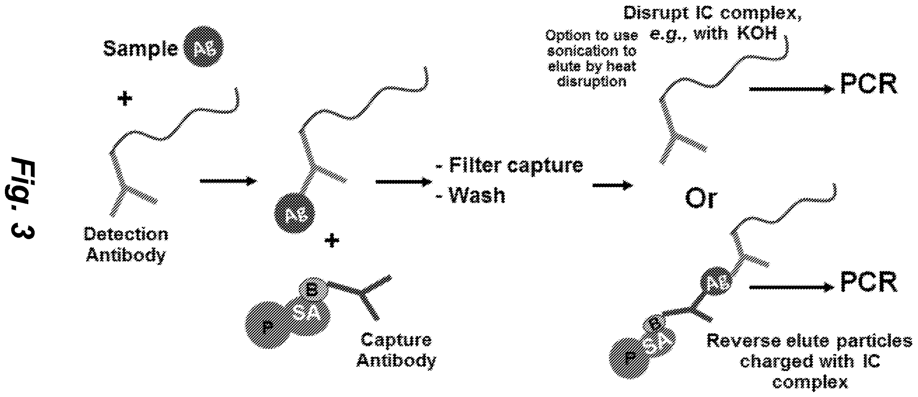

The capture antibody attached to the particle binds an analyte which can then be bound by a detection antibody forming an immunocomplex (or in the case of an indirect assay a labeled immunocomplex). It is noted that Figure 2 illustrates an immunocomplex produced by a direct sandwich assay. After washing the signal DNA is released, *e.g.,* as described below) and amplified for detection *(see, e.g.,* Figure 3).

In certain embodiments the particle is not attached to a surface and the <particle>-<capture Ab>-<analyte>-<detection Ab-Signal DNA> immunocomplex or the <particle>-<capture Ab>-<analyte>-<detection Ab>-<label Ab-Signal DNA> labeled immunocomplex are formed in solution/suspension. The resulting immunocomplex or labeled immunocomplex can then be immobilized, *e*.*g*., by chemical binding of the particle to a substrate such as a matrix material as described herein (e.g., via a biotin/avidin interaction) or the resulting immunocomplex or labeled immunocomplex can be immobilized by simple mechanical entrapment and/or adsorption on a porous substrate such as a matrix material described herein.

Mechanisms of release of the signal DNA depend on the particular coupling scheme used to attach the signal DNA to the detection antibody or, in the case of an indirect assay, to the label antibody. In various embodiments the entire immunocomplex or the entire labeled immunocomplex, or a portion of the immunocomplex or labeled immunocomplex, or a signal DNA or fragment of a signal DNA is released and delivered to a temperature controlled channel or chamber for amplification.

Figure 4, panels A-D, shows some illustrative, but non-limiting, methods of coupling a signal DNA to a detection antibody or to a label antibody. As illustrated in Figure 4, panel A, in certain embodiments the signal DNA can be chemically conjugated to the detection antibody (or label antibody).

Typically a linker is used to chemically conjugate an antibody to a nucleic acid. Generally the linker or comprises a functional group. Functional groups include monofunctional linkers comprising a reactive group as well as multifunctional linkers comprising two or more reactive groups capable of forming a bond with two or more different functional targets. In some embodiments, the multifunctional linkers are heterobifunctional linkers comprising two or more different reactive groups.

Suitable reactive groups include, but are not limited to thiol (-SH), carboxylate (COOH), carboxyl (- COOH), carbonyl, amine (NH₂), hydroxyl (-OH), aldehyde (-CHO), alcohol (ROH), ketone (R₂CO), active hydrogen, ester, sulfhydryl (SH), phosphate (-PO₃), or photoreactive moieties. Amine reactive groups include, but are not limited to *e*.*g*., isothiocyanates, isocyanates, acyl azides, NHS esters, sulfonyl chlorides, aldehydes and glyoxals, epoxides and oxiranes, carbonates, arylating agents, imidoesters, carbodiimides, and anhydrides. Thiol-reactive groups include, but are not limited to *e.g.,* haloacetyl and alkyl halide derivates, maleimides, aziridines, acryloyl derivatives, arylating agents, and thiol- disulfides exchange reagents. Carboxylate reactive groups include, but are not limited to *e*.*g*., diazoalkanes and diazoacetyl compounds, such as carbonyldiimidazoles and carbodiimides. Hydroxyl reactive groups include, but are not limited to *e.g.,* epoxides and oxiranes, carbonyldiimidazole, oxidation with periodate, N,N'- disuccinimidyl carbonate or N-hydroxylsuccimidyl chloroformate, enzymatic oxidation, alkyl halogens, and isocyanates. Aldehyde and ketone reactive groups include, but are not limited to *e*.*g*., hydrazine derivatives for schiff base formation or reduction amination. Active hydrogen reactive groups include, but are not limited to *e*.*g*., diazonium derivatives for mannich condensation and iodination reactions. Photoreactive groups include, but are not limited to *e.g.,* aryl azides and halogenated aryl azides, benzophenones, diazo compounds, and diazirine derivatives.

Other suitable reactive groups and classes of reactions useful in forming chemical conjugates include those that are well known in the art of bioconjugate chemistry. Currently favored classes of reactions for chemical conjugation of antibodies include, but are not limited to nucleophilic substitutions (e.g., reactions of amines and alcohols with acyl halides, active esters), electrophilic substitutions (e.g., enamine reactions), and additions to carbon-carbon and carbon-heteroatom multiple bonds (e.g., Michael reaction, Diels-Alder addition). These and other useful reactions are discussed in, for example, March (1985) Advanced Organic Chemistry, 3rd Ed., John Wiley & Sons, New York, Hermanson (1996) Bioconjugate Techniques, Academic Press, San Diego; and Feeney et al. (1982) Modification of Proteins; Advances in Chemistry Series, Vol. 198, American Chemical Society, Washington, D.C.

In certain embodiments the linker is typically capable of forming covalent bonds to both molecule(s), *e*.*g*., the antibody and the nucleic acid. Particularly suitable linkers are well known to those of skill in the art and include, but are not limited to, straight or branched-chain carbon linkers, heterocyclic carbon linkers, or peptide linkers. In certain embodiments the linkers can be joined to the constituent amino acids of the antibody through their side groups (e.g., through a disulfide linkage to cysteine). However, in certain embodiments, the linkers will be joined to the alpha carbon amino and carboxyl groups of a terminal amino acid on the antibody.

In certain embodiments a bifunctional linker having one functional group reactive with a group on the antibody and another group reactive on the nucleic acid can be used to form the desired conjugate. Alternatively, derivatization can be performed to provide functional groups. Thus, for example, procedures for the generation of free sulfhydryl groups on peptides are also known (See U.S. Pat. No. 4,659,839).

In certain embodiments the linking agent is a heterobifunctional linker comprising two or more different reactive groups. For example, a heterobifunctional linker such as cysteine may comprise an amine reactive group and a thiol-reactive group can interact with an aldehyde on a derivatized peptide. In certain embodiments linkers can be coupled to a lysine. Additional combinations of reactive groups suitable for heterobifunctional linkers include, for example, amine- and sulfhydryl reactive groups; carbonyl and sulfhydryl reactive groups; amine and photoreactive groups; sulfhydryl and photoreactive groups; carbonyl and photoreactive groups; carboxylate and photoreactive groups; and arginine and photoreactive groups. In certain embodiments suitable linkers include, but are not limited to succinimidyl 4-hydrazinonicotinate acetone hydrazone (SANH), or succinimidyl 4-(*N*-maleimidomethyl), cyclohexane-1-carboxylate (SMCC), and the like

Many procedures and linker molecules for attachment of various molecules to peptides or proteins are known *(see, e.g.,* European Patent Application No. 188,256; U.S. Patent Nos. 4,671,958, 4,659,839, 4,414,148, 4,699,784; 4,680,338; 4,569,789; and 4,589,071; and Borlinghaus et al. (1987) Cancer Res. 47: 4071-4075; van Buggenum et al. (2106) Sci. Rep., 6: 22675; Gong et al. (2015) Bioconjug. Chem., 27: 217-227; and the like).

In certain embodiments the linker joining the label DNA to the detection antibody (or to the label antibody) is a non-cleavable linker.

As an alternative to chemical conjugation, the antibody can be joined to the nucleic acid using an avidin/biotin interaction *(see, e.g.,* Figure 4, panel B). Such interactions include, but are not limited to avidin/biotin interactions, streptavidin/biotin interactions, neutravidin/biotin interactions and the like. Avidin biotin interactions are essentially irreversible under relevant chemical conditions.

In certain embodiments, particularly where the attachment of the signal DNA to the antibody is non-cleavable, delivery of the signal DNA to the amplification reaction site *(e.g.,* a temperature-controlled channel or chamber) can be accomplished simply by delivering the entire immunocomplex (or labeled immunocomplex) to the amplification reaction. Thus, for example where the immunocomplex is formed on a particle as described herein, the particle can simply be reverse-eluted from its mechanical entrapment (e.g., in the matrix material). Reversing the flow direction (from the direction that initially delivered the particle-bound immunocomplex will release a sufficient amount of immunocomplex for detection in an amplification reaction.

In certain embodiments the immunocomplex can simply be disrupted to free the detection antibody bearing the signal DNA or the label antibody bearing the signal DNA and the antibody bound signal DNA is delivered to the amplification reaction. Methods of disrupting an antibody/analyte complex are well known to those of skill in the art. such methods include, but are not limited to heating the antibody/analyte complex or subjecting the antibody/analyte complex to basic solution (e.g., KOH). In certain embodiments the antibody/analyte complex can be disrupted using, for example, high salt conditions, denaturing conditions such as SDS/urea (e.g., 8M), glycine^{∗}HCL, and the like.

In certain embodiments the antibody is attached to the signal DNA using a cleavable linker. Numerous cleavable linkers are known to those of skill in the art *(see, e.g.,* U.S. Patent Nos. 4,618,492; 4,542,225, 4,625,014, and the like). Illustrative cleavable linkers include, but are not limited to, acid-labile linkers, base labile linkers, protease cleavable linkers, disulfide linkers, and the like. Acid-labile linkers are designed to be stable at approximately neutral pH, but become unstable and degrade under low pH conditions. Protease-cleavable linkers are designed to be susceptible to various proteases that can be provided in the cartridge to effect such cleavage. One illustrative, but non-limiting, protease cleavable linker comprises a Val-Cit linkage that is rapidly hydrolyzed by various cathepsins. Linkers containing a disulfide bond can readily be cleaved using, for example dithiothreitol (DTT) or tris(2-carboxyethyl)phosphine (TECP).

In one illustrative, but non-limiting embodiments, conjugation fo an antibody to a signal DNA can be accomplished using an alkyne-azide cycloaddition (the Cu-free click reaction), in which the antibody is activated with a dibenzocyclooctyne (DBCO) moiety and subsequently linked covalently with an azide-modified DNA *(see, e.g.,* Gong et al. (2015) Bioconjug. Chem., 27: 217-225). The linker can readily incoproate a protease cleaable linkage, a disulfide linkage , and the like. In another illustrative, but non-limiting approach, antibodies are functionalized with chemically cleavable NHS-s-s-tetrazine. Double-stranded DNA is functionalized with TCO by enzymatic addition of N₃-dATP and coupling to trans-cyclooctene-PEG -dibenzocyclooctyne. Conjugates are then efficiently obtained by mixing the functionalized antibodies and dsDNA at low molar ratios of, *e.g., 1:2. (see, e.g.,* van Buggenum et al. (2016) Sci. Rep., 6: 22675). In certain embodiments the PEG comprising the linker is a PEG12.

Where the antibody is attached to the signal DNA using a cleavable linker the cartridge can contain the reagents *(e.g.,* protease, DTT, acid, base, *etc.)* suitable for cleaving the linker. When the signal is to be released, the cartridge is operated to contact the immunocomplex (or labeled immunocomplex) with the cleavage reagent thereby releasing the signal DNA which can be transported to the amplification reaction, *e.g.,* to the temperature-controlled reaction chamber or channel).

In certain embodiments the signal DNA is attached to a bead which is also attached to the detection antibody or to the label antibody *(see, e.g.,* Figure 4, panel C). Where the antibody or the signal DNA is attached to the bead by a cleavable linker the signal DNA can be released from the particle, or the bead-signal DNA complex can be released from the antibody by cleaving the linker, *e.g.,* as described above. Where the signal DNA and antibody are attached to the bead by non-cleavable means, (e.g., non-cleavable linkers, biotin/avidin interactions, *etc.)* the antibody/analyte complex can be disrupted, *e*.*g*., as described above, thereby releasing the detection antibody-bead-signal DNA moiety or the label antibody-bead-signal DNA moiety which can be delivered to the amplification reaction. In certain embodiments heat can be sufficient to release the signal DNA from the bead.

In certain embodiments, e.g., where the signal DNA is chemically conjugated to the antibody, or where the signal DNA is attached to the antibody by a biotin/avidin interaction, or where the antibody and signal DNA are attached to a bead, a signal DNA sequence can be released by cleavage of a portion of the signal DNA. In certain embodiments the signal DNA can be prepared so that it incorporates a nucleotide that is readily cleaved using a chemical reagent. Thus, for example, U.S. Patent No: 6,610,492 describes nucleic acids comprising any of a number of nucleotides a modified heterocyclic nitrogen base that is readily cleaved. In certain embodiments the nuclei acid is cleaved using a chemical base, particular a base comprising an amine. Illustrative bases include but are not limited to 3-pyrrolidinol, 2-pyrrolidinemethanol, 3-pyrrolidinemethanol, 4-hydroxypiperidine, 4-piperidineethanol, and the like.

In certain embodiments the signal DNA is provided with a nucleotide sequence that is recognized by a restriction endonuclease (a restriction site). Contacting the signal DNA with the corresponding restriction endonuclease cleaves the signal DNA. Typically when the signal DNA is to be cleaved, the signal DNA is designed so that the cleaved fragment has sufficient length and sequence identity to permit amplification and detection.

In another illustrative, but non-liming embodiment, the antibody-signal DNA conjugate can be created using the Tus-*Ter* lock (*see, e.g.,* Morin et al. (2011) Analyst, 136(22): 4815-4821. In this approach, Tus peptide sequence (also known as terminus utilization substance) is fused to an antibody and the signal DNA is provided with a Ter sequence. The Ter sequence is bound by the Tus peptide thereby providing an immunoconjugate. In certain embodiments signal DNA is contacted with the detection antibody or the label antibody after formation of the immunocomplex. In other embodiments, the Tus-Ter immunoconjugation is provided before formation of the immunocomplex.

In another approach, the detection antibody or the label antibody is expressed on the surface of a phage *(e.g.,* a filamentous phage) and the signal DNA is provided inside the phage *(see, e.g.,* Figure 4, panel D). After formation of the immunocomplex or labeled immunocomplex, the signal DNA is released by lysis of the phage using heat and/or lytic reagents. The released signal DNA is then delivered to the amplification reaction.

In various embodiments the amplification of one or more signal DNAs comprises subjecting a reaction mixture containing the signal DNA(s) to amplification conditions, and monitoring a signal *(e.g.,* an optical signal) of an indicator in the reaction mixture. In certain embodiments the signal comprises an optical signal selected from the group consisting of a fluorescent signal, a chemiluminescent signal, an electrochemiluminescent signal, and a colorimetric signal. In certain embodiments the optical signal comprises a fluorescent optical signal generated by a fluorescent indicator. In certain embodiments the fluorescent indicator is a non-specific intercalating dye that binds to double-stranded DNA products, while in other embodiments, the fluorescent indicator comprises a target sequence specific probe. In certain embodiments the target sequence specific probe is selected from the group consisting of a TAQMAN probe, a SCORPION probe, and a MOLECULAR BEACON.

In certain embodiments the immuno-PCR is performed on a plurality of different analytes (e.g., at least 2, or at least 3, or at least 4, or at least 5, or at least 6 different analytes) in the same cartridge. In certain embodiments each analyte comprising said plurality of analytes is derived from the same sample in said cartridge. In certain embodiments the signal DNAs representing each of the analytes comprising the plurality are amplified sequentially in the same temperature controlled channel or chamber. In certain embodiments the chamber is "flushed" and "cleaned" between amplification reactions to reduce or avoid cross-contamination between amplification reactions. Thus, for example, in certain embodiments, the temperature controlled channel or chamber is washed with a wash solution. In certain embodiments after removing the wash solution air is transferred into the temperature controlled channel or chamber and the channel or chamber is heated to a temperature at or above a DNA denaturation temperature (e.g., from about 90°C to about 99°C).

In certain embodiments the signal DNAs representing each of the analytes comprising the plurality are amplified simultaneously each in a different temperature controlled channel or chamber in said cartridge. In certain embodiments the signal DNAs representing each of the analytes comprising said plurality are amplified simultaneously in the same temperature controlled channel or chamber in the cartridge and amplification of each signal DNA provides a different a distinguishable optical signal.

In certain embodiments the cartridge-based immuno-PCR can be performed using one or more blocking agents to reduce or prevent non-specific binding.

Illustrative blocking agents include but are not limited to various polymers, and/or detergents, and/or carbohydrates (e.g., PEG, Pluronics F68/F108/F127 (F68 preferred), PVP, Biolipidure 802 (NOF America), Tween 20 or 80, TEGME (Tre(ethylene glycol) monoethyl ether), TEG (Tegraethylene glycol), and the like) and/or various proteins, and/or surfactants and/or polysaccharides (e.g., casein, BSA, goat IgG, bovine IgG, Stabilcoat (ThermoFisher), iota-carrageenan, dextran sulfate, mouse serum, and the like).

As noted above, the immuno-PCR methods performed in a cartridge as described herein are typically significantly faster and substantially less labor-intensive than traditional immuno-PCR methods. In certain embodiments the method can be completed in about 1 hour or less, or in about 50 minutes or less, or in about 40 minutes or less, or in about 30 minutes or less, or in about 20 minutes or less, or in about 15 minutes or less.

### Immuno-PCR combined with Nucleic Acid Amplification.

In various embodiments the cartridges provided herein provide for nucleic acid analysis (e.g., detection and/or quantitation of a nucleic acid) in addition the immuno-PCR reactions. The ability to perform both an immuno-PCR reaction and a nucleic acid amplification reaction in the same cartridge permits, *inter alia,* an immuno-PCR analysis of one or more target analyte(s) to reflex to a nucleic acid analysis of the same sample, or conversely, a nucleic acid analysis of one or more analytes to reflex to an immuno-PCR assay.

In certain embodiments the nucleic acid analysis is performed on the same sample as then immuno-PCR where the nucleic acid analysis draws the sample from the same sample chamber as the immuno-PCR assay (see, e.g., Figure 10A). In other embodiments, the immuno-PCR analysis can be performed on a sample drawn from a first sample chamber, while a nucleic acid analysis is performed provided on a sample drawn from a second sample chamber (see, e.g., Figure 10B). The latter approach permits different sample processing for samples used in immuno-PCR and samples used in a nucleic acid analysis. Thus, for example, the nucleic acid analysis can involve a DNA isolation and/or purification that is performed prior to placing a sample in the cartridge, or alternatively, can be performed by the cartridge itself. Accordingly, in certain embodiments the sample for nucleic acid analysis is added directly to the reaction cartridge, while in other embodiments, the sample is mixed with one or more reagents. In certain embodiments DNA preparation typically involves preparing substantially isolated DNA. This may involve lysing cells to release DNA, removing particulates and cellular debris, and/or removing protein components to provide a sample comprising substantially pure nucleic acids (e.g., substantially pure DNA and/or a substantially pure combination of DNA and RNA). In one illustrative, but non-limiting, embodiment, the sample *(e.g.,* a tissue sample) is added to a lysis reagent, agitated and then inserted into the cartridge for further processing. Alternatively, all sample processing is performed in the cartridge.

Accordingly in various embodiments, the cartridge-based immuno-PCR methods contemplated herein further comprise amplifying a nucleic acid other than the signal DNA(s) used in the immuno-PCR. In certain embodiments amplifying a nucleic acid other than said signal DNA acid comprises:
binding a nucleic acid from said sample to a matrix material;
washing the bound nucleic acid to provide a washed nucleic acid;
eluting the washed nucleic acid; and
subjecting the washed nucleic acid to an amplification reaction to amplify a target nucleic acid sequence if present in the washed nucleic acid.

In certain embodiments the nucleic acid is bound to the same matrix material used to bind or otherwise immobilize the immuno-PCR immunoconjugate or labeled immunoconjugate. In other embodiments a second and different matrix material (e.g., in a second and different chamber/channel) is used to bind and elute the nucleic acid.

In certain embodiments amplifying a nucleic acid other than the signal DNA is performed on nucleic acid(s) obtained from the same sample in the sample chamber used for said immuno-PCR. In certain embodiments amplifying a nucleic acid other than the signal DNA is performed on nucleic acid(s) obtained from a sample in a sample chamber different than the sample chamber used for the immuno-PCR. In certain embodiments the signal DNA(s) and the nucleic acid(s) other than a signal DNA are amplified sequentially in the same temperature controlled channel or chamber. In certain embodiments the signal DNA is amplified in the same temperature controlled channel or chamber before the amplification of the nucleic acid other than a signal DNA and in other embodiments the signal DNA is amplified after the amplification of the nucleic acid other than a signal DNA. In certain embodiments the temperature controlled channel and/or chamber is washed and/or flushed between an immuno-PCR signal DNA amplificaotin and an amplficiation reaction performed on a nucleic acid other than a signal DNA. In certain embodiments the washing comprises washing the temperature controlled channel or chamber with a wash solution. In certain embodiments the washing and flushing comprises removing the wash solution from the temperature controlled channel or chamber transferring air into the temperature controlled channel or chamber and heating the channel or chamber to a temperature at or above a DNA denaturation temperature (e.g., from about 90°C to about 99°C).

In certain embodiments the signal DNA(s) and the nucleic acid(s) other than a signal DNA are amplified simultaneously each in a different temperature controlled channel or chamber in the cartridge. In certain embodiments the signal DNA(s) and the nucleic acid(s) other than a signal DNA are amplified simultaneously in the same temperature controlled channel or chamber in the cartridge and amplification of the signal DNA(s) and the nucleic acid(s) other than a signal DNA provide different and distinguishable signals (e.g., different and distinguishable optical signals). In certain embodiments the amplification of a nucleic acid other than a signal DNA comprises subjecting a reaction mixture containing the nucleic acid other than a signal DNA to amplification conditions, and monitoring a signal *(e.g.,* an optical signal) of an indicator in the reaction mixture. In certain embodiments the signal comprise an optical signal selected from the group consisting of a fluorescent signal, a chemiluminescent signal, an electrochemiluminescent signal, and a colorimetric signal. In certain embodiments the optical signal is a fluorescent optical signal generated by a fluorescent indicator. In certain embodiments the fluorescent indicator is a non-specific intercalating dye that binds to double-stranded DNA products or a comprises a target sequence specific probe. In certain embodiments the fluorescent indicator comprises a target sequence specific probe is selected from the group consisting of a TAQMAN probe, a SCORPION probe, and a MOLECULAR BEACON.

The foregoing description of cartridge-base immuno-PCR and combined immuno-PCR/nucleic acid analysis methods are illustrative and non-limiting. Using the teaching provided herein numerous other cartridge-based immuno-PCR and combined immuno-PCR/nucleic acid analysis methods will be available to one of skill in the art.

### Cartridge, modules, and systems for Immuno-PCR and Optional Nucleic Acid Analysis.

### Cartridges

In various embodiments cartridges are provided for performing the immuno-PCR methods described herein. In certain embodiments cartridges are provided for performing the immuno-PCR methods described herein as well as amplification of one or more nucleic acids other than the signal DNA(s) used in the immuno-PCR reactions.

In certain illustrative, but non-limiting embodiments the cartridge comprises:
one or more sample receiving chamber(s);
one or more chambers comprising a matrix material that acts as a filter and/or a DNA binding agent;
one or more temperature controlled channel(s) or chamber(s); and
a plurality of chambers containing reagents and/or buffers for performing immuno-PCR, where:
   the plurality of chambers comprises a chamber containing a capture antibody that binds the analyte that is to be detected;
   the plurality of chambers comprises a chamber containing a detection antibody where the detection antibody is optionally attached directly or indirectly to a signal DNA;
   the plurality of chambers comprise one or more chamber(s) containing a PCR master mix;
   the plurality of chambers comprises one or more chambers containing primers for amplifying all or a region of the immuno-PCR signal DNA; and
   the plurality of chambers comprises a chamber containing a probe for detecting all or a region of the immuno-PCR signal DNA(s).

In certain embodiments the PCR master mix, and/or the primers, and/or the probe(s) are in the same chamber. In various embodiments the PCR primers, and/or probes, and/or polymerase in the master mix are provided as beads. In certain embodiments the cartridge contains detection antibodies for the detection of a single analyte, while in other embodiments the cartridge contains detection antibodies for the detection of a plurality of analytes. In certain embodiments the cartridge contains capture antibodies for the capture of a single analyte, while in other embodiments the cartridge contains detection antibodies for the detection of a plurality of analytes. In certain embodiments the plurality of analytes comprises at least 2, or at least 3, or at least 4, or at least 5, or at least 6, or at least 7, or at least 8, or at least 9, or at least 10 different analytes.

In certain embodiments the immuno-PCR cartridge contains one or mor types of capture antibody attached (e.g., chemically conjugated or joined via a biotin/avidin interaction, or immobilized by a magnetic attraction) to the wall or floor of a chamber or channel. of a reaction chamber or to the matrix material. In certain embodiments the immuno-PCR cartridge contains one or more capture antibodies attached to particle(s) (e.g., latex, glass, Teflon, metal *(e.g.* noble metal such as gold), semiconductor, magnetic, *etc.).* In certain embodiments the capture antibody is chemically conjugated to the particle *(e.g.* as described above). In certain embodiments the capture antibody is attached to the particle via a biotin/streptavidin interaction. In certain embodiments the particle ranges in size from about 0.5 µm, or from about 1 µm up to about 10 µm, or up to about 3 µm, or wherein the particle ranges in size from about 1 µm up to about 2.8 µm.

In certain embodiments the plurality of chambers comprising the immuno-PCR cartridge comprise one or more chambers containing one or more blocking agents that can reduce non-specific binding. Illustrative blocking agents include, but are not limtited to various polymers, and/or detergents, and/or carbohydrates (e.g., PEG, Pluronics F68/F108/F127 (F68 preferred), PVP, Biolipidure 802 (NOF America), Tween 20 or 80, TEGME (Tre(ethylene glycol) monoethyl ether), TEG (Tegraethylene glycol), and the like) and/or various proteins, and/or surfactants and/or polysaccharides (e.g., casein, BSA, goat IgG, bovine IgG, Stabilcoat (ThermoFisher), iota-carrageenan, dextran sulfate, mouse serum, and the like).

In certain embodiments the cartridge contains reagents for a direct sandwich assay *(e.g.,* the cartridge contains, *inter alia,* a capture antibody (which may be attached to a particle), and a detection antibody that has an attached signal DNA or that has a moiety *(e.g.,* a Tus protein) configured to attach a signal DNA). In certain embodiments the cartridge contains reagents for an indirect sandwich assay *(e.g.,* the cartridge contains, *inter alia,* a capture antibody (which may be attached to a particle), a detection antibody, and a label antibdoy that has an attached signal DNA or that has a moiety *(e.g.,* a Tus protein) configured to attach a signal DNA). In certain embodiments the signal DNA is chemically conjugated to the detection antibody, or when the label antibody is present, the signal DNA is chemically conjugated to the label antibody. In certain embodiments the signal DNA is chemically conjugated to the antibody through a cysteine, through a lysine, or through a carbohydrate. In certain embodiments the signal DNA is chemically conjugated to the antibody with a linker comprising a C₆ to C₁₈ linker, or a C₆ to C₁₂ linker. In certain embodiments the linker is a heterobifunctional cross-linker. In certain embodiments the signal DNA is chemically conjugated to the antibody with succinimidyl 4-hydrazinonicotinate acetone hydrazone (SANH) or succinimidyl 4-(*N*-maleimidomethyl) cyclohexane-1-carboxylate (SMCC). In certain embodiments the signal DNA is chemically conjugated to the antibody via an azide modification of the DNA and linkage to the antibody through a dibenzocyclooctyne (DBCO) moiety. In certain embodiments the linker comprise a DBCO-PEG-NHS linker.

In certain embodiments the signal DNA is chemically attached to the detection antibody, or where a label antibody is present to the label antibody, with a cleavable linker. Illustrative, cleavable linkers include, but are not limited to a linker containing a cleavable disulfide linkage, a base-cleavable linker, and an acid cleavable linker, a linker containing a protease/peptidase recognition site, or a linker containing a restriction site. In certain embodiments the cleavable linker comprises a disulfide linkage cleavable with DTT. In certain embodiments the cleavable linker additionally comprises a tetrazine.

In certain embodiments the signal DNA is attached to the detection antibody using an avidin/biotin interaction, or when the label antibody is present, the signal DNA is attached to the label antibody using an avidin/biotin interaction.

In certain embodiments the detection antibody, or the label antibody when present, is attached to, *e*.*g*., genetically fused to, a Tus protein and the signal DNA comprises a *Ter* sequence that is recognized and bound by the Tus protein.

In certain embodiments the detection antibody is attached to a bead and the signal DNA is to the same bead, or when the label antibody is present, the label antibody is attached to a bead and the signal DNA is attached to the same bead *(e.g.,* a gold bead). In certain embodiments the antibody and/or the signal DNA are chemically conjugated to the bead *(e.g.,* as described abve), while in other embodiments, the antibody and/or the signal DNA are attached to the bead via a biotin/streptavidin interaction. In certain embodiments the bead comprises a material such as a latex, silica, teflon, a noble metal, a semiconductor material, and a magnetic material. In certain embodiments the bead comprises gold.

In certain embodiments the detection antibody is presented on the surface of a phage *(e.g.,* a phage from a phage display library) and the signal DNA is contained within the phage, or when the label antibody is present, the label antibody is presented on the surface of a phage and the signal DNA is contained within the phage.

In various embodiments, the cartridge is configured to additionally perform an analysis (e.g., amplification and detection and/or quantification) of a nucleic acid other an immuno-PCR signal DNA. In such embodiments, the plurality of chambers can further comprise a chamber containing PCR primers and/or probes for amplifying and detecting a nucleic acid other than the signal nucleic acid.

In certain embodiments, where the target analyte comprises a polypeptide the nucleic acid other than the signal nucleic acid can comprise a nucleic acid encoding the polypeptide or a fragment thereof. In certain embodiments, where the target analyte comprises a polypeptide, the nucleic acid other than the signal nucleic acid can comprise a nucleic acid characteristic of a cell, tissue, or organism that produces the polypeptide.

In certain embodiments the cartridge comprise one or more chambers containing a TAQMAN probe, and/or a SCORPION probe, and/or a MOLECULAR BEACON. In certain embodiments the cartridge comprises one or more chambers containing reagents for TaqMan PCR reactions.

In certain embodiments the cartridge comprises one or more chambers containing one or more fluorescent probes that are markers for amplified signal DNA, and optionally one or more fluorescent probes that are markers for amplified sequence other than amplified signal DNA. In certain embodiments the probes comprise a fluorescent reporter dye and a quencher dye, where the probes provides a signal upon cleavage by the 5' to 3' nuclease activity of Taq DNA polymerase. In certain embodiments the the primers for amplifying a nucleic acid other than the signal nucleic acid, and/or the probe for detecting a nucleic acid other than the signal nucleic acid are provided as beads.

In various embodiments the temperature controlled channel(s) or chamber(s) in the cartridge are thermocycling channel(s) or chamber(s). In various embodiments the matrix material is one that can adsorb, or chemically bind, or mechanically trap an immunocomplex (e.g., a bead-bound immunocomplex or bead-bound labeled immunocomplex). In certain embodiments the matrix material is one that can additionally bind and elute a nucleic acid. In certain embodiments the matrix material comprises a material is selected from the group consisting of glass or silica, an ion exchange resin, and hydroxyapatite.

In various embodiments illustrative, but non-limiting embodiments, the sample receiving chamber(s), the chamber(s) comprising a matrix material, the plurality of chambers containing reagents, and the temperature-controlled heating channel(s) or chamber(s), are selectively in fluid communication. In certain embodiments the sample receiving chamber(s), the chamber(s) comprising a matrix material, the plurality of chambers containing reagents, and the temperature-controlled heating channel(s) or chamber(s), are selectively in fluid communication by microfluidic channels and valves.

In certain embodiments of the cartridge the sample receiving chamber, the chamber comprising a matrix material, the plurality of chambers containing reagents, and the temperature-controlled heating channel or chamber or a port into the temperature controlled channel or chamber, are disposed around a central valve and selectively in fluid communication with a channel in the central valve, wherein the central valve is configured to accommodate a plunger that is capable of drawing fluid into or out of a chamber in fluid communication with the central valve.

In various embodiments the cartridge is configured so that, when in use, the cartridge comprises a chamber containing a sample, a chamber containing the detection antibody, a chamber containing the capture antibody, a chamber containing a wash buffer, and a chamber containing a PCR master mix for amplifying the signal DNA. In certain embodiments the cartridge, when in use, comprises a chamber containing a sample, a chamber containing the detection antibody, a chamber containing the capture antibody, a chamber contain a wash buffer, a chamber containing KOH, a chamber containing HEPES or Tris-HCl, and a chamber to receive waste. In certain embodiments the cartridge, when in use, comprises a chamber containing a sample, a chamber containing the detection antibody in PBS at about pH 7.25, a chamber containing the capture antibody in PBS at about pH 7.25, a chamber contain a PBS wash buffer, a chamber containing KOH, a chamber containing HEPES at about pH 8.25 or Tris-HCL at about pH 7.4, and a chamber to receive waste.

In various embodiments the cartridge is configured so that the sample receiving chamber, said column(s), the plurality of chambers, and the temperature controlled channel or chamber, are selectively in fluid communication. In certain embodiments the selective fluid communication is provided by microfluidic channels and valves. In certain embodiments the seelctive fluid communction is provided by providing the sample receiving chamber, said column(s), said plurality of chambers, the heating channel or chamber or a port into the heating channel or chamber, disposed around a central valve and selectively in fluid communication with a channel in said central valve.

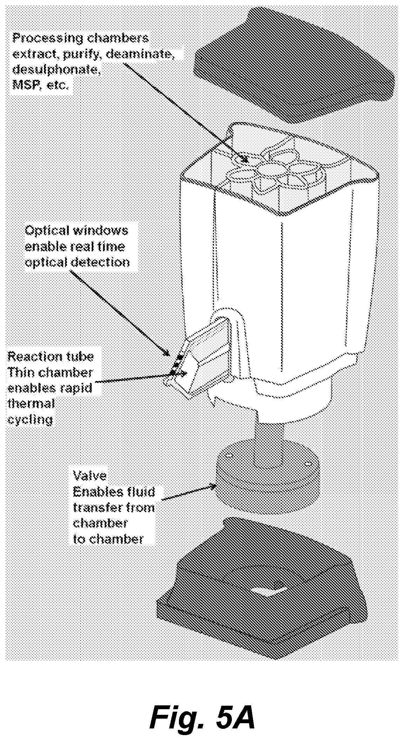

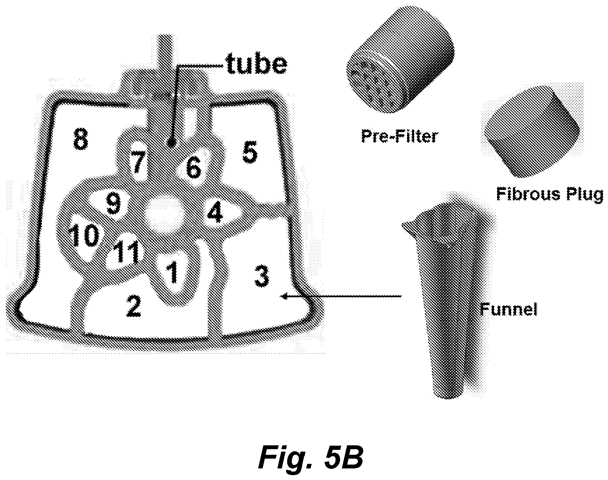

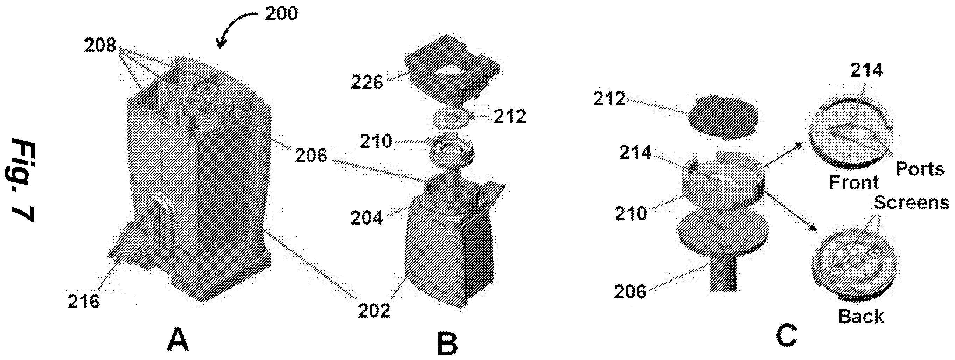

Figures 5A, 5B, 6A, 6B and 7 illustrate a cartridge suitable for practice of the methods described herein. The illustrated cartridges are based on the GENEXPERT^{®} cartridige (Cepheid, Inc., Sunnyvale, CA). As shown in Figure 7, panel A the cartridge 200 comprises a cartridge body 202 containing a plurality of reagent and/or buffer chambers 208. The chambers are disposed around a central syringe barrel 206 that is in fluid communication with a valve body **210** (panel B and Figure 5B) and that is sealed with a gasket **204.** The valve body **210** can comprise a cap **212** and the entire cartridge body can be supported on a cartridge base **226.** The cartridge typically contains one or channels or cavities (chamber(s)) **214** that can contain a matrix material as described herein that can function to immobilize an immuno-PCR immunocomplex and, optionally to bind and elute a nucliec acid. In various embodiments the cartridge further comprises one or more temperature controlled channels or chambers **216** that can, in certain embodiments, function as thermocycling chambers. A "plunger" not shown can be operated to draw fluid into the syringe barrel **206** and rotation of the syringe barrel **206** and assciated valve body **210** provides selective fluid communication between the various reagent chambers **208** and channels, reaction chamber(s), and the temperature controlled channels or chambers. Thus, the various reagent chambers **208,** reaction chambers, matrix material(s), and temperature controlled chambers or channles are selectively in fluid communiction by rotation of the plunger and reagent movement (e.g., chamber loading or unloading) is operated by the "syringe" action of the plunger.

As shown in Figure 5A, in certain embodiments, the cartridge provides optical windows to provide real-time detection of, *e*.*g*., amplification products, base identity in sequencing operations, and the like.

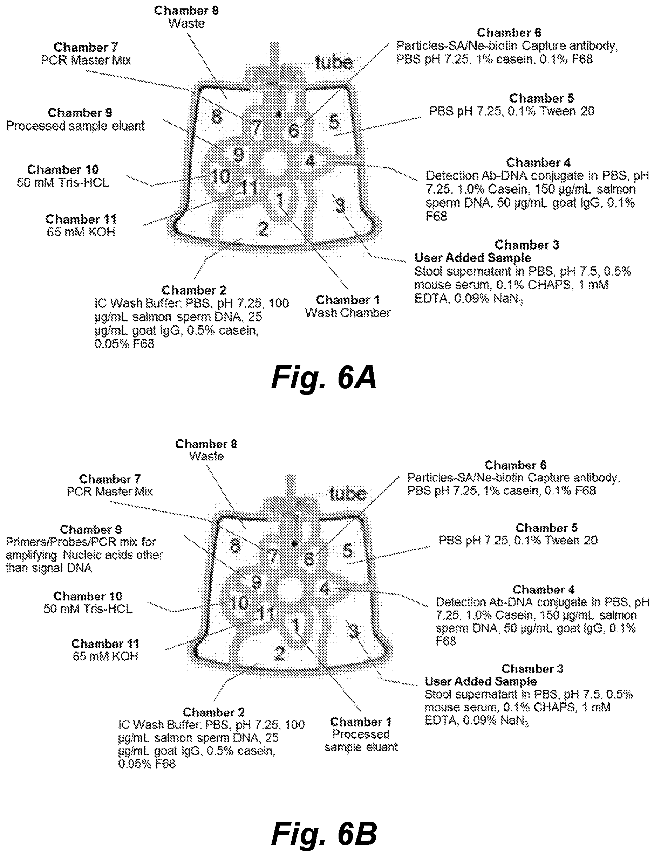

While the methods described above (and in Example 1, see, e.g., Figures 6A and 6B) are described with respect to specific chambers in the GENEXPERT^{®} cartridge, it will be recognized that the particular reagent/chamber assignments can be varied depending on the particularities of the immuno-PCR and/or additional nucleic acid detection/quantification. It will also be recognized that in certain embodiments, variants of the GENEXPERT^{®} cartridge are also contemplated. Such variants can include, but are not limited to, more reagent chambers or fewer reagent chambers and/or different sized chambers, two (or more) sample receiving chambers, two (or more) temperature controlled channels or chambers, stacked cartridges (providing control of two cartridges by one module), and the like.

### Reaction modules.

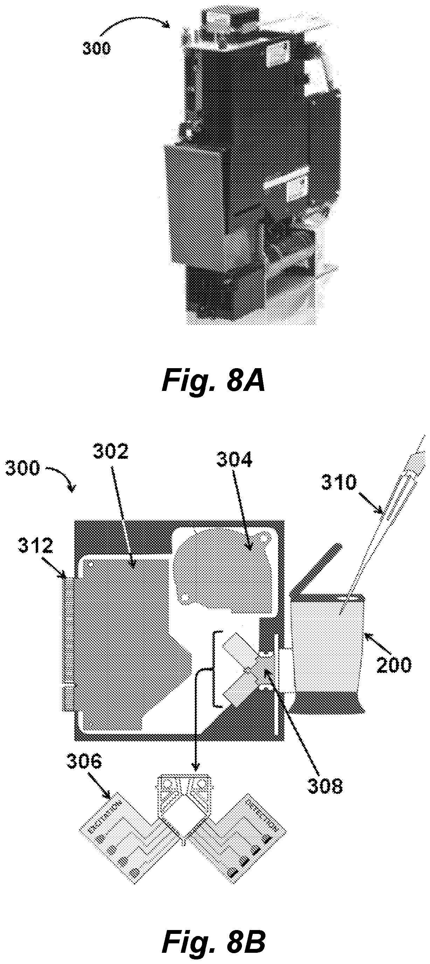

In certain embodiments the cartridge **200** is configured for insertion into a reaction module **300,** *e.g.,* as shown in Figure 8A. As illustrated in Figure 8B the module is configured to receive the cartridge **200.** In certain embodiments the reaction module provides heating plates **308** to heat the temperature controlled chamber or channel. The module can optionally additionally include a fan **304** to provide cooling where the temperature controlled channel or chamber is a thermocycling channel or chamber. Electronic circuitry **302** can be provided to pass information (e.g., optical information) top a computer for analysis. In certain embodiments the module can contain optical blocks **306** to provide excitation and/or detection of one or more *(e.g.,* 1, 2, 3, 4, or more) optical signals representing, *e*.*g*., signal DNAs amplified for various immuno-PCR targets and/or various amplified nucleic acids other than imnmuno-PCR signal DNA(s).. In various embodiments an electrical connector **312** can be provided for interfacing the module with a system (*e*.*g*. system cotroller or with a discrete analysis/controller unit. As illustrated, in Figure 8B the sample can be introduced into the cartridge using a pipette **310.**

In certain embodiments, the module also contains a controller that operates a plunger in the syringe barrel and the rotation of the valve body.

### Systems.

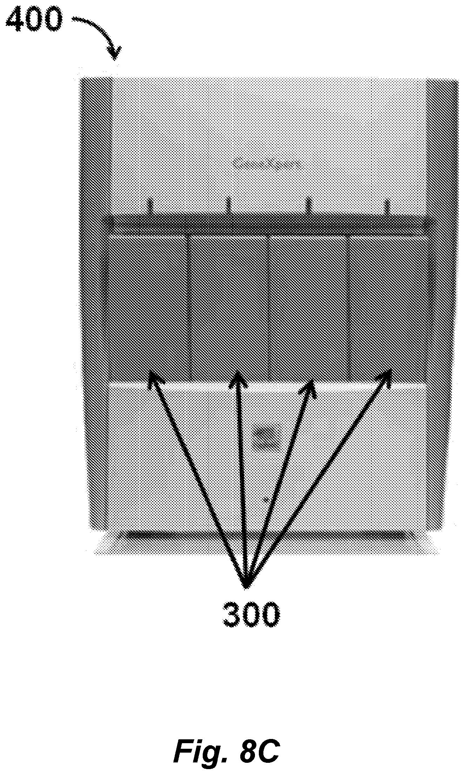

In certain embodiments a system (e.g., a processing unit) is provided. One illustrative, but non-limiting embodiment is shown in Figure 8C. In certain embodiments, the processing unit comprises an enclosure configured to contain one or more sample processing modules where each processing module is configured to hold and operate a removable cartridge as described herein. In certain embodiments the system is configured to operate the sample processing modules to perform an immuno-PCR assay for one or more target analytes and optionally to determine the level of one or more target RNA/DNA sequences (other than immuno-PCR signal DNA(s)) within a corresponding removable sample cartridge. Typically the processing on a sample within the corresponding removable sample cartridge involves operating the cartridge to perform a method as described herein. In certain embodiments the system is configured to contain one sample processing module. In certain embodiments the system is configured to contain at least two sample processing modules, or at least 4 sample processing modules, or at least 8 sample processing modules, or at least 12 sample processing modules, or at least 16 sample processing modules, or at least 20 sample processing modules, or at least 24 sample processing modules, or at least 28 sample processing modules, or at least 32 sample processing modules, or at least 64 sample processing modules, or at least 128 sample processing modules. In certain embodiments the system provides a user interface that allows the user input operational instructions and/or to monitor operation of the cartridges to determine the presence and/or quantity of one or more immuno-PCR analytes and/or the presence and/or quantity of one or more nucleic acids that are not immuno-PCR signal DNAs.

While the methods described herein are described primarily with reference to the GENEXPERT^{®} cartridge by Cepheid Inc. (Sunnyvale, CA) *(see, e.g.,* Figure 5A), it will be recognized, that in view of the teachings provided herein the methods can be implemented on other cartridge/microfluidic systems. Such cartridge/microfluidic systems can include, for example microfluidic systems implemented using soft lithography, micro/nano-fabricated microfluidic systems implemented using hard lithography, and the like.

### Detection/Quantification of signal DNA(s) and/or Nucleic Acids Other Than Immuno-PCR DNA(s).

In various embodiments, the signal DNA(s) from immuno-PCR reactions are amplified for detection and/or quantification. In certain embodiments nucleic acids *(e.g.,* DNA, mRNA, *etc.)* other than immuno-PCR signal DNAs *(e.g.,* from immuno-PCR reactions run in the same cartridge) are additionally amplified for detection and/or quantification. In certain embodiments the amplification comprise any of a number of methods including, but not limited to polymerase chain reaction (PCR), ligase chain reaction (LCR), ligase detection reaction (LDR), multiplex ligation-dependent probe amplification (MLPA), ligation followed by Q-replicase amplification, primer extension, strand displacement amplification (SDA), hyperbranched strand displacement amplification, multiple displacement amplification (MDA), nucleic acid strand-based amplification (NASBA), rolling circle amplification (RCA), and the like.

In illustrative, but non-limiting embodiments, the amplification reaction may produce an optical signal that is proportional to the amount of amplified target nucleic acid *(e.g.,* signal DNA). Illustrative optical signals include, but are not limited to a fluorescent signal, a chemiluminescent signal, an electrochemiluminescent signal, a colorimetric signal, and the like. In certain embodiments the optical signal is a fluorescent optical signal generated by a fluorescent indicator. In certain embodiments the fluorescent indicator is a non-specific intercalating dye that binds to double-stranded DNA products, while in certain other embodiments, the fluorescent indicator comprises a target sequence specific probe *(e.g.,* a TAQMAN^{®} probe, a SCORPION^{®} probe, a MOLECULAR BEACON^{®}, and the like).

Single immuno-PCR reactions, or multiple immuno-PCR reactions run sequentially (or simultaneously in separate temperature controlled channels or chambers) can also use the same detectable label since sequentially run immuno-PCR signal DNAs are analyzed sequentially and the simultaneous immuno-PCR signal DNAs are distinguished by the occurrence in different temperature controlled channels or chambers. The same holds true when the cartridge is additionally used for analysis/amplification of one or more target nucleic acids (other than the immuno-PCR signal DNAs). Thus, the signal produced by this amplification can be distinguished from other amplification products because it is not run at the same time and/or because it is run in a different reaction channel/chamber.

However where multiple immuno-PCR products are run simultaneously in same chambers and/or the nucleic acid amplification (for a nucleic acid other than an immuno-PCR signal DNA) is also run simultaneously in the same chamber the reaction products of for each analysis are typically detected and/or quantified by the use of different and distinguishable labels.

In certain embodiments amplification products (amplified signal DNA, and amplified nucleic acid from nucleic acid analysis when performed) can be detected using methods well known to those of skill in the art. In certain embodiments the amplification is a straightforward simple PCR amplification reaction. In certain embodiments, however, a nested PCR reaction is used to the immuno-PCR signal DNA target(s) and amplified nuclieic acid from the nucleic acid analysis when performed.

In various embodiments, multiplexed PCR assays are contemplated, particulary where it is desired to analyse multiple immuno-PCR analytes in the same amplification reaciton, and/or where it is desired to analyse products of the nucleic acid analsyiss in the same amplification reaction as the immuno-PCR analyses. In certain embodimens in such multiplexed amplification reactions, each probe *(e.g.,* for each specific analyte) has its own specific dye/fluor so that it is detectable independently of the other probes.

In certain embodiments, ttypically, for signal generation, the probes used in various amplification reactions utilize a change in the fluorescence of a fluorophore due to a change in its interaction with another molecule or moiety brought about by changing the distance between the fluorophore and the interacting molecule or moiety for detection and/or quantification of the amplified product. Alternatively, other methods of detecting a polynucleotide in a sample, including, but not limited to, the use of radioactively-labeled probes, are contemplated.

Fluorescence-based assays typically rely for signal generation on fluorescence resonance energy transfer, or "FRET", according to which a change in fluorescence is caused by a change in the distance separating a first fluorophore from an interacting resonance energy acceptor, either another fluorophore or a quencher. Combinations of a fluorophore and an interacting molecule or moiety, including quenching molecules or moieties, are known as "FRET pairs." The mechanism of FRET-pair interaction typically requires that the absorption spectrum of one member of the pair overlaps the emission spectrum of the other member, the first fluorophore. If the interacting molecule or moiety is a quencher, its absorption spectrum typically overlaps the emission spectrum of the fluorophore (s*ee, e.g.,* Stryer (1978) Ann. Rev. Biochem. 47: 819-846; Selvin (1995) Meth. Enzymol. 246: 300-335; and the like). Efficient FRET interaction is typically achieved when the absorption and emission spectra of the pair have a large degree of overlap. The efficiency of FRET interaction is linearly proportional to that overlap. Typically, a large magnitude of signal (*i.e.,* a high degree of overlap) is desired. FRET pairs, including fluorophore-quencher pairs, are therefore typically chosen on that basis.

A variety of labeled nucleic acid hybridization probes and detection assays that utilize FRET and FRET pairs are known. One such scheme is described by Cardullo et al. (1988) Proc. Natl. Acad. Sci. USA, 85: 8790-8794, and in Heller et al. EP 0070685. This scheme uses a probe comprising a pair of oligodeoxynucleotides complementary to contiguous regions of a target DNA strand. One probe molecule contains a fluorescent label, a fluorophore, on its 5' end, and the other probe molecule contains a different fluorescent label, also a fluorophore, on its 3' end. When the probe is hybridized to the target sequence, the two labels are brought very close to each other. When the sample is stimulated by light of an appropriate frequency, fluorescence resonance energy transfer from one label to the other occurs. FRET produces a measurable change in spectral response from the labels, signaling the presence of targets. One label could be a "quencher," which can be, *inter alia,* an interactive moiety (or molecule) that releases the accepted energy as heat.

Another type of nucleic acid hybridization probe assay utilizing a FRET pair is the "TAQMAN^{®}" assay described, *e.g*., U.S. Patent Nos. 5,210,015, and 5,538,848. The probe is typically a a single-stranded oligonucleotide labeled with a FRET pair. In a TAQMAN^{®} assay, a DNA polymerase releases single or multiple nucleotides by cleavage of the oligonucleotide probe when it is hybridized to a target strand. That release provides a way to separate the quencher label and the fluorophore label of the FRET pair.

In certain embodiments non-FRET fluorescent probes, such as those described in, *e.g.,* Tyagi *et al.,* U.S. Pat. No. 6,150,097 can also be used. For example, the Tiyagi *et al.* patent describes how changes in the absorption spectra of the label pair can be used as a detectable signal as an alternative to change in fluorescence. When change in absorption is utilized, the label pair may include any two chromophores, that is, fluorophores, quenchers and other chromophores. The label pair may even be identical chromophores.

In some embodiments, dyes and other moieties, such as quenchers, are introduced into primers and/or probes used in the methods and cartridges described herein. In certain embodiments such dyes and quenchers include, but are not limited to dyes (fluors) suitable for use as FRET probes. In certain embodiments the dyes and/or quenchers comprise modified nucleotides. A "modified nucleotide" refers to a nucleotide that has been chemically modified, but still functions as a nucleotide. In some embodiments, the modified nucleotide has a chemical moiety, such as a dye or quencher, covalently attached, and can be introduced into a polynucleotide, for example, by way of solid phase synthesis of the polynucleotide. In some embodiments, the modified nucleotide includes one or more reactive groups that can react with a dye or quencher before, during, or after incorporation of the modified nucleotide into the nucleic acid. In some embodiments, the modified nucleotide is an amine-modified nucleotide, *i.e.,* a nucleotide that has been modified to have a reactive amine group. In some embodiments, the modified nucleotide comprises a modified base moiety, such as uridine, adenosine, guanosine, and/or cytosine. In some embodiments, the amine-modified nucleotide is selected from 5-(3-aminoallyl)-UTP; 8-[(4-amino)butyl]-amino-ATP and 8-[(6-amino)butyl]-amino-ATP; N6-(4-amino)butyl-ATP, N6-(6-amino)butyl-ATP, N4-[2,2-oxy-bis-(ethylamine)]-CTP; N6-(6-Amino)hexyl-ATP; 8-[(6-Amino)hexyl]-amino-ATP; 5-propargylamino-CTP, 5-propargylamino-UTP. In some embodiments, nucleotides with different nucleobase moieties are similarly modified, for example, 5-(3-aminoallyl)-GTP instead of 5-(3-aminoallyl)-UTP. Many amine modified nucleotides are commercially available from, *e.g.,* Applied Biosystems, Sigma, Jena Bioscience and TriLink. An illustrative, but non-limiting list of suitable fluors is shown in Table 1.

**Table 1. Illustrative, but non-limiting fluorophores (fluorescent labels) for use in the primers and/or probes described herein.**

| **Dye** | **Absorbance Wavelength** | **Emission Wavelength** |
|---|---|---|
| Alexa fluor | 345 | 442 |
| Alexa fluor 430 | 430 | 545 |
| Alexa fluor 488 | 494 | 517 |
| Alexa fluor 532 | 530 | 555 |
| Alexa fluor 546 | 556 | 573 |
| Alexa fluor 555 | 556 | 573 |
| Alexa fluor 568 | 578 | 603 |
| Alexa fluor 594 | 590 | 617 |
| Alexa fluor 633 | 621 | 639 |
| Alexa fluor 633 | 650 | 668 |
| Alexa fluor 660 | 663 | 690 |
| Alexa fluor 680 | 679 | 702 |
| Allophycocyanin | 650 | 660 |
| Aminocoumarin | 350 | 445 |
| Cy2 | 490 | 510 |
| Cy3 | 550 | 570 |
| Cy3.5 581 | 581 | 596 |
| Cy5 | 650 | 670 |
| Cy5.5 | 675 | 694 |
| Cy7 | 743 | 770 |
| FAM | 495 | 516 |
| Fluorescein FITC | 495 | 518 |
| HEX | 535 | 556 |
| Hydroxycoumarin | 325 | 386 |
| Methoxycoumarin | 360 | 410 |
| Red 613 | 480;565 | 613 |
| Rhodamine Red-X | 560 | 580 |
| Rox | 575 | 602 |
| R-phycoerythrin (PE) | 480;565 | 578 |
| Tamara | 565 | 580 |
| Texas Red | 615 | 615 |
| TRITC | 547 | 572 |
| TruRed | 490;675 | 695 |

If the assay is designed to detect one target DNA sequence (*e.g*., one immuno-PCR signal DNA) then only one fluorescent hybridization probe needs to be used and, in certain embodiments, FAM, TET, or HEX (or one of their alternatives listed in Table 2) will be a good fluorophore to label the probe. These fluorophores can readily be excited and detected in various spectrofluorometric thermal cyclers. In addition, because of the availability of phosphoramidites derivatives of these fluorophores and the availability of quencher-linked control- pore glass columns, fluorescent hybridization probes with these labels can be entirely synthesized in an automated DNA synthesis process, with the advantage of relatively less expensive and less labor intensive probe manufacture.

**Table 2. Additional illustrative fluorophore labels for fluorescent hybridization probes.**

| **Fluorophore** | **Alternative Fluorophore** | **Excitation (nm)** | **Emission (nm)** |
|---|---|---|---|
| Cy3³ | NED², Quasar 570¹, Oyster 556⁴ | 550 | 570 |
| Cy5³ | LC red 670⁵, Quasar 670¹, Oyster 645⁴ | 650 | 670 |
| HEX | JOE, VIC ^{B}, CAL Fluor Orange 560¹ | 535 | 555 |
| LC red 640⁵ | CAL Fluor Red 635 ^{A} | 625 | 640 |
| LC red 705⁵ | Cy5.5³ | 680 | 710 |
| ROX | LC red 610⁵, CAL Fluor Red 610¹ | 575 | 605 |
| TET | CAL Fluor Gold 540¹ | 525 | 540 |
| Texas red | LC red 610⁵, CAL Fluor Red 610¹ | 585 | 605 |
| TMR | CAL Fluor Red 590¹ | 555 | 575 |
| ¹⁾CAL and Quasar fluorophores are available from Biosearch Technologies; ²⁾VIC and NED are available from Applied Biosystems; ³⁾Cy dyes are available from Amersham Biosciences; ⁴⁾Oyster fluorophores are available from Integrated DNA Technologies; and ⁵⁾LC (Light Cycler) fluorophores are available from Roche Applied Science. | | | |

In certain embodiments, multiple targets are detected in a single multiplex reaction, e.g., different signal DNAs for different immuno-PCR reactions, and/or amplification produces for an amplification reaction run for a nucleic acid other an an immuno-PCR signal DNA. In some embodiments, each probe that is targeted to a different amplification product is spectrally distinguishable (detectably different) from the other probes utilized in the multiplex reaction. Probe combinations suitable for multiplex detection are known to those of skill in the art. For example, illustrative combinations of detectably different fluorphores in four target multiplex systems include, but are not limited to:
1) FAM, TMR, Texas red, and Cy5;
2) FAM, TET, TMR, and Texas Red;
3) FAM, HEX, Texas red, and Cy5; and
4) FAM, Cy3, Texas red, and Cy5.

An illustrative combination of detectably different fluorphores in a five target multiplex systems is FAM, TET, TMR, Texas Red, and Cy5. Illustrative combinations of detectable different fluorophores in a six target multiplex system include, but are not limited to:
1) FAM, TET, HEX, TMR, ROX, and Texas red; and
2) FAM, HEX, LC red 610, LC red 640, LC red 670, and LC red 705.

It will be recognized that these combinations of fluorophores are illustrative and non-limiting and numerous other fluorophores will be available to those of skill in the art.

As noted above, for the design of fluorescent hybridization probes that utilize fluorescence resonance energy transfer (FRET), fluorophore-quencher pairs that have sufficient spectral overlap should be chosen. Fluorophores with an emission maximum between 500 and 550 nm, such as FAM, TET and HEX, are best quenched by quenchers with absorption maxima between 450 and 550 nm, such as dabcyl, BHQ-1, and the like (*see, e.g.,* Table 3 for illustrative quencher labels). Fluorophores with an emission maximum above 550 nm, such as rhodamines (including TMR, ROX and Texas red) and Cy dyes (including Cy3 and Cy5) are effectively quenched by quenchers with absorption maxima above 550 nm (including BHQ-2).

For the design of fluorescent hybridization probes that utilize contact quenching, any non-fluorescent quencher can serve as a good acceptor of energy from the fluorophore. For example, Cy3 and Cy5 are effectively quenched by the BHQ-1 and BHQ-2 quenchers.

**Table 3. Illustrative quencher labels for fluorescent hybridization probes.**

| **Quencher** | **Absorption Maximum (nm)** |
|---|---|
| BHQ-1⁴ | 534 |
| BHQ-2⁴ | 580 |
| BHQ-3⁴ | 670 |
| Dabcyl | 475 |
| DDQ-I¹ | 430 |
| DDQ-II¹ | 630 |
| Eclipse² | 530 |
| Iowa Black FQ³ | 532 |
| Iowa Black RQ³ | 645 |
| QSY-21⁵ | 660 |
| QSY-7⁵ | 571 |
| ¹⁾DDQ or Deep Dark Quenchers are available from Eurogentec; ²⁾Eclipse quenchers are available from Epoch Biosciences; ³⁾Iowa quenchers are available from Integrated DNA Technologies; ⁴⁾BHQ or Black Hole quenchers are available from Biosearch Technologies; and ⁵⁾QSY quenchers are available from Molecular Probes. | |

In certain embodiments nucleotides can quench the fluorescence of fluorophores, with guanosine being the most efficient quencher, followed by adenosine, cytidine and thymidine. In general, fluorophores with an excitation wavelength between 500 and 550 nm are quenched more efficiently by nucleotides than fluorophores with longer excitation wavelengths. In designing fluorescent hybridization probes, it can be desirable to avoid placing a fluorophore label directly next to a guanosine, to ensure higher fluorescence signals from the fluorophore.

The stabilizing effect of some fluorophore-quencher pairs that interact by contact quenching can have important consequences for the design of hybridization probes (*see, e.g.,* Marras et al. (2002) Nucleic Acids Res. 30: e122; Johansson et al.(2002) J. Am. Chem. Soc. 124: 6950-6956). For example, it has been observed that hybridization probes labeled with a fluorophore quenched by either BHQ-1 or BHQ-2 show an increase in hybrid melting temperature of about 4°C, compared to hybridization probes with the same probe sequence, but labeled with fluorophores quenched by dabcyl. It is also noted that strong affinity has been observed between the Cy dyes, Cy3 and Cy5, and the Black Hole quenchers, BHQ-1 and BHQ-2.

In view of the foregoing and the Examples and teachings provided herein, numerous primer/probe combinations will be available for use in the methods and cartridges described herein.

### Targets/Analytes for Immuno-PCR Detection/Quantification and Optional Nucleic Acid Analysis.

The immuno-PCR cartridges and methods described herein find use in a wide number of contexts. For example, immuno-PCR can be used to identify the presence of, and/or quantify any of a number of organisms or microorganisms including, but not limited to viruses, bacteria, fungi, plants, algae, protozoans, and the like including, but not limited to, any pathogenic forms/species/strains of such organisms or microorganisms. In certain embodiments the immuno-PCR can be used to identify toxins produced by any of these microorganism including. In various embodiments the immuno-PCR devices and methods described herein can be used to identify various cancers or cancer markers and/or to identify cancer cells or microorganisms that show drug resistance (*e.g*., MRSA, drug-resistant tuberculosis, doxorubicin or gentamicin resistant cancer cells, and the like). The immuno-PCR assays find use, *inter alia,* in the medical field as well as in environmental studies *(e.g.,* to screen water, and soil contaminants), and in the agriculture field *(e.g.,* to screen beef, poultry, various food crops, and the like).

Accordingly, the cartridge is loaded *(e.g.,* or configured to be loaded with) with a sample comprising a material from a biological, environmental, medical, or patient source. In various embodiments sample comprises an environmental sample selected from the group consisting of surface matter, soil, water, vegetation, and an industrial sample. In certain embodiments the sample comprises a food or agriculture sample, selected from the group consisting of meat, meat products, avian or avian products, milk or milk products, and farm crops, and organic waste. In certain embodiments the sample comprises a biological sample selected from the group consisting of a culture, blood, saliva, cerebral spinal fluid, urine, stool, bronchial aspirates, tracheal lavage, pleural fluid, milk, lymph, sputum, semen, needle aspirates, punch biopsies, surgical biopsies, cryopreserved sections, FFPE sections, and the like.

In certain embodiments the immuno-PCR cartridges and methods of use thereof are utilized to detect and/or quantify various pathogenic bacteria and/or viruses or toxins produced by such pathogenic bacteria and/or viruses, and thereby diagnose and/or characterize a disease state and facilitate selection of an appropriate therapeutic regimen. In this regard, it is noted that immuno-PCR has been used to detect and/or quantify a number of viruses, bacteria, and bacterial toxins, *e.g*., as identified in Table 4. In addition, immuno-PCR has been used to detect prion proteins (*see,* Table 4) and can thus be used to detect prion diseases (*e.g.*, bovine or human spongiform encephalopathy), and/or to screen animal produces for prion infection. Using the teaching provided herein and in the references shown in Table 4, the various described immuno-PCR assays can readily be implemented on cartridges described herein.

**Table 4. Illustrative targets (analytes/antigen) that have been detected using immuno-PCR.**

| **Viral antigens** | | |
|---|---|---|
| | Hepatitis B | Maia et al. (1995) J. Virol. Meth. 52: 273-286; |
| | surface antigen (HbsAg) | Wacker et al. (2007) Biochem. Biophys. Res. Commun. |
| | | 357: 391-396 |
| | Bovine herpesvirus 1 antigen | Mweene et al. (1996) J. Clin. Microbiol. 34: 748-750 |
| | NSP-1 of equine influenza virus | Ozaki et al. (2001) Jpn. J. Vet. Res. 48: 187-195 |
| | Respiratory syncytial virus (RSV) surface protein | Perez et al. (2013) Meth. Mol. Biol. 1026: 93-110. |
| | Purified Foot and Mouth Disease (FMDV) | Ding et al. (2011) Virol. J. 8: 148 |
| | H5N1 avian influenza virus (AIV) | Deng et al. (2011) Mol. Biol. Rep. 38: 1941-1948; |
| | | Deng et al. (2011) Vet. Immunol. Immunopathol. 141: 183-189. |
| | HIV P24 antigen | Barletta et al. (2004) Am. J. Clin. Pathol. 122: 20-27; |
| | | Barletta et al. (2009) J. Virol. Meth. 157: 122-132 |
| | Norovirus capsid | Tian and Mandrell (2006) J. Appl. Microbiol. 100; 564-574 |
| | Rotavirus | Adler et al. (2005) Biochem. Biophys. Res. Commun. 333: 1289-1294 |
| | Equine influenza | Ozaki et al. (2001) J. Vet. Res. 48: 187-195 |
| | Hantavirus nucleocapsid protein | Chen et al. (2009) J. Immunol. Meth. 346: 64-70; Guo et al. (2006) Nucleic Acids Res. 34: e62 |
| | Avian influenza virus | Deng et al. (2010) Mol. Biol. Rep. 38: 1941-1948 |
| **Bacterial antigens** | | |
| | *Pasteurella piscicida* | Liang et al. (2003) J. Immunol. Meth. 279: 101-110; |
| | | Kakizaki, E. et al. (1996) Lett. Appl. Microbiol. 23: 101-103 |
| | Group A Streptococcus | Liang et al. (2003) J. Immunol. Meth. 279: 101-110; Barletta (2006) Mol. Aspects Med. 27: 224-253 |
| | *Escherichia coli* β-glucuronidase (GUD) | Chang and Huang (1997) J. Immunol. Meth. 208: 35-42 |
| | *Staphylococcus aureus* | Huang and Chang (2004) Clin. Chem. 50: 1673-1674 |
| | *Bacteroides fragilis* | Zhang and Pan (1998) Anaerobe 4: 189-196 |
| | *Streptococcus pyogenes* group A | Liang et al. (2003) J. Immunol. Meth. 279: 101-110. |
| | *Staphylococcus aureus* protein A | Huang and Chang (2004) Clin. Chem. 50: 1673-1674 |
| | *X. fastidiosa* 9a5c bacterial strain | Peroni et al. (2008) J. Microbiol. Meth. 75: 302-307 |
| | *Y. pestis* in dental pulp | Malou et al. (2012) PLoS ONE 7: e31744 |
| | *M. tuberculosis* ESAT-6, CFP-10, CFP-21 and MPT-64 antigens | Mehta et al. (2012) FEMS Immunol. Med. Microbiol. 66: 20-36 |

| **Bacterial toxins** | | |
|---|---|---|
| | Shiga toxin 2 from Shiga toxin- | Zhang et al. (2008) J. Clin. Microbiol. 46: 1292-1297; |
| | producing *Escherichia coli* (STEC) | He et al. (2011) Appl. Environ. Microbiol. 77: 3558-3564. |
| | *Clostridium botulinum* neurotoxin A | Chao et al. (2004) Toxicon, 43: 27-34 |
| | *Staphyloccus aureus* enterotoxin A | Rajkovic et al. (2006) Appl. Environ. Microbiol. 72: 6593-6599; |
| | *Staphyloccus aureous* enterotoxin B | Fischer et al. (2007) J. Mol. Med. (Berl), 85: 461-469. |
| | *Bacillus thuringiensis* toxin | Allen et al. (2006) J. Immunol. Meth. 308: 109-115 |

| **Prion proteins** | | |
|---|---|---|
| | Recombinant bovine prion | Gofflot et al. (2004) J. Immunoassay Immunochem. 25: 241-258 |
| | Recombinant human prion | Gofflot et al. (2005) Clin. Chem. 51: 1605-1611 |
| | Recombinant prion protein | Guo et al. (2006) Nucleic Acids Res. 34: e62 |

| **Antibody** | | |
|---|---|---|
| | Mumps-specific IgG in serum | McKie et al. (2002) J. Immunol. Meth. 270: 135-141 |

| **Other** | | |
|---|---|---|
| | *Angiostrongylus cantonensis* 204 kDa AcL5 (fifth-stage larvae) | Chye et al. (2004) Clin. Chem. 50: 51-57 |
| | Aflatoxin B1 | Babu and Muriana (2011) J. Microbiol. Meth. 86: 188-194 |

In various embodiments antigenic markers characteristic of various microorganism are detected and/or quantified using cartridge-based immuno-PCR as described herein to provide detection of the presence and/or abundance of the microorganism. Such screens find use in patient diagnosis, environmental studies, and in screens of livestock, meat, and agricultural products.

In certain embodiments the immuno-PCR cartridges and methods described herein are used to detect and/or to quantify various bacterial toxins. Bacterial toxins include, but are not limited to Botulinum neurotoxins, Clostridium difficile toxin A, toxin B, lipopolysaccharide or endotoxin (associated with gram negative bacteria), tetanus toxin, shiga toxin, shiga-like toxin, *Staphlocccal* toxins *(e.g., S. aureus* enterotoxin A and B), Anthrax toxin, Cyanotoxin, Diphtheria toxin, Exotoxin, Pertussis toxin, and the like.

Also contemplated is the detection of various antigenic markers characteristic of fungi and/or mycotoxins produced by various fungi. Mycotoxins that may be detected using the cartridge-based immuno-PCR described herein include, but are not limited to aflatoxins, ochratoxin, ctrinin, ergot alkaloids, patulin, fusarium toxins and the like. Aflatoxins are a type of mycotoxin produced by *Aspergillus* species of fungi, such as *A. flavus* and *A. parasiticus,* and the like. Aflatoxins include four different types of mycotoxins designated B₁, B₂, G₁, and G₂. Aflatoxin B₁, the most toxic, is a potent carcinogen and has been directly correlated to adverse health effects, such as liver cancer, in many animal species.^{[8]} Aflatoxins are largely associated with commodities produced in the tropics and subtropics, such as cotton, peanuts, spices, pistachios, and maize. Ochratoxin a mycotoxin that comes in three secondary metabolite forms, all of which are produced by *Penicillium* and *Aspergillus* species. The three forms differ in that Ochratoxin B (OTB) is a nonchlorinated form of Ochratoxin A (OTA) and that Ochratoxin C (OTC) is an ethyl ester form Ochratoxin A. *Aspergillus ochraceus* is found as a contaminant of a wide range of commodities including beverages such as beer and wine. *Aspergillus carbonarius* is the main species found on vine fruit, which releases its toxin during the juice making process. OTA has been labeled as a carcinogen and a nephrotoxin, and has been linked to tumors in the human urinary tract. Citrinin has been identified in over a dozen species of *Penicillium* and several species of *Aspergillus.* Some of these species are used to produce human foodstuffs such as cheese (*Penicillium camemberti*), sake, miso, and soy sauce (*Aspergillus oryzae*). Citrinin is associated with yellowed rice disease in Japan and acts as a nephrotoxin in all animal species tested. It is associated with many human foods including but not limited to wheat, rice, corn, barley, oats, rye, and food colored with Monascus pigment. Ergot alkaloids include compounds produced as a toxic mixture of alkaloids in the sclerotia of species of *Claviceps,* which are common pathogens of various grass species. The ingestion of ergot sclerotia from infected cereals, commonly in the form of bread produced from contaminated flour, cause ergotism the human disease historically known as St. Anthony's Fire. There are two forms of ergotism: gangrenous, affecting blood supply to extremities, and convulsive, affecting the central nervous system. Modern methods of grain cleaning have significantly reduced ergotism as a human disease, however it is still an important veterinary problem. Patulin is a toxin produced by the *P. expansum, Aspergillus, Penicillium,* and *Paecilomyces* fungal species. *P. expansum* is especially associated with a range of moldy fruits and vegetables, in particular rotting apples and figs. It is destroyed by the fermentation process and so is not found in apple beverages, such as cider. Although patulin has not been shown to be carcinogenic, it has been reported to damage the immune system in animals. In 2004, the European Community set limits to the concentrations of patulin in food products. Fusarium toxins are produced by over 50 species of *Fusarium* and have a history of infecting the grain of developing cereals such as wheat and maize. They include a range of mycotoxins, such as: the fumonisins, which affect the nervous systems of horses and may cause cancer in rodents; the trichothecenes, which are most strongly associated with chronic and fatal toxic effects in animals and humans; and zearalenone, which is not correlated to any fatal toxic effects in animals or humans. Some of the other major types of *Fusarium* toxins include: beauvercin and enniatins, butenolide, equisetin, and fusarins.

In certain embodiments the immuno-PCR cartridges and methods described herein are used for the detection of various algal antigenic markers and/or toxins produced by various algae. Various algae and algal toxins are important pathogens, particular in the sea-food industry. Algal toxins include toxins produced by cyanobacteria (*e.g., Microcystis sp. Anabaena sp. Aphanizomenon sp. Nodularia sp. Oscillatoria sp, etc.*) and include hepatotoxins *(e.g.* microcystins and nodularin) and neurotoxins (e.g. anatoxin-a, and anatoxin-a(s)), domoic acid which is a neurotoxin produced by *Pseudonitzschia sp.* responsible for amnesic shellfish poising; saxitoxin, a neurotoxin produced by *Alexandrium sp.* that is responsible for paralytic shellfish poisoning, brevetoxin a neurotoxin produced by *Gymnodinuum sp.,* and the like.

In certain embodiments the immuno-PCR cartridges and methods described herein are used for the detection of various drug resistance genes or antigenic markers characteristic of various strains of drug-resistant pathogen. Thus, in certain embodiments immuno-PCR cartridges and methods described herein can be used to rapidly identify drug resistant bacteria and inform therapeutic strategies, or, in the case of cancer, to identify certain drug resistant cancer cells with can determine the most appropriate chemotherapeutic regimen. Illustrative drug resistant bacteria include, but are not limited to drug-resistant strains of *Staphylococcus aureus* (MRSA), *Burkholderia cepacian, Pseudomonas aeruginosa, Clostridium difficile, Klebsiella pneumoniae, Escherichia coli (E. coli), Acinetobacter baumannii, Mycobacterium tuberculosis, Neisseria gonorrhoeae, Streptococcus pyogenes,* and the like.

In certain embodiments, the immuno-PCR cartridges and methods described herein are used to detect one or more markers of an inflammatory response *(e.g.,* a host-response inflammation). Such markers include, but are not limited to of IL-8, calprotectin, and lactoferrin.

In certain embodiments, the immuno-PCR cartridges and methods described herein are used to detect one or more cancer markers. Such markers include, but are not limited to the cancer markers shown in Table 5.

**Table 5. Illustrative cancer markers and associated references.**

| Marker | Reference |
|---|---|
| 5 alpha reductase | Délos et al. (1998) Int J Cancer, 75:6 840-846 |
| α-fetoprotein | Esteban et al. (1996) Tumour Biol., 17(5): 299-305 |
| AM-1 | Harada et al. (1996) Tohoku J Exp Med., 180(3): 273-288 |
| APC | Dihlmannet al. (1997) Oncol Res., 9(3) 119-127 |
| APRIL | Sordat et al. ('998) J Exp Med., 188(6): 1185-1190 |
| BAGE | Böel et al. (1995) Immunity, 2: 167-175. |
| β-catenin | Hugh et al. (1999) Int J Cancer, 82(4): 504-11 |
| Bc12 | Koty et al. (1999) Lung Cancer, 23(2): 115-127 |
| bcr-abl (b3a2) | Verfaillie et al. ('996) Blood, 87(11): 4770-4779 |
| CA-125 | Bast et al. ('998) Int J Biol Markers, 13(4): 179-187 |
| CASP-8/FLICE | Mandruzzato et al. (1997) J Exp Med., 186(5): 785-793. |
| Cathepsins | Thomssen et al. (1995) Clin Cancer Res., 1(7): 741-746 |
| CD19 | Scheuermann et al. (1995) Leuk Lymphoma, 18(5-6): 385-397 |
| CD20 | Knox et al. (1996) Clin Cancer Res., 2(3): 457-470 |
| CD21, CD23 | Shubinsky et al. (1997) Leuk Lymphoma, 25(5-6): 521-530 |
| CD22, CD38 | French et al. (1995) Br J Cancer, 71(5): 986-994 |
| CD33 | Nakase et al. (1996) Am J Clin Pathol., 105(6): 761-768 |
| CD35 | Yamakawa et al. Cancer, 73(11): 2808-2817 |
| CD44 | Naot et al. (1997) Adv Cancer Res., 71: 241-319 |
| CD45 | Buzzi et al. (1992) Cancer Res., 52(14): 4027-4035 |
| CD46 | Yamakawa et al. (1994) Cancer, 73(11): 2808-2817 |
| CD5 | Stein et al. (1991) Clin Exp Immunol., 85(3): 418-423 |
| CD52 | Ginaldi et al. (1998) Leuk Res., 22(2): 185-191 |
| CD55 | Spendlove et al. (1999) Cancer Res., 59: 2282-2286. |
| CD59 (791Tgp72) | Jarvis et al. (1997) Int J Cancer, 71(6): 1049-1055 |
| CDC27 | Wang et al. (1999) Science, 284(5418): 1351-1354 |
| CDK4 | Wölfel et al. (1995) Science, 269(5228): 1281-1284 |
| CEA | Kass et al. (1999) Cancer Res., 59(3): 676-683 |
| c-myc | Watson et al. (1991) Cancer Res., 51(15): 3996-4000 |
| Cox-2 | Tsujii et al. (1998) Cell, 93: 705-716 |
| DCC | Gotley et al. (1996) Oncogene, 13(4): 787-795 |
| DcR3 | Pitti et al. (1998) Nature, 396: 699-703 |
| E6/E7 | Steller et al. (1996) Cancer Res., 56(21): 5087-5091 |
| EGFR | Yang et al. (1999) Cancer Res., 59(6): 1236-1243. |
| EMBP | Shiina et al. (1996) Prostate, 29(3): 169-176. |
| Ena78 | Arenberg et al. (1998) J. Clin. Invest., 102: 465-472. |
| FGF8b and FGF8a | Dorkin et al. (1999) Oncogene, 18(17): 2755-2761 |
| FLK-1/KDR | Annie and Fong (1999) Cancer Res., 59: 99-106 |
| Folic Acid Receptor | Dixon et al. (1992) J BioI Chem., 267(33): 24140-72414 |
| G250 | Divgi et al. (1998) Clin Cancer Res., 4(11): 2729-2739 |
| GAGE-Family | De Backer et al. (1999) Cancer Res., 59(13): 3157-3165 |
| gastrin 17 | Watson et al. (1995) Int J Cancer, 61(2): 233-240 |
| Gastrin-releasing hormone (bombesin) | Wang et al. (1996) Int J Cancer, 68(4): 528-534 |
| GD2/GD3/GM2 | Wiesner and Sweeley (1995) Int J Cancer, 60(3): 294-299 |
| GnRH | Bahk et al. (1998) Urol Res., 26(4): 259-264 |
| GnTV | Hengstler et al. (1998) Recent Results Cancer Res., 154: 47-85 |
| gp100/Pmel17 | Wagner et al. (1997) Cancer Immunol Immunother., 44(4): 239-247 |
| gp-100-in4 | Kirkin et al. (1998) APMIS, 106(7): 665-679 |
| gp15 | Maeurer et al.(1996) Melanoma Res., 6(1): 11-24 |
| gp75/TRP-1 | Lewis et al. (1995) Semin Cancer Biol., 6(6): 321-327 |
| hCG | Hoermann et al. (1992) Cancer Res., 52(6): 1520-1524 |
| Heparanase | Vlodavsky et al. (1999) Nat Med., 5(7): 793-802 |
| Her2/neu | Lewis et al. (1995) Semin Cancer Biol., 6(6): 321-327 |
| Her3 | |
| HMTV | Kahl et al.(1991) Br J Cancer, 63(4): 534-540 |
| Hsp70 | Jaattela et al. (1998) EMBO J., 17(21): 6124-6134 |
| hTERT (telomerase) | Vonderheide et al. (1999) Immunity, 10: 673-679. 1999. |
| IGFR1 | Ellis et al. (1998) Breast Cancer Res. Treat., 52: 175-184 |
| IL-13R | Murata et al. (1997) Biochem Biophys Res Commun., 238(1): 90-94 |
| iNOS | Klotz et al. (1998) Cancer, 82(10): 1897-1903 |
| Ki 67 | Gerdes et al. (1983) Int J Cancer, 31: 13-20 |
| KIAA0205 | Guéguen et al. (1998) J Immunol., 160(12): 6188-6194 |
| K-ras, H-ras, | Abrams et al. (1996) Semin Oncol., 23(1): 118-134 |
| N-ras | |
| KSA (CO17-1A) | Zhang et al. (1998) Clin Cancer Res., 4(2): 295-302 |
| LDLR-FUT | Caruso et al. (1998) Oncol Rep., 5(4): 927-930 |
| MAGE Family (MAGE1, MAGE3, etc.) | Marchand et al. (1999) Int J Cancer, 80(2): 219-230 |
| Mammaglobin | Watson et al. (1999) Cancer Res., 59: 13 3028-3031 |
| MAP17 | Kocher et al. (1996) Am J Pathol., 149(2): 493-500 |
| Melan-A/ MART-1 | Lewis and Houghton (1995) Semin Cancer Biol., 6(6): 321-327 |
| mesothelin | Chang et al. (1996) Proc. Natl. Acad. Sci., USA, 93(1): 136-140 |
| MIC A/B | Groh et al. (1998) Science, 279: 1737-1740 |
| MT-MMP's, such as MMP2, MMP3, MMP7, MMP9 | Sato and Seiki (1996) J Biochem (Tokyo), 119(2): 209-215 |
| Mox1 | Candia et al. (1992) Development, 116(4): 1123-1136 |
| Mucin, such as MUC-1, MUC-2, MUC-3, and MUC-4 | Lewis and Houghton (1995) Semin Cancer Biol., 6(6): 321-327 |
| MUM-1 | Kirkin et al. (1998) APMIS, 106(7): 665-679 |
| NY-ESO-1 | Jager et al. (1998) J. Exp. Med., 187: 265-270 |
| Osteonectin | Graham et al. (1997) Eur J Cancer, 33(10): 1654-1660 |
| p15 | Yoshida et al. (1995) Cancer Res., 55(13): 2756-2760 |
| P170/MDR1 | Trock et al. (1997) J Natl Cancer Inst., 89(13): 917-931 |
| p53 | Roth et al. (1996) Proc. Natl. Acad. Sci., USA, 93(10): 4781-4786. |
| p97/melanotransferrin | Furukawa et al. (1989) J Exp Med., 169(2): 585-590 |
| PAI-1 | Grøndahl-Hansen et al. (1993) Cancer Res., 53(11): 2513-2521 |
| PDGF | Vassbotn et al. (1993) Mol Cell Biol., 13(7): 4066-4076 |
| Plasminogen (uPA) | Naitoh et al. (1995) Jpn J Cancer Res., 86(1): 48-56 |
| PRAME | Kirkin et al. (1998) APMIS, 106(7): 665-679 |
| Probasin | Matuo et al. (1985) Biochem Biophys Res Commun., 130(1): 293-300 |
| Progenipoietin | - |
| PSA | Sanda et al. (1999) Urology, 53(2): 260-266. |
| PSM | Kawakami et al. (1997) Cancer Res., 57(12): 2321-2324 |
| RAGE-1 | Gaugler et al. (1996) Immunogenetics, 44(5): 323-330 |
| Rb | Dosaka-Akita et al. (1997) Cancer, 79(7): 1329-1337 |
| RCAS1 | Sonoda et al. (1996) Cancer, 77(8): 1501-1509. |
| SART-1 | Kikuchi et al.(1999( Int J Cancer, 81(3): 459-466 |
| SSX gene family | Gure et al. (1997) Int J Cancer, 72(6): 965-971 |
| STAT3 | Bromberg et al. (1999) Cell, 98(3): 295-303 |
| STn (mucin assoc.) | Sandmaier et al. (1999) J Immunother., 22(1): 54-66 |
| TAG-72 | Kuroki et al. (1990)Cancer Res., 50(16): 4872-4879 |
| TGF-α | Imanishi etal. (1989) Br J Cancer, 59(5): 761-765 |
| TGF-β | Picon et al. (1998) Cancer Epidemiol Biomarkers Prey, 7(6): 497-504 |
| Thymosin β 15 | Bao et al. (1996) Nature Medicine. 2(12), 1322-1328 |
| IFN-α | Moradi et al. (1993) Cancer, 72(8): 2433-2440 |
| TPA | Maulard et al. (1994) Cancer, 73(2): 394-398 |
| TPI | Nishida et al.(1984) Cancer Res 44(8): 3324-9 |
| TRP-2 | Parkhurst et al. (1998) Cancer Res., 58(21) 4895-4901 |
| Tyrosinase | Kirkin et al. (1998) APMIS, 106(7): 665-679 |
| VEGF | Hyodo et al. (1998) Eur J Cancer, 34(13): 2041-2045 |
| ZAG | Sanchez et al. (1999) Science, 283(5409): 1914-1919 |
| p16INK4 | Quelle et al. (1995) Oncogene Aug. 17, 1995; 11(4): 635-645 |
| Glutathione S-transferase | Hengstler (1998) et al. Recent Results Cancer Res., 154: 47-85 |

In view of the foregoing, in certain embodiments the immuno-PCR cartridge(s) described herein can include capture antibodies and detection antibodies that bind to an analyte selected from the group consisting of a viral antigen, a bacterial antigen, a prion, a parasitic antigen, a mycotoxin, an algal antigen, a fungal antigen, a cancer marker, and a drug-resistance gene product. In certain embodiments the capture antibody and the detection antibody are antibodies that bind to a viral antigen selected from the group consisting of Hepatitis B surface antigen, Hepatitis C surface antigen, bovine herpesvirus antigen, norovirus capsid, rotavirus, Angiostrongylus cantonensis worms, Hantavirus protein, avian influenza viral antigen, HIV-1 antigen, and H5N1 antigen. In certain embodiments the capture antibody and the detection antibody are antibodies that bind to a prion selected from the group consisting or bovine PRP, human brain PRP. In certain embodiments the capture antibody and the detection antibody are antibodies that bind to a bacterial antigen or bacterial toxin selected from the group consisting of C. *difficile* antigen, *C. difficile* Toxin A, *C. difficile Toxin* B, *P. piscidia, E. coli* antigen, *Bacteroides fragilis,* BoNT/A, *Streptococcus pyogenes* group A, *Staphylococcus aureus, Y. pestis* antigen, and *M. tuberculosis* antigen. In certain embodiments the capture antibody and the detection antibody are antibodies that bind to a bacterial toxin selected from the group consisting of shiga toxin 2, *Clostridium botulinum* neurotoxin A, Staphylococcal enterotoxin, *Bacillus thurigiensis* toxin, *C. difficile* Toxin A, and C. *difficile* Toxin B. In certain embodiments the capture antibody and the detection antibody are antibodies that bind to a polypeptide encoded by a drug resistance gene *(e.g.,* a drug-resistance polypeptide such as p-glycoprotein p glycoprotein, OleC polypeptide, mbcF polypeptide, MsrA polypeptide(s), bexA polypeptide, bexB polypeptide, kpsT polypeptide, kpsM polypeptide, and the like). In certain embodiments the capture antibody and the detection antibody are antibodies that bind to a drug-resistant strain of *Staphylococcus aureus* (MRSA), *Burkholderia cepacian, Pseudomonas aeruginosa, Clostridium difficile, Klebsiella pneumoniae, Escherichia coli (E. coli), Acinetobacter baumannii, Mycobacterium tuberculosis, Neisseria gonorrhoeae,* and/or *Streptococcus pyogenes,* or to a polypeptide encoded by a drug-resistance gene in these strains.

In certain embodiments the immuno-PCR cartridge(s) described herein can include capture antibodies and detection antibodies that bind to an analyte that comprises a markers of an inflammatory response *(e.g.,* a host-response inflammation). Such markers include, but are not limited to of IL-8, calprotectin, and lactoferrin.

In certain embodiments the immuno-PCR cartridge(s) described herein can include capture antibodies and detection antibodies that bind to an analyte that comprises a cancer marker. Such markers include, but are not limited to the cancer markers shown in Table 5.

Because, in various embodiments, the cartridges described herein permit both an immuno-PCR analysis (of one or more target analytes which may or may not be a polypeptide) and a nucleic acid analysis (of one or more target nucleic acids other than immuno-PCR signal DNA in the cartridge) it is possible to first assay for an immuno-PCR target and then perform a reflex assay for a related nucleic acid, or to first assay for a nucleic acid target and then perform a reflex immuno-PCR assay for a related target analyte.

Thus, for example where an immuno-PCR assay is directed to an antigen characteristic of a pathogenic strain of an organism, the nucleic acid analysis can be directed to a nucleic acid that encodes that marker *(e.g.,* for validation) or to a nucleic acid that encodes a drug-resistance gene or marker to provide first identification of the pathogen and second determination of the drug resistance status of that pathogen. Conversely, in certain embodiments these roles of the immuno-PCR and the nucleic acid analysis can be reversed.

Where an immuno-PCR assay is directed to markers of an inflammatory response *(e.g.,* IL-8, calprotectin, lactoferrin, *etc.),* the nucleic acid analysis can be directed to a nucleic acid that encodes that marker *(e.g.,* for validation).

Similarly where an immuno-PCR assay is directed to an antigen characteristic of a cancer cell *(e.g.,* a cancer marker), the nucleic acid analysis can be directed to a nucleic acid that encodes that marker *(e.g.,* a marker shown in Table 5, for validation) or to a nucleic acid that encodes a drug-resistance gene or marker to provide first identification of the cancer and then its drug-resistance status to inform chemotherapeutic protocols. Conversely, in certain embodiments these roles of the immuno-PCR and the nucleic acid analysis can be reversed.

In another example, an immuno-PCR can be used to identify a toxin produced by an organism and the nucleic acid analysis can be used to specifically identify the organism or strain producing the toxin. Conversely, in certain embodiments these roles of the immuno-PCR and the nucleic acid analysis can be reversed.

In various embodiments the immuno-PCR cartridge additionally contains primers and probes for detecting and/or quantifying a nucleic acid that encodes an analyte or a fragment of an analyte selected from the group consisting of a viral antigen, a bacterial antigen, a prion, a parasitic antigen, a mycotoxin, and an analyte from a cancer cell. In certain embodiments the immuno-PCR cartridge additionally contains primers and probes for detecting and/or quantifying a nucleic acid that encodes an viral antigen or a fragment of thereof where the antigen is selected from the group consisting of Hepatitis B surface antigen, Hepatitis C surface antigen, bovine herpesvirus antigen, norovirus capsid, rotavirus, Angiostrongylus cantonensis worms, Hantavirus protein, avian influenza viral antigen, HIV-1 antigen, and H5N1 antigen. In certain the immuno-PCR cartridge additionally contains primers and probes for detecting and/or quantifying a nucleic acid that encodes a bacterial antigen or bacterial toxin or fragment thereof where the bacterial antigen or bacterial toxin is selected from the group consisting of C. *difficile* antigen, C. *difficile* Toxin A, *C. difficile* Toxin B, *P. piscidia, E. coli* antigen, *Bacteroides fragilis,* BoNT/A, *Streptococcus pyogenes* group A, *Staphylococcus aureus, Y. pestis* antigen, and M. *tuberculosis* antigen. In certain the immuno-PCR cartridge additionally contains primers and probes for detecting and/or quantifying a nucleic acid that encodes a bacterial toxin or fragment thereof where the bacterial toxin is selected from the group consisting of shiga toxin 2, *Clostridium botulinum* neurotoxin A, Staphylococcal enterotoxin, *Bacillus thurigiensis* toxin, *C. difficile* Toxin A, and C. *difficile* Toxin B. In certain the immuno-PCR cartridge additionally contains primers and probes for detecting and/or quantifying a nucleic acid that encodes a polypeptide expressed by a drug resistance gene *(e.g.,* MRP, p-glycoprotein p glycoprotein, OleC polypeptide, mbcF polypeptide, MsrA polypeptide(s), bexA polypeptide, bexB polypeptide, kpsT polypeptide, kpsM polypeptide, and the like).

The various analytes and "reflex" assays described above are illustrative and non-limiting. In view of the teachings provided herein, numerous other assay targets and assay combinations will be recognized by one of skill in the art.

### Kits.

In various embodiments kits are provided for performing the methods described herein. In an illustrative embodiment the kits comprises one or more cartridges configured to perform immuno-PCR as described herein. In certain embodiments the kits additionally include a second cartridge *(e.g.,* a GENEXPERT^{®} cartridge) configured to prepare and analyze a nucleic acid sample as described herein. In various embodiments the kits comprise a cartridge configured to perform immuno-PCR as described herein as well as an additional nucleic acid amplification as described herein.

In certain embodiments a cartridge in the kit is configured to be loaded with a sample comprising a material from a biological, environmental, medical, or patient source. In various embodiments the kit can further provide one or more instruments and/or devices for obtaining and/or preparing a sample for use in a cartridge provided in the kit. In certain embodiments the kit contains one or more instruments and/or devices for obtaining and/or preparing a sample comprising an environmental sample selected from the group consisting of surface matter, soil, water, vegetation, and an industrial sample. In certain embodiments the kit contains one or more instruments and/or devices for obtaining and/or preparing a biological sample selected from the group consisting of a culture, blood, saliva, cerebral spinal fluid, urine, stool, bronchial aspirates, tracheal lavage, pleural fluid, milk, lymph, sputum, semen, needle aspirates, punch biopsies, surgical biopsies, cryopreserved sections, FFPE sections, and the like. In certain embodiments the kit contains one or more instruments and/or devices for obtaining and/or preparing a sample comprising a food or agriculture sample selected from the group consisting of meat, meat products, avian or avian products, milk or milk products, and farm crops, and organic waste.

In certain embodiments where the the kits additionally include a second cartridge *(e.g.,* a GENEXPERT^{®} cartridge) configured to prepare and analyze a nucleic acid sample as described herein or where the kits comprise a cartridge configured to perform immuno-PCR as described herein as well as an additional nucleic acid amplification as described herein, the kits can additionally contain reagents for processing and preparing a nucleic acid sample. Such reagents can include, for example, a lysis solution, e.g., as described in PCT Pub. No: WO 2014/052551, PCT Application No: PCT/US16/41917, and the like. In certain embodiments the kit comprises a container containing proteinase K and/or a container containing ethanol, and/or the kit contains a column for DNA preparation.

In certain embodiments the kit contains instructional materials teaching the use of a cartridge for immuno-PCR or the use of a cartridge for immuno-PCR and an additional nucleic acid analysis. Where a sample preparation cartridge (e.g., as described in PCT Application No: PCT/US16/37422) is included in the kit the kit can additionally contain instructional materials teaching the use and operation of the sample preparation cartridge.

While the instructional materials in the kits described above typically comprise written or printed materials they are not limited to such. Any medium capable of storing such instructions and communicating them to an end user is contemplated by this invention. Such media include, but are not limited to electronic storage media *(e.g.,* magnetic discs, tapes, cartridges, chips), optical media *(e.g.,* CD ROM), and the like. Such media may include addresses to internet sites that provide such instructional materials.

### EXAMPLES

The following examples are offered to illustrate, but not to limit the claimed invention.

### Example 1

### Cartridge-Based Immuno-PCR

One objective of this study was to extend use of the Cepheid GENEXPERT^{®} cartridge or similar cartridges to immuno-PCR alone, or in combination with a nucleic acid amplification (e.g., qPCR of DNA or RNA). It is believed that the ability to detect polypeptides by immuno-PCR using the GENEXPERT^{®} cartridge in addition to nucleic acid analysis provides significant utility because transcriptional and translational patterns not always positively correlated. The ability to assay for a polypeptide and then reflex the sample to assay for, *e.g.,* a corresponding nucleic acid, or conversely to assay for a target nucleic acid and then reflex the sample to assay for, *e.g.,* a corresponding polypeptide increases the likelihood of detection of the desired target analyte(s). Such a system is well suited for the detection and/or quantification of pathogens (e.g., bacteria, viruses, protozoal pathogens, *etc.),* bacterial toxins, environmental contaminants, to identify bacterial and cells *(e.g.,* cancer cells) that exhibit drug resistance, to detect prion "infected" animals, food products, and patients, and the like.

As a model system GENEXPERT^{®} cartridges were used for immuno-PCR detection of *Clostridium difficile* toxin B. One performance goal was detection of C. difficile toxin B at a concentration ranging from about 2.5 to about 1,000 ng/mL with a time to result of less than about 60 minutes, and an imprecision of less than about 15% across the concentration range.

*Clostridium difficile* is the cause of *Clostridium difficile-*associated diarrhea (CDAD), an antibiotic-associated diarrhea, and pseudomembranous colitis (PMC). In these disorders bacterial overgrowth of *Clostridium difficile* develops in the colon, typically as a consequence of antibiotic usage. Clindamycin and broad-spectrum cephalosporins have been most frequently associated with CDAD and PMC, but almost all antimicrobials may be responsible. Disease is related to production of toxin A and/or B. Treatment typically involves withdrawal of the associated antimicrobials and, if symptoms persist, orally administered and intraluminally active metronidazole, vancomycin, or fidaxomicin. Intravenous metronidazole may be used if an oral agent cannot be administered. In recent years, a more severe form of CDAD with increased morbidity and mortality has been recognized as being caused by an epidemic toxin-hyperproducing strain of *Clostridium difficile* (NAP1 strain). Many toxin-hyperproducing isolates also contain the binary toxin gene and are resistant quinolones.

Traditionally, diagnosis relied upon 1) clinical and epidemiologic features, 2) culture (which is labor intensive and time consuming), 3) cytotoxicity assays, which are labor intensive and time consuming, and 4) toxin detection immunoassays (which are insensitive).

The cartridge-based immuno-PCR described herein facilitates rapid detection of asymptomatic colonization, confirmation of active infection, and can reduce overdiagnosis, particularly when used with a reflex to a corresponding nucleic acid analysis.

Stool samples, solid, semi-solid or liquid weare utilized. For solid and semi-solid samples, using a sterile swab such as Pur-Wraps (Puritan Medical), coat the swab with the specimen equal to about 3 mm in diameter. For liquid fecal specimens, place swab into to specimen for at least 10 seconds or alternatively use approximately 50 - 300 µL of sample for processing. Soiled swabs or liquid fecal samples are transferred to 100 - 1,000 µL of sample extraction/diluent reagent containing but not limited to 0.5% mouse serum, 0.1% CHAPS, 1 mM EDTA, 0.09% sodium azide or 0.02% thimerosal as preservatives in PBS at pH 7.5. Diluted samples are thoroughly vortexed for approximately 10 seconds. Samples can be centrifuged at 1,000 to 5,000 rpm for up to five minutes to remove solids or alternatively transferred to the cartridge sample chamber (Figure 5B chamber 3) containing an optional pre-filter or fibrous plug inserted into a funnel allowing on cartridge sample filtering. Diluted samples are stored at 2 - 8 °C for approximately 72 hours or alternatively stored at ≤ -20°C for longer periods. The test sample containing C. *diff.* toxin B was

A capture antibody (mouse monoclonal anti-Toxin B Ab from BBI solutions) was attached to avidin conjugated particles (including in various embodiments, latex particle (Spherotech), silica particles (Bangs Lab), and hydrophilic and hydrophobic magnetic particles (Dynal, ThermoFisher) using a biotin/avidin interaction via an NHS-PEG12-Biotin, spacer arm 56Å (ThermoFisher) conjugated to the capture antibody through lysines using N-hydroxysuccinimide ester. The particles ranges in size from about 1 µm to about 2.9 µm.

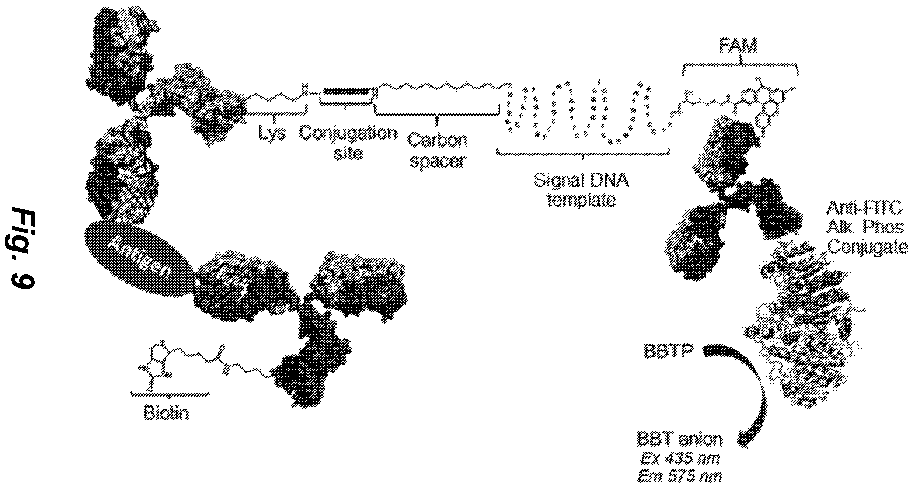

A detection antibody (mouse monoclonal anti-Toxin B Ab from BBI solutions) was attached to a signal DNA *(see,* SEQ ID NO:4 in Table 6, below) through a C12 linker by NHS ester conjugation through antibody lysines (Innova Biosciences kit) to provide attachment to the antibody and periodate oxidation of terminal sialic acid of glycan structure to form reactive aldehyde, reductive amination with 1° amine of the signal DNA template to form a stable conjugate (2° amine bond). An illustrative immunocomplex formed this conjugated signal DNA, is schematically illustrated in Figure 9.

Primers and probes used for PCR amplification and detection of the signal DNA are shown below in Table 6.

**Table 6. Primers, probe, and signal DNA used to detect C. difficile toxin B.**

| Name | Sequence | SEQ ID NO |
|---|---|---|
| Forward and reverse TAQMAN^{®} primers: | | |
| im001FwdV5 | 5'-TGTGGTCTATGTCGTCGTT-3' | 1 |
| im001RevV4 | 5'-TAGGAATTCTACGCCTCGAG-3' | 2 |
| Taqman Probe | | |
| Im001CF4_Q38v4^{∗} | 5′-(CF4-3)(CL4)CGC TAG TAG TTC CTG GGC TGC A(CDQ38)-3′ | 3 |
| Signal DNA Template conjugated to Detection Antibody:^{∗∗} | | |
| | 5′-NH₂-(C12 spacer)-TGT GGT CTA TGT CGT CGT TCG CTA GTA GTT CCT GGG CTG CAC TCG AGGCGTAGAATTCCTAC-FAM-3′ | 4 |
| ^{∗} CF4-3 = fluorophore CF4-3, CDQ38 = quencher, and CL4 = carbon linker | | |
| ^{∗∗}3' FAM was employed to allow troubleshooting in traditional ELISA micro titer plate format with anti-FITC alkaline phosphatase conjugate | | |

The PCR master mix provided in the GENEXPERT^{®} cartridge comprised 50 mM KCL, 6 mM MgCl₂, 200 µM dNTPs, 0.5 U/µL Pheonix Taq (Enzymatics), 0.25 µM Primers and Probe (shown in, Table 6), 1 M Betaine, 10 mM (NH₄)₂SO₄, 0.2% Tween 20, 0.2 mg/mL BSA, and 25 mM HEPES pH 8.2.

In one illustrative embodiment, the GENEXPERT^{®} cartridge was configured as shown in Table 7, and illustrated in Figure 6A.

**Table 7. One illustrative embodiment showing chamber contents for use of a GENEXPERT^{®} cartridge for measurement of a biomarker.**

| **Chamber** # | **Chamber Contents** | **Initial Volume (µL)** |
|---|---|---|
| 1 | Wash chamber | 0 |
| 2 | IC Wash Buffer: PBS, pH 7.25, 100 µg/mL salmon sperm DNA, 25 µg/mL goat IgG, 0.5% casein, 0.05% F68 | 1500 |
| 3^{∗} | Sample receiving chamber: User Added Sample stool supernatant in PBS, pH 7.5, 0.5% mouse serum, 0.1% CHAPS, 1 mM EDTA, 0.09% NaN₃ | 600 |
| 4 | Detection Ab-DNA conjugate in PBS, pH 7.25, 1.0% Casein, 150 µg/mL salmon sperm DNA, 50 µg/mL goat IgG, 0.1% F68. Optionally provided as beads. | 50 |
| 5 | Wash Buffer: PBS, pH 7.25, 0.1% Tween 20 | 1500 |
| 6 | Particles-SA/Ne-biotin Capture antibody, PBS pH 7.25, 1% casein, 0.1% F68. Optionally provided as beads-of-beads. | 50 |
| 7 | PCR Master Mix, probes/primers optionally provided as beads. | 75 |
| 8 | Waste-receiving chamber | 0 |
| 9 | Sample eluant chamber | 0 |
| 10 | 50 mM Tris-HCL | 200 |
| 11 | 65 mM KOH | 100 |
| ^{∗}Sample is added to chamber 3 by user. While study was performed using stool sample, other samples can readily be utilized. | | |

After loading, the cartridge was placed in a reaction module (see, e.g., Figure 8A) which was placed in a processing unit (see, e.g., Figure 8C), and operated to perform immuno-PCR. The thermocycling parameters for detection of the signal DNA using signal DNA, primers, and probes shown in Table , are provided below in Table 8.

**Table 8. Thermocycling parameters.**

| **Cycle** | Temperature and Time |
|---|---|
| 1 cycle | 96°C, 15s |
| 40 cycles | 96°C, 5s |
| | 66°C, 6s |
| | 72°C, 10s |

The foregoing cartridge-base immuno-PCR is illustrative and non-limiting. Other variations under consideration include, *inter alia,* covalent linkage of the capture antibody to the particle (e.g., through a carbohydrate, lysine, or cysteine) instead of linkage via streptavidin/biotin, cleavable linkers (e.g., base-labile linkers, acid labile linkers, disulfide linkers, and restriction sites) to join the detection antibody to the signal DNA as well as various glycoconjugation methods (e.g., sodium periodate oxidation of cis-diols (galactose or sialic acid), or remodeling of glycan structure with a mixture of β 1,4-galactosyltransferase (Gal T) and α2,6-sialytransferase (Sial T) followed by mild oxidation of sialic acid for conjugation), and the like.

The use of various blocking agents, *e.g.,* to prevent or reduce non-specific binding is also under investigation. Such agent s include, but are not limited to various polymers/detergents/ carbohydrates (e.g., PEG, Pluronics F68/F108/F127 (F68 preferred), PVP, Biolipidure 802 (NOF America), Tween 20 or 80, TEGME (Tre(ethylene glycol) monoethyl ether), TEG (Tegraethylene glycol), and the like) and/or various proteins/surfactants/polysaccharides (*e.g.*, casein, BSA, goat IgG, bovine IgG, Stabilcoat (ThermoFisher), iota-carrageenan, dextran sulfate, mouse serum, and the like), herring or salmon sperm DNA, and the like.

Also particle modifications for direct antibody conjugation are under review. In certain embodiments such modifications utilize common functional groups such as carboxylate, aldehyde, hydrazide, amide, azide, etc. for direct antibody attachment or for linker attachment. In certain embodiments the use of hydrophilic spacer arms are also contemplated. Such spacer/linkers comprises a moiety such as PEG, and/or polymers containing phosphorylcholine hydrophilic pendant groups, and the like.

It is understood that the examples and embodiments described herein are for illustrative purposes only.

### Example 2

### Cartridge-Based Immuno-PCR: Detection of Human IL-8 and Clostridium difficile Toxin B

This study illustrates the detection of human interleukin-8 (IL-8) and *Clostridium difficile* Toxin B using cartridge-based immune-PCR and compares the results obtained using this format to those obtained using a manual format.

### Materials and Methods

### Capture Antibody Biotinylation

Mouse monoclonal anti-*C. Difficile* toxin B (BBI Solutions) antibody (0.5 mg in PBS pH 7.2, 0.1% NaN₃) was biotinylated with 10-fold excess of EZ-LINK^{™} NHS-PEG₁₂-Biotin (Thermo Fisher) using a 250 mM stock solution of the modification reagent in DMSO. The reaction was incubated at room temperature for 30 minutes. The reaction mixture was desalted and buffer exchanged into PBS pH 7.4 and 0.09% NaN₃ using a ZEBA^{™} spin desalting column (MWCO 30 kDa, Thermo Fisher). Quantification was performed using A₂₈₀ measurements and used without further purification. Biotinylation of mouse monoclonal anti-Human IL-8 (BD Biosciences) was performed as described above with 20-fold excess of biotinylation reagent and using VIVASPIN^{®} 500, 50 kDa MWCO (Sartorius) concentrator for desalting, buffer exchange and concentration.

The mouse monoclonal anti-C. *Difficile* toxin B can also be directly conjugated to carboxyl modified latex particles (Thermo Fisher) via EDAC/Sulfo-NHS coupling at 20 - 30 µg of antibody per mg of particles. Briefly, carboxylate particles were activated in activation/coupling buffer (MES buffer pH 6.0) with a 20:1 ratio of Sulfo-NHS to carboxylate and an EDAC to carboxylate ratio of 2.5:1 at room temperature for 40 minutes. Reaction byproducts were removed by centrifugation and the activated particles were washed once with the activation/coupling buffer. For antibody conjugation, the particles were resuspended with the activation/coupling buffer, sonicated at 10% amplitude for 4 - 6 seconds followed by addition of the capture antibody. The final particle concentration was 2% (w/v) during antibody conjugation with the antibody at 0.2 - 0.3 mg/mL. Conjugation proceeded overnight at 2 - 8°C. The reaction was quenched with 30 µL of ethanolamine, and the particles were washed six times with PBS, 0.1% Triton X-100, pH 7.43 with sonication at 20% amplitude for 2 - 3 for the first two wash cycles. The amount of antibody covalently attached to the particles was estimated using the Micro BCA assay (Thermo Fisher).

### Detection Antibody DNA Conjugation

### Anti-Human IL-8 Detection Antibody Preparation

The DNA conjugate for the mouse monoclonal anti-Human IL-8 antibody was performed using the THUNDER-LINK^{®} Oligo conjugation system (Innova Biosciences) through the available antibody surface lysines. Briefly, 100 µg of the antibody was activated with the activation reagent for 45 minutes at room temperature and desalted using a PD-10 column equilibrated with the accompanying wash buffer.

The DNA template derivatized on the 5'-end with a primary amine and on the 3'-end with FAM (Integrated DNA Technologies, Table 9) was activated according to the manufacturer's protocol. Briefly, a stock solution of the template was diluted with the supplied wash buffer to 100 µM, added to the activation reagent, and reacted for 35 minutes at room temperature. The mixture was desalted using a PD-10 column equilibrated with wash buffer.

The antibody-DNA conjugate was produced by mixing 3-fold excess of the activated template to the activated antibody and reacting for 1.5 hours at room temperature. Conjugate Clean Up reagent, 600 µL, was added to the mixture and incubated on ice for 30 minutes, and centrifuged at 15,000 x g for 5 minutes to isolate the conjugate pellet. The conjugate was resuspended with PBS pH 7.2 and 0.09% NaN₃. Quantitation was performed using the Bradford assay.

### Anti-C. Diff Toxin B Detection Antibody Preparation

Formation of the mouse monoclonal anti-C. *Diff* Toxin B antibody DNA conjugate was achieved by forming a bis-arylhydrazone linkage between the antibody and the DNA template. The Im001_NH2 DNA template (Table 9) was resuspended with modification buffer (0.1 M phosphate, pH 8.0, 0.15 M NaCl) to 1.7 nmol/OD. An excess of succinimidyl 4-formylbenzoate (SFB, TriLink Biotechnologies) in DMF was added to the amino-template, and mixed at 600 RPM for 2.5 hours at room temperature. The aldehyde modified template was doubly desalted and buffer exchanged into the conjugation buffer (0.1 M phosphate, pH 6.0, 0.15 M NaCl) using a ZEBA^{™} spin column at 1,500 x g for 2 minutes.

The antibody, 516 µg, was exchanged into the modification buffer using a ZEBA^{™} spin column with a resulting concentration of 2.5 mg/mL as measured by absorption at 280 nm. Twelve equivalents of succinimidyl 4-hydrazinonicotinate acetone hydrazone (SANH, TriLink Biotechnologies) in DMF was added to the antibody, and mixed at 600 RPM for 2.5 hours at room temperature. A ZEBA^{™} spin column was used to desalt and exchange the modified antibody into the conjugation buffer. The modified antibody concentration was determined using the Bradford assay.

The antibody-DNA conjugate was formed by mixing an excess of the aldehyde modified DNA template with the hydrazone modified antibody overnight at 4°C. The resulting conjugate was purified by size exclusion (Superdex 200 Increase 10/300, GE Healthcare) under an isocratic gradient of PBS pH 7.3 at 0.75 mL/min. The antibody-DNA conjugate was concentrated using a VIVASPIN^{®} 500 centrifugal concentrator with a 50 kDa MWCO. The conjugate concentration was determined to be 3.5 mg/mL using the MICRO BCA^{™} assay (Thermo Fisher).

**Table 9. DNA Template**

| Name | Sequence | SEQ ID NO |
|---|---|---|
| Im001_NH₂ | 5'-NH2-C12- TGT GGT CTA TGT CGT CGT TCG CTA GTA GTT CCT GGG CTG CAC TCG AGG CGT AGA ATT CCT AC-3' | 5 |
| Im001_NH_{2_}FAM | 5'-NH2-C12- TGT GGT CTA TGT CGT CGT TCG CTA GTA GTT CCT GGG CTG CAC TCG AGG CGT AGA ATT CCT AC – FAM-3' | 6 |

### Particle Based Immuno-PCR (iPCR) Assay using Filter Plate

### Capture Particle Preparation

Biotinylated antibodies, 4 µg/mL, were immobilized on 1 µm streptavidin coated latex microspheres (Spherotech) at a concentration of 2 × 10⁹ particles/mL in assay diluent (PBS pH 7.4, 1% casein and 1% BSA) for 1 hour at room temperature with mixing. The immobilized antibody bead suspensions were washed 5x with 1 mL PBST (PBS pH 7.3, 0.1% Tween 20) or PBS-TX (0.1% Triton X-100). Between washes, particles were recovered by centrifugation at 9,000 × g for 4 minutes. Immobilized antibody beads were blocked with STABILCOAT^{®} Plus Microarray Stabilizer (Surmodics) for 2 hours at 37°C with mixing at 600 RPM and stored at 4°C until used. Prior to use, particles were washed 1x with PBS pH 7.3 and brought to a working concentration of ∼2.8 × 10⁹ particles/mL with assay diluent.

### C. Diff Toxin B and Human IL-8 Filter Plate Assays

1. A 10-fold serial dilution C. *Difficile* Toxin B (Enzo Life Sciences) was performed using assay diluent covering a 0-1,600 ng/mL range. Assay diluent was used for 0 ng/mL.
2. A 5-fold serial dilution of Human IL-8 (Perpotech) was performed using assay diluent covering a 0-10,000 pg/mL range. Assay diluent was used for 0 pg/mL.
3. Pre-wetting and surface blocking of a 96 well PCF (polycarbonate) filter plate with a 0.45 µm pore size (Millipore Sigma) was performed using 150 µL of assay diluent at room temperature for 1 hour prior to use. Vacuum filtration at 7" Hg of vacuum was used to remove the diluent using the MULTISCREEN^{™} Vacuum Manifold (Millipore Sigma).
4. Toxin B Assay
   a. 75 µL assay diluent was added to all wells.
   b. 25 µL of particles at working concentration (∼2.8 × 10⁹ particles/mL) was added to all wells.
   c. 25 µL of each serially diluted toxin B was added to the appropriate wells. Assay diluent was used for the zero control. Assay performed in duplicate.
   d. Plate was sealed and placed on a shaker at 650 RPM for 5 minutes. Reaction was filtered at 6 - 7" Hg.
   e. Reaction was washed 10x with 200 µL of PBST.
   f. 100 µL of anti-C. *Diff* toxin B antibody-DNA conjugate (0.5 µg/mL) was added to all wells.
   g. Plate was sealed and placed on a shaker at 650 RPM for 5 minutes. Reaction was filtered at 6 -7 " Hg.
   h. Reaction was washed 10x with 200 µL of PBST.
   i. 65 µL 25 mM KOH was added to each well, the plate was sealed, placed on a shaker at 650 RPM for 5 minutes.
   j. 70 µL 50 mM Tris-Cl pH 7.0 was added to each well and the eluant collected into a solid bottom 96 well plate by vacuum at 6 -7 " Hg.
5. IL-8 Assay
   a. 50 µL assay diluent was added to all wells.
   b. 25 µL of particles at working concentration (∼2.8 × 10⁹ particles/mL) was added to all wells.
   c. 25 µL of each serially diluted toxin B was added to the appropriate wells. Assay diluent was used for the zero control. Assay performed in duplicate.
   d. Plate was sealed and placed on a shaker at 650 RPM for 10 minutes. Reaction was filtered at 6 - 7" Hg.
   e. Reaction was washed 5x with 250 µL of PBST.
   f. 100 µL of anti-Human IL-8 antibody-DNA conjugate (30 ng/mL) was added to all wells.
   g. Plate was sealed and placed on a shaker at 650 RPM for 10 minutes. Reaction was filtered at 6 -7 " Hg.
   h. Reaction was washed 9x with 225 µL of PBST.
   i. 65 µL of 25 mM KOH was added to each well, the plate was sealed, placed on a shaker at 650 RPM for 5 minutes.
   j. 70 µL of 50 mM Tris-Cl pH 7.0 was added to each well and the eluant collected into a solid bottom 96 well plate by vacuum at 6 -7 " Hg.
6. Detection by PCR
   a. 2 µL of assay eluant was added to a 96 well PCR plate. Then, 18 µL of PCR master mix containing 2 units of Pheonix Hot Start Taq polymerase (Enzymatics) in 1x GC buffer supplemented with 0.2 mg/mL BSA, 0.2% Tween 20, 0.4 mM magnesium (2.4 mM Mg²⁺ total), 200 µM dNTPs, 0.5 µM Fwd primer, 1.0 µM Rev primer, and 0.35 µM Probe (Table 2).
   b. A two-step PCR was performed according to the protocol listed in Table 10.

**Table 10. PCR Primers, Probe and PCR Protocol**

| Name | Sequence | SEQ ID NO |
|---|---|---|
| Primer Fwd | 5'-GGTCTATGTCGTCGTTCG-3' | 7 |
| Primer Rev | 5'- AGGAATTCTACGCCTCGA-3' | 8 |
| Probe | CF4-3-(CL4)CTAGTAGTTCCTGGGCTGCAC-CDQ38 | 9 |
| PCR Protocol | 95°C 30s, 35 cycles of 95°C 10s and 64°C 35s | |

### Automated Immuno-PCR Assay using the GeneXpert Cartridge

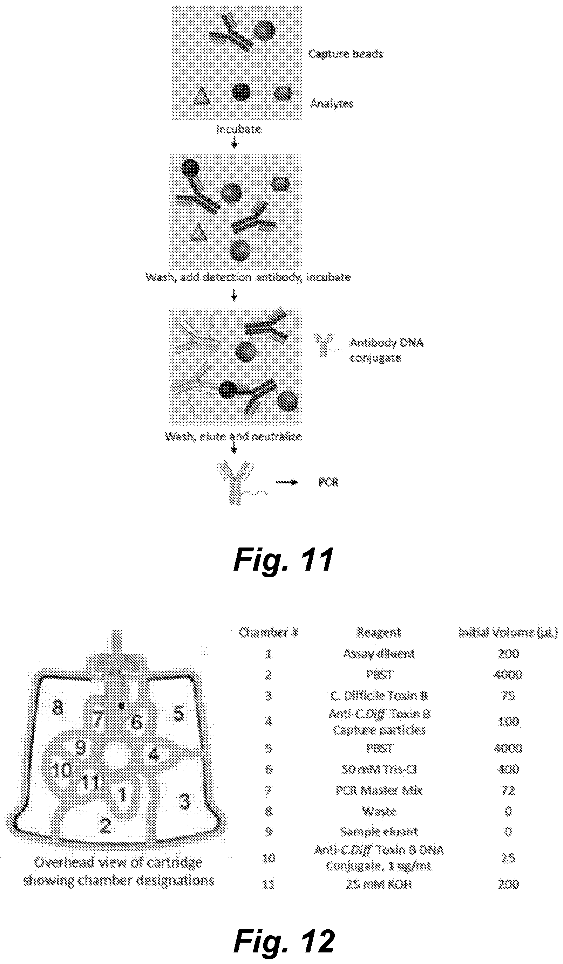

Figure 11 depicts a summary of the automated assay process. Figure 12 shows the cartridge chamber (CH) assignments, reagents, and initial volumes. The assay reagents were dispensed into cartridge A⁺ equipped with a glass fiber filter and a pore size of 0.7 µm.

### 1. Automated Toxin B Assay

### Cartridge priming

a. 500 µL of PBST (CH2) was used to prime the direct and filter fluidic paths and delivered to CH8.

### Toxin B capture

b. 25 µL of *C.Diff*toxin B standard is aspirated from CH3 and delivered to capture particles in CH4.
c. In CH3, for 5 minutes, the sample is incubated with the capture particles with two mixing events at 142 seconds.
d. To move toxin B immobilized on the particles to the filter, 125 µL of CH3 contents is moved from CH3 through the filter path into CH8.
e. CH3 is washed by moving 200 µL of PBST from CH2 to CH3 and then moving CH3 contents to CH8 across the filter path.
f. Particle immobilized toxin B is washed 10x with 250 µL aliquots of PBST by moving PBST from CH2 to CH8 through the filter path.

### Toxin B Detection

g. For detection, 75 µL of assay diluent (CH1) is aspirated across the filter path and transferred to CH10 moving the particles charged with toxin B to the anti-*C.Diff* toxin B DNA conjugate.
h. In CH10, for 5 minutes, the sample is incubated with the particles with two mixing events at 142 seconds.
i. To move the immobilized immune complex to the filter, 100 µL of CH10 contents is moved from CH10 through the filter path into CH8.
j. CH10 is washed by moving 200 µL of PBST from CH5 to CH10 and then moving CH10 contents to CH8 across the filter path.
k. The immobilized immune complex is washed 10x with 250 µL aliquots of PBST by moving PBST from CH5 to CH8 through the filter path.

### Immune complex disruption and neutralization

1. 65 µL of KOH is aspirated from CH11 and dispensed into CH9 across the filter containing the particle immobilized immune complex and mixed 2 times.
m. 15 µL of KOH is aspirated from CH9 into the syringe balancing the fluid contents into three spaces: 15 µL in syringe, 40 µL in filter region, and 10 µL in CH9.
n. In the filter region, the particles charged with the immune complex are sonicated at a 40% amplitude for 15 seconds, mixed, and followed by a 60 second wait step. The process is repeated one more time for a total of 2 minutes exposure of the immune complex to KOH. (Note: Elution can be performed with or without sonication and for 2 - 5 minute duration.)
o. 70 µL of Tris-Cl is moved from CH6 to CH9 through the filter path to neutralize the eluted DNA template.

### PCR preparation and detection

p. 8 µL of the sample is moved from CH9 to CH7 and mixed with the PCR master mix one time. CH7 contains 72 uL of PCR master mix containing 6.4 units of Pheonix Hot Start Taq polymerase (Enzymatics) in 1x GC buffer supplemented with 0.2 mg/mL BSA, 0.2% Tween 20, 0.4 mM magnesium (2.4 mM Mg²⁺ total), 200 µM dNTPs, 0.5 µM Fwd primer, 1.0 µM Rev primer, and 0.35 µM Probe (Table 2).
q. 70 µL of the PCR mixture is aspirated into the PCR reaction tube.
r. PCR is performed according to the protocol described in Table 10. A proprietary fluorophore and quencher is used to prepare the Taqman probe. The emission signal generated from hydrolysis of the probe is detected between 620 - 645 nm.

### Results

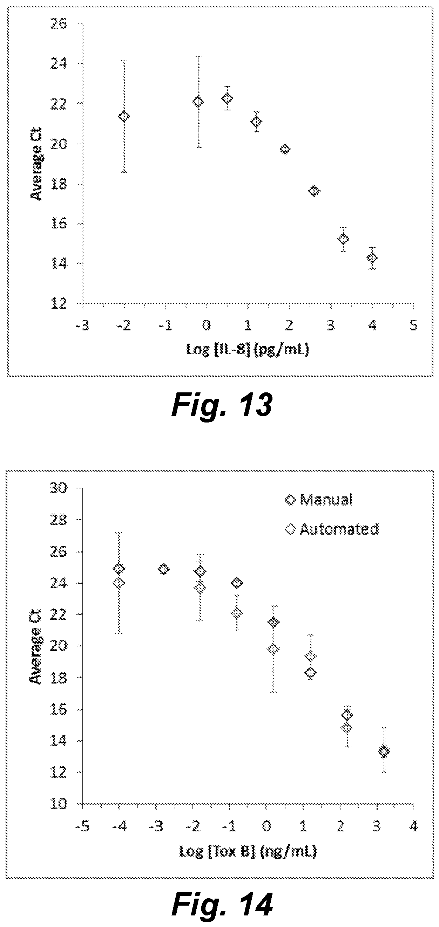

The human IL-8 iPCR dose response was created by combining fivefold dilutions (10,000 - 0.64 pg/mL) of the recombinant cytokine and measured in duplicate. The results are shown in Fig. 13. The dynamic range (80 - 10,000 pg/mL) and limit of detection (80 pg/mL) are limited by the non-specific interactions of the anti-human IL8-DNA detection conjugate with the particle surface. The intra-assay precision CV was ≤ 4% representing acceptable performance in the dose range of 3.2 - 10,000 pg/mL. The high imprecision (% CV > 10) of the assay occurs at the low and zero dose concentrations, 0.64 pg/mL and 0 pg/mL, Table 11.

**Table 11. Human IL-8 Average Ct and Assay Imprecision**

| **[IL.8) (pg/mL)** | **Ave. Ct** | **SD** | **%CV** |
|---|---|---|---|
| 0 | 21.4 | 2.79 | 13.1 |
| 1 | 22.1 | 2.27 | 10.3 |
| 3.2 | 22.3 | 0.59 | 2.6 |
| 16 | 21.1 | 0.50 | 2.4 |
| 80 | 19.7 | 0.10 | 0.5 |
| 400 | 17.6 | 0.16 | 0.9 |
| 2,000 | 15.2 | 0.60 | 4.0 |
| 10,000 | 14. 3 | 0.53 | 3.7 |

The C. *Diff* Toxin B iPCR manual filter plate assay demonstrated ≤ 4% intra-assay CV across the tenfold dilution range of 1,600 - 0.0016 ng/mL (Figure 14 and Table 12) with a limit of detection at 0.16 ng/mL. In comparison, automation of the assay using the GENEXPERT^{®} cartridge demonstrated higher imprecision (up to 14% CV) across the assay range (Table 4). The limit of detection was reduced 1,000-fold due to the replicate imprecision. It is anticipated that further improvements to the automated cartridge fluidic protocol will reduce the assay imprecision and increase the assay sensitivity.

**Table 12. C. Difficile Toxin B Average Ct and Assay Imprecision**

| **[Toxin B] (ng/mL)** | **Ave. Ct** | **Manual SD** | **%Cv** | **Ave. Ct** | **Automated SD** | **%CV** |
|---|---|---|---|---|---|---|
| 0 | 24.9 | 0.24 | 1.0 | 24 | 3.2 | 12.4 |
| 0.0016 | 24.9 | 0.25 | 1.0 | N/A | N/A | N/A |
| 0.016 | 24.7 | 0.63 | 2.5 | 23.7 | 2.1 | 8.9 |
| 0.16 | 24.0 | 0.14 | 0.6 | 22.1 | 1.1 | 5.0 |
| 1.6 | 21.5 | 0.06 | 0.3 | 19.8 | 2.7 | 13.6 |
| 16 | 18.3 | 0.22 | 1.2 | 19.3 | 1.4 | 7.3 |
| 160 | 15.6 | 0.61 | 3.9 | 14.8 | 1.2 | 0.6 |
| 1600 | 13.2 | 0.28 | 2.1 | 13.4 | 1.4 | 1.5 |

To compare the performance between automation of the C. *Difficile* toxin B Immuno-PCR assay with the filter plate assay, toxin B was spiked into assay diluent (PBS, 1% BSA, 0.05% NaN3, pH 7.4), ten-fold serially diluted, and assayed with each format with both iterations taking one hour to result. Both formats result in a %CV of <10% across the dilution range (Table 13).

**Table 13. C. Difficile Toxin B Automated and Manual Assay Imprecision**

| **[Toxin B] (ng/mL** | **Manual** | | **Automated** | |
|---|---|---|---|---|
| | **Ave. Ct** | **%CV** | **Ave. Ct** | **%CV** |
| **0** | 29.7 ±1,1 | 3.8 | 22.1 ±1.2 | 5.5 |
| **0.0025** | 28.3 ±1.6 | 5.7 | N/A | |
| **0.025** | 28.3 ±0.81 | 2.8 | 21.1 ±1.6 | 7.6 |
| **0.25** | 27.0 ±0.41 | 1.5 | 23.2 ±0.57 | 2.4 |
| **2.5** | 22.9 ±0.07 | 0.3 | 21.9 ±0.31 | 1.4 |
| **25** | 18.4 ±0.47 | 2.5 | 18.8 ±0.40 | 2.1 |
| **250** | 15.6 ±0.12 | 0.8 | 16.7 ±0.26 | 1.6 |
| **2500** | 15.0 ±0.18 | 1.2 | 15.7 ±0.21 | 1.4 |

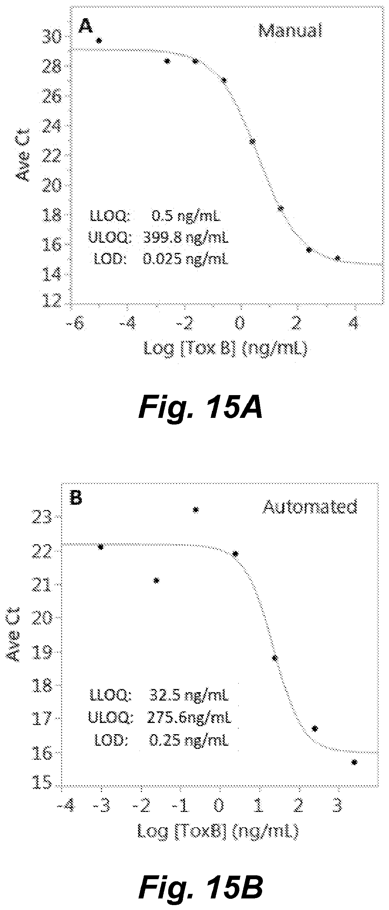

The dose response curves were fitted using a four parameter logistic fit with the upper limit of quantitation (ULOQ) and lower limit of quantitation (LLOQ) calculated using the inverse predication of the plotted concentration. Three times the standard deviation was either subtracted or added to the average Ct values for the LLOQ or ULOQ calculations respectively using a 95% confidence level. Figure 15 depicts the fitted dose response curves between the two methods. There is a 65-fold reduction in the LOD with the automated process in comparison with the manual method, however, the LLOQ (32.5 ng/mL) for the automated process is still clinically relevant and sufficient to support treatment selection for patients with C. *Difficile* infection.

## Claims

1. A cartridge for performing immuno-PCR to detect and/or quantify one or more target analytes, and/or detecting and/or quantifying a nucleic acid, said cartridge comprising:
a sample receiving chamber;
a chamber comprising a matrix material that acts as a filter and/or a DNA binding agent;
a temperature controlled channel or chamber; and
a plurality of chambers containing reagents for performing immuno-PCR, wherein:
said plurality of chambers comprises a chamber containing a capture antibody that binds the analyte that is to be detected;
said plurality of chambers comprises a chamber containing a detection antibody where said detection antibody is optionally attached directly or indirectly to a signal DNA;
said plurality of chambers comprises a chamber containing a PCR master mix;
said plurality of chambers comprises a chamber containing primers for amplifying all or a region of said signal DNA; and
said plurality of chambers comprises a chamber containing a probe for detecting all or a region of said signal DNA,
wherein said sample receiving chamber, said chamber comprising a matrix material, said temperature controlled heating channel or chamber or a port into said temperature controlled channel or chamber, and said plurality of chambers are disposed around a central valve and selectively in fluid communication with a channel in said central valve, wherein said central valve is configured to accommodate a plunger that is capable of drawing fluid into or out of a chamber in fluid communication with said central valve.

2. The cartridge according to claim 1, wherein said central valve comprises a syringe barrel in fluid communication with a valve body and that is sealed with a gasket, and wherein said syringe barrel and said valve body are configured to rotate.

3. The cartridge according to claims 1-2, wherein said PCR primers, and/or probes, and/or polymerase in said master mix are provided as beads; and/or wherein said cartridge contains detection antibodies for the detection of a single analyte or a plurality of analytes; and/or wherein said cartridge contains capture antibodies for the capture of a single analyte.

4. The cartridge according to any one of claims 1-3, wherein said capture antibody is attached to a particle; or wherein said capture antibody is attached to a particle and wherein said particle ranges in size at least about 0.5 µm, or from about 1 µm up to about 10 µm, or up to about 3 µm, or wherein said particle ranges in size from about 1 µm up to about 2.8 µm.

5. The cartridge according to any one of claims 1-4, wherein said plurality of chambers further comprises a chamber containing PCR primers for amplifying a nucleic acid other than said signal DNA; and/or wherein said plurality of chambers further comprises a chamber containing a probe for detecting and/or quantifying a nucleic acid other than said signal DNA; and/or wherein said target analyte comprises a polypeptide and a nucleic acid other than said signal DNA comprise a nucleic acid encoding said polypeptide or a fragment thereof; and/or wherein said target analyte comprises a polypeptide and a nucleic acid other than said signal DNA comprise a nucleic acid characteristic of a cell, tissue, or organism that produces said polypeptide.

6. The cartridge according to any one of claims 1-5, wherein said cartridge is configured so that, when in use, said cartridge comprises:
a chamber containing a sample;
a chamber containing said detection antibody;
a chamber containing said capture antibody;
a chamber containing a wash buffer; and
a chamber containing a PCR master mix for amplifying said signal DNA.

7. A method of performing immuno-PCR to detect and/or quantify one or more target analytes, and/or detecting and/or quantifying a target nucleic acid, said method comprising: providing a sample in a sample receiving chamber of a cartridge configured for performing immuno-PCR; and using said cartridge in an immuno-PCR method comprising:
contacting said analyte with a capture antibody under conditions where said capture antibody binds said analyte, forming a capture antibody/analyte complex;
contacting said analyte with a detection antibody attached to a signal DNA under conditions where said detection antibody specifically binds to and forms an immunocomplex with said capture antibody/analyte complex;
releasing said immunocomplex or a portion thereof comprising said signal DNA and delivering said immunocomplex or a portion thereof into a temperature controlled channel or chamber; and
performing a nucleic acid amplification in said temperature controlled channel or chamber to detect and/or quantify said signal nucleic acid thereby detecting and/or quantifying said analyte, wherein said cartridge is a cartridge according to any one of claims 1-6.

8. A method of performing immuno-PCR to detect and/or quantify one or more target analytes, and/or detecting and/or quantifying a nucleic acid, said method comprising: providing a sample in a sample receiving chamber of a cartridge configured for performing immuno-PCR; and using said cartridge in an immuno-PCR method comprising:
contacting said analyte with a capture antibody under conditions where said capture antibody binds said analyte, forming a capture antibody/analyte complex;
contacting said analyte with a detection antibody where said contacting is under conditions where said detection antibody specifically binds to and forms an immunocomplex with said capture antibody/analyte complex;
contacting said immunocomplex with a labeled antibody that binds to said detection antibody where said labeled antibody is attached to a signal DNA and said contacting is under conditions where said labeled antibody specifically binds to said detection antibody to form a labeled immunocomplex;
releasing said labeled immunocomplex or a portion thereof comprising said signal DNA and delivering said immunocomplex or a portion thereof into a temperature controlled channel or chamber; and
performing a nucleic acid amplification in said temperature controlled channel or chamber to detect and/or quantify said signal nucleic acid thereby detecting and/or quantifying said analyte, wherein said cartridge is a cartridge according to any one of claims 1-6.

9. The method according to any one of claims 7-8, wherein said method is performed on a plurality of different analytes in the same cartridge; and/or wherein said method can be completed in about 1 hour or less, or in about 50 minutes or less, or in about 40 minutes or less, or in about 30 minutes or less, or in about 20 minutes or less, or in about 15 minutes or less.

10. The method according to any one of claims 7-9, wherein
said method is performed in a cartridge according to any one of claims 5-6; and
said method further comprises amplifying a nucleic acid other than said signal DNA.

11. The method of claim 10, wherein said amplifying a nucleic acid other than said signal DNA comprises:
binding a nucleic acid from said sample to a matrix material;
washing the bound nucleic acid to provide a washed nucleic acid;
eluting the washed nucleic acid; and
subjecting said washed nucleic acid to an amplification reaction to amplify a target nucleic acid sequence if present in said washed nucleic acid.

12. The method according to any one of claims 10-11, wherein said nucleic acid other than said signal DNA comprises a DNA or an RNA; and/or wherein said amplifying a nucleic acid other than said signal DNA is performed on nucleic acid(s) obtained from the same sample in the sample chamber used for said immuno-PCR or on nucleic acid(s) obtained from a sample in a sample chamber different than the sample chamber used for said immuno-PCR; and/or wherein the signal DNAs and the nucleic acid(s) other than a signal DNA are amplified sequentially in the same temperature controlled channel or chamber.

13. A system for performing immuno-PCR to detect and/or quantify one or more target analytes, and/or to detect and/or to quantify a nucleic acid, said system comprising:
an enclosure configured to contain one or more sample processing modules, each sample processing module configured to hold a removable cartridge according to any one of claims 1-6, where said system is configured to operate the sample processing modules to perform immuno-PCR to determine the presence and/or quantity of one or more target analytes and/or to determine the level of one or more target DNA sequences within a corresponding removable sample cartridge, wherein said processing on a sample within the corresponding removable sample cartridge performs a method according to any one of claims 7-12.

14. A kit for performing immuno-PCR to detect and/or to quantify one or more target analytes, and/or to detect and/or to quantify a nucleic acid, said kit comprising:
a container containing a cartridge according to any one of claims 1-6.

15. The kit of claim 14, wherein said kit further comprises a container containing one or more reagents for preparing a sample for immuno-PCR.

## Patentansprüche

1. Kartusche zum Durchführen einer Immun-PCR zum Nachweisen und/oder Quantifizieren eines oder mehrerer Zielanalyten und/oder zum Nachweisen und/oder Quantifizieren einer Nukleinsäure, wobei die Kartusche Folgendes umfasst:
eine Probenaufnahmekammer;
eine Kammer, umfassend ein Matrixmaterial, das als ein Filter und/oder als ein DNA-Bindemittel wirkt;
eine(n) temperaturgesteuerte(n) Kanal oder Kammer; und
eine Vielzahl von Kammern, die Reagenzien zum Durchführen der Immun-PCR enthalten, wobei:
die Vielzahl von Kammern eine Kammer umfassen, die einen Fangantikörper enthält, der den nachzuweisenden Analyten bindet;
die Vielzahl von Kammern eine Kammer umfassen, die einen Nachweisantikörper enthält, wobei der Nachweisantikörper optional direkt oder indirekt an eine Signal-DNA angelagert ist;
die Vielzahl von Kammern eine Kammer umfassen, die einen PCR-Mastermix enthält;
die Vielzahl von Kammern eine Kammer umfassen, die Primer zum Amplifizieren der gesamten oder einer Region der Signal-DNA enthält; und
die Vielzahl von Kammern eine Kammer umfassen, die eine Sonde zum Nachweisen der gesamten oder einer Region der Signal-DNA enthält,
wobei die Probenaufnahmekammer, die Kammer, die das Matrixmaterial umfasst, der/die temperaturgesteuerte Kanal oder Kammer oder eine Öffnung in den/die temperaturgesteuerte(n) Kanal oder Kammer und die Vielzahl von Kammern um ein zentrales Ventil herum angeordnet sind und selektiv in Fluidkommunikation mit einem Kanal in dem zentralen Ventil stehen, wobei das zentrale Ventil dazu konfiguriert ist, einen Kolben unterzubringen, der Fluid in eine oder aus einer Kammer in Fluidkommunikation mit dem zentralen Ventil ziehen kann.

2. Kartusche nach Anspruch 1, wobei das zentrale Ventil einen Spritzenzylinder umfasst, der in Fluidverbindung mit einem Ventilkörper steht und der mit einer Dichtung abgedichtet ist, und wobei der Spritzenzylinder und der Ventilkörper dazu konfiguriert sind, sich zu drehen.

3. Kartusche nach den Ansprüchen 1-2, wobei die PCR-Primer und/oder Sonden und/oder die Polymerase im Mastermix als Kügelchen bereitgestellt sind; und/oder wobei die Kartusche Nachweisantikörper zum Nachweis eines einzelnen Analyten oder mehrerer Analyten enthält; und/oder wobei die Kartusche Fangantikörper zum Fangen eines einzelnen Analyten enthält.

4. Kartusche nach einem der Ansprüche 1-3, wobei der Fangantikörper an ein Teilchen angelagert ist; oder wobei der Fangantikörper an ein Teilchen angelagert ist und wobei das Teilchen eine Größe im Bereich von mindestens etwa 0,5 µm oder von etwa 1 µm bis zu etwa 10 µm oder bis zu etwa 3 µm aufweist, oder wobei das Teilchen eine Größe im Bereich von etwa 1 µm bis etwa 2,8 µm aufweist.

5. Kartusche nach einem der Ansprüche 1-4, wobei die Vielzahl von Kammern ferner eine Kammer umfassen, die PCR-Primer zum Amplifizieren einer anderen Nukleinsäure als der Signal-DNA enthält; und/oder wobei die mehreren Kammern ferner eine Kammer umfassen, die eine Sonde zum Nachweisen und/oder Quantifizieren einer anderen Nukleinsäure als der Signal-DNA enthält; und/oder wobei der Zielanalyt ein Polypeptid umfasst und eine andere Nukleinsäure als die Signal-DNA eine Nukleinsäure umfasst, die für das Polypeptid oder ein Fragment davon codiert; und/oder wobei der Zielanalyt ein Polypeptid umfasst und eine andere Nukleinsäure als die Signal-DNA eine Nukleinsäure umfasst, die charakteristisch für eine Zelle, ein Gewebe oder einen Organismus ist, die/das/der das Polypeptid produziert.

6. Kartusche nach einem der Ansprüche 1-5, wobei die Kartusche so konfiguriert ist, dass die Kartusche im Gebrauch Folgendes umfasst:
eine Kammer, die eine Probe enthält;
eine Kammer, die den Nachweisantikörper enthält;
eine Kammer, die den Fangantikörper enthält;
eine Kammer, die einen Waschpuffer enthält; und
eine Kammer, die einen PCR-Mastermix zum Amplifizieren der Signal-DNA enthält.

7. Verfahren zum Durchführen einer Immun-PCR zum Nachweisen und/oder Quantifizieren eines oder mehrerer Zielanalyten und/oder zum Nachweisen und/oder Quantifizieren einer Zielnukleinsäure, wobei das Verfahren Folgendes umfasst: Bereitstellen einer Probe in einer Probenaufnahmekammer einer Kartusche, die dazu konfiguriert ist, die Immun-PCR durchzuführen; und Verwenden der Kartusche in einem Immun-PCR-Verfahren, das Folgendes umfasst:
Inkontaktbringen des Analyten mit einem Fangantikörper unter Bedingungen, bei denen der Fangantikörper den Analyten bindet, wodurch ein Fangantikörper/Analyt-Komplex gebildet wird;
Inkontaktbringen des Analyten mit einem Nachweisantikörper, der an eine Signal-DNA angelagert ist, unter Bedingungen, bei denen der Nachweisantikörper spezifisch an den Fangantikörper/Analyt-Komplex bindet und einen Immunkomplex mit diesem bildet;
Freisetzen des Immunkomplexes oder eines Abschnitts davon, der die Signal-DNA umfasst, und Abgeben des Immunkomplexes oder eines Abschnitts davon in eine(n) temperaturgesteuerte(n) Kanal oder Kammer; und
Durchführen einer Nukleinsäureamplifikation in dem/der temperaturgeregelten Kanal oder Kammer, um die Signalnukleinsäure nachzuweisen und/oder zu quantifizieren, wodurch der Analyt nachgewiesen und/oder quantifiziert wird, wobei die Kartusche eine Kartusche nach einem der Ansprüche 1-6 ist.

8. Verfahren zum Durchführen einer Immun-PCR zum Nachweisen und/oder Quantifizieren eines oder mehrerer Zielanalyten und/oder zum Nachweisen und/oder Quantifizieren einer Nukleinsäure, wobei das Verfahren Folgendes umfasst: Bereitstellen einer Probe in einer Probenaufnahmekammer einer Kartusche, die dazu konfiguriert ist, die Immun-PCR durchzuführen; und Verwenden der Kartusche in einem Immun-PCR-Verfahren, das Folgendes umfasst:
Inkontaktbringen des Analyten mit einem Fangantikörper unter Bedingungen, bei denen der Fangantikörper den Analyten bindet, wodurch ein Fangantikörper/Analyt-Komplex gebildet wird;
Inkontaktbringen des Analyten mit einem Nachweisantikörper, wobei das Inkontaktbringen unter Bedingungen erfolgt, bei denen der Nachweisantikörper spezifisch an den Fangantikörper/Analyt-Komplex bindet und einen Immunkomplex mit diesem bildet;
Inkontaktbringen des Immunkomplexes mit einem markierten Antikörper, der an den Nachweisantikörper bindet, wobei der markierte Antikörper an eine Signal-DNA angelagert ist und das Inkontaktbringen unter Bedingungen erfolgt, bei denen der markierte Antikörper spezifisch an den Nachweisantikörper bindet, um einen markierten Immunkomplex zu bilden;
Freisetzen des markierten Immunkomplexes oder eines Abschnitts davon, der die Signal-DNA umfasst, und Abgeben des Immunkomplexes oder eines Abschnitts davon in eine(n) temperaturgesteuerte(n) Kanal oder Kammer; und
Durchführen einer Nukleinsäureamplifikation in dem/der temperaturgeregelten Kanal oder Kammer, um die Signalnukleinsäure nachzuweisen und/oder zu quantifizieren, wodurch der Analyt nachgewiesen und/oder quantifiziert wird, wobei die Kartusche eine Kartusche nach einem der Ansprüche 1-6 ist.

9. Verfahren nach einem der Ansprüche 7-8, wobei das Verfahren an mehreren unterschiedlichen Analyten in derselben Kartusche durchgeführt wird; und/oder wobei das Verfahren in etwa 1 Stunde oder weniger oder in etwa 50 Minuten oder weniger oder in etwa 40 Minuten oder weniger oder in etwa 30 Minuten oder weniger oder in etwa 20 Minuten oder weniger oder in etwa 15 Minuten oder weniger abgeschlossen werden kann.

10. Verfahren nach einem der Ansprüche 7-9, wobei
das Verfahren in einer Kartusche nach einem der Ansprüche 5-6 durchgeführt wird; und
das Verfahren ferner das Amplifizieren einer anderen Nukleinsäure als der Signal-DNA umfasst.

11. Verfahren nach Anspruch 10, wobei das Amplifizieren einer anderen Nukleinsäure als der Signal-DNA Folgendes umfasst:
Binden einer Nukleinsäure aus der Probe an ein Matrixmaterial;
Waschen der gebundenen Nukleinsäure, um eine gewaschene Nukleinsäure bereitzustellen;
Eluieren der gewaschenen Nukleinsäure; und
Unterziehen der gewaschenen Nukleinsäure einer Amplifikationsreaktion, um eine Zielnukleinsäuresequenz zu amplifizieren, falls sie in der gewaschenen Nukleinsäure vorhanden ist.

12. Verfahren nach einem der Ansprüche 10-11, wobei die andere Nukleinsäure als die Signal-DNA eine DNA oder eine RNA umfasst; und/oder wobei das Amplifizieren einer anderen Nukleinsäure als der Signal-DNA an (einer) Nukleinsäure(n) durchgeführt wird, die aus derselben Probe in der für die Immun-PCR verwendeten Probenkammer erhalten wurde(n), oder an (einer) Nukleinsäure(n), die aus einer Probe in einer anderen Probenkammer als der für die Immun-PCR verwendeten Probenkammer erhalten wurde(n); und/oder wobei die anderen Signal-DNAs und Nukleinsäure(n) als die Signal-DNA sequentiell in demselben/derselben temperaturgesteuerten Kanal oder Kammer amplifiziert werden.

13. System zum Durchführen einer Immun-PCR zum Nachweisen und/oder Quantifizieren eines oder mehrerer Zielanalyten und/oder zum Nachweisen und/oder zum Quantifizieren einer Nukleinsäure, wobei das System Folgendes umfasst:
ein Gehäuse, das dazu konfiguriert ist, ein oder mehrere Probenverarbeitungsmodule zu enthalten, wobei jedes Probenverarbeitungsmodul dazu konfiguriert ist, eine entfernbare Kartusche nach einem der Ansprüche 1-6 zu halten, wobei das System dazu konfiguriert ist, die Probenverarbeitungsmodule zu betreiben, um eine Immun-PCR zum Bestimmen des Vorhandenseins und/oder der Quantität eines oder mehrerer Zielanalyten und/oder zum Bestimmen des Spiegels einer oder mehrerer Ziel-DNA-Sequenzen in einer entsprechenden entfernbaren Probenkartusche durchzuführen, wobei die Verarbeitung an einer Probe in der entsprechenden entfernbaren Probenkartusche ein Verfahren nach einem der Ansprüche 7-12 durchführt.

14. Kit zum Durchführen einer Immun-PCR zum Nachweisen und/oder zum Quantifizieren eines oder mehrerer Zielanalyten und/oder zum Nachweisen und/oder zum Quantifizieren einer Nukleinsäure, wobei das Kit Folgendes umfasst:
einen Behälter, der eine Kartusche nach einem der Ansprüche 1-6 enthält.

15. Kit nach Anspruch 14, wobei das Kit ferner einen Behälter umfasst, der ein oder mehrere Reagenzien zum Herstellen einer Probe für die Immun-PCR enthält.

## Revendications

1. Cartouche permettant de réaliser une immuno-PCR pour détecter et/ou quantifier un ou plusieurs analytes cibles, et/ou détecter et/ou quantifier un acide nucléique, ladite cartouche comprenant :
une chambre de réception d'échantillon ;
une chambre comprenant un matériau matriciel qui agit comme un filtre et/ou un agent de liaison à un ADN ;
un canal ou une chambre à température régulée ; et
une pluralité de chambres contenant des réactifs pour réaliser une immuno-PCR :
ladite pluralité de chambres comprenant une chambre contenant un anticorps de capture qui se lie à l'analyte qui doit être détecté ;
ladite pluralité de chambres comprenant une chambre contenant un anticorps de détection où ledit anticorps de détection est éventuellement fixé directement ou indirectement à un ADN signal ;
ladite pluralité de chambres comprenant une chambre contenant un mélange maître pour PCR;
ladite pluralité de chambres comprenant une chambre contenant des amorces pour amplifier la totalité ou une région dudit ADN signal ; et
ladite pluralité de chambres comprenant une chambre contenant une sonde pour détecter la totalité ou une région dudit ADN signal,
ladite chambre de réception d'échantillon, ladite chambre comprenant un matériau matriciel, ledit canal ou ladite chambre de chauffage à température régulée ou un orifice dans ledit canal ou ladite chambre à température régulée, et ladite pluralité de chambres étant disposés autour d'une soupape centrale et de manière sélective en communication fluidique avec un canal dans ladite soupape centrale, ladite soupape centrale étant conçue pour recevoir un piston qui est capable d'aspirer un fluide dans ou hors d'une chambre en communication fluidique avec ladite soupape centrale.

2. Cartouche selon la revendication 1, ladite soupape centrale comprenant un cylindre de seringue en communication fluidique avec un corps de soupape et qui est scellé avec un joint, et ledit cylindre de seringue et ledit corps de soupape étant conçus pour tourner.

3. Cartouche selon les revendications 1 à 2, lesdites amorces de PCR et/ou sondes et/ou polymérase dans ledit mélange maître étant fournies sous la forme de billes ; et/ou ladite cartouche contenant des anticorps de détection pour la détection d'un seul analyte ou d'une pluralité d'analytes ; et/ou ladite cartouche contenant des anticorps de capture permettant la capture d'un seul analyte.

4. Cartouche selon l'une quelconque des revendications 1 à 3, ledit anticorps de capture étant fixé à une particule ; ou ledit anticorps de capture étant fixé à une particule et ladite particule présentant une taille allant d'au moins environ 0,5 µm, ou d'environ 1 µm jusqu'à environ 10 µm, ou jusqu'à environ 3 µm, ou ladite particule présentant une taille allant d'environ 1 µm jusqu'à environ 2,8 µm.

5. Cartouche selon l'une quelconque des revendications 1 à 4, ladite pluralité de chambres comprenant en outre une chambre contenant des amorces de PCR pour amplifier un acide nucléique différent dudit ADN signal ; et/ou ladite pluralité de chambres comprenant en outre une chambre contenant une sonde pour détecter et/ou quantifier un acide nucléique différent dudit ADN signal ; et/ou ledit analyte cible comprenant un polypeptide et un acide nucléique différent dudit ADN signal comprenant un acide nucléique codant ledit polypeptide ou un fragment de celui-ci ; et/ou ledit analyte cible comprenant un polypeptide et un acide nucléique différent dudit ADN signal comprenant un acide nucléique caractéristique d'une cellule, d'un tissu ou d'un organisme qui produit ledit polypeptide.

6. Cartouche selon l'une quelconque des revendication 1 à 5, ladite cartouche étant conçue de sorte que, lors de son utilisation, ladite cartouche comprend :
une chambre contenant un échantillon ;
une chambre contenant ledit anticorps de détection ;
une chambre contenant ledit anticorps de capture ;
une chambre contenant un tampon de lavage ; et
une chambre contenant un mélange maître pour PCR pour amplifier ledit ADN signal.

7. Procédé de réalisation d'une immuno-PCR pour détecter et/ou quantifier un ou plusieurs analytes cibles, et/ou détecter et/ou quantifier un acide nucléique cible, ledit procédé comprenant :
la fourniture d'un échantillon dans une chambre de réception d'échantillon d'une cartouche conçue pour réaliser une immuno-PCR ; et utiliser ladite cartouche dans un procédé d'immuno-PCR comprenant :
la mise en contact dudit analyte avec un anticorps de capture dans des conditions dans lesquelles ledit anticorps de capture se lie audit analyte, formant un complexe anticorps de capture/analyte ;
la mise en contact dudit analyte avec un anticorps de détection fixé à un ADN signal dans des conditions dans lesquelles ledit anticorps de détection se lie spécifiquement à et forme un immunocomplexe avec ledit complexe anticorps de capture/analyte ;
la libération dudit immunocomplexe ou d'une partie de celui-ci comprenant ledit ADN signal et l'administration dudit immunocomplexe ou d'une partie de celui-ci dans un canal ou une chambre à température régulée ; et
la réalisation d'une amplification d'acide nucléique dans ledit canal ou ladite chambre à température régulée pour détecter et/ou quantifier ledit signal d'acide nucléique, détectant et/ou quantifiant ainsi ledit analyte, ladite cartouche étant une cartouche selon l'une quelconque des revendications 1 à 6.

8. Procédé de réalisation d'une immuno-PCR pour détecter et/ou quantifier un ou plusieurs analytes cibles, et/ou détecter et/ou quantifier un acide nucléique, ledit procédé comprenant :
la fourniture d'un échantillon dans une chambre de réception d'échantillon d'une cartouche conçue pour réaliser une immuno-PCR ; et utiliser ladite cartouche dans un procédé d'immuno-PCR comprenant :
la mise en contact dudit analyte avec un anticorps de capture dans des conditions dans lesquelles ledit anticorps de capture se lie audit analyte, formant un complexe anticorps de capture/analyte ;
la mise en contact dudit analyte avec un anticorps de détection où ladite mise en contact se fait dans des conditions dans lesquelles ledit anticorps de détection se lie spécifiquement à et forme un immunocomplexe avec ledit complexe anticorps de capture/analyte ;
la mise en contact dudit immunocomplexe avec un anticorps marqué qui se lie audit anticorps de détection où ledit anticorps marqué est fixé à un ADN signal et ladite mise en contact se fait dans des conditions dans lesquelles ledit anticorps marqué se lie spécifiquement audit anticorps de détection pour former un immunocomplexe marqué ;
la libération dudit immunocomplexe marqué ou d'une partie de celui-ci comprenant ledit ADN signal et l'administration dudit immunocomplexe ou d'une partie de celui-ci dans un canal ou une chambre à température régulée ; et
la réalisation d'une amplification d'acide nucléique dans ledit canal ou ladite chambre à température régulée pour détecter et/ou quantifier ledit signal d'acide nucléique, détectant et/ou quantifiant ainsi ledit analyte, ladite cartouche étant une cartouche selon l'une quelconque des revendications 1 à 6.

9. Procédé selon l'une quelconque des revendications 7 à 8, ledit procédé étant réalisé sur une pluralité d'analytes différents dans la même cartouche ; et/ou ledit procédé pouvant être achevé en environ 1 heure ou moins, ou en environ 50 minutes ou moins, ou en environ 40 minutes ou moins, ou en environ 30 minutes ou moins, ou en environ 20 minutes ou moins, ou en environ 15 minutes ou moins.

10. Procédé selon l'une quelconque des revendications 7 à 9,
ledit procédé étant réalisé dans une cartouche selon l'une quelconque des revendications 5 à 6 ; et
ledit procédé comprenant en outre l'amplification d'un acide nucléique différent dudit ADN signal.

11. Procédé selon la revendication 10, ladite amplification d'un acide nucléique différent dudit ADN signal comprenant :
la liaison d'un acide nucléique dudit échantillon à un matériau matriciel ;
le lavage de l'acide nucléique lié pour fournir un acide nucléique lavé ;
l'élution de l'acide nucléique lavé ; et
la soumission dudit acide nucléique lavé à une réaction d'amplification pour amplifier une séquence d'acide nucléique cible si elle est présente dans ledit acide nucléique lavé.

12. Procédé selon l'une quelconque des revendications 10 à 11, ledit acide nucléique différent dudit ADN signal comprenant un ADN ou un ARN ; et/ou ladite amplification d'un acide nucléique différent dudit ADN signal étant effectuée sur un ou des acides nucléiques obtenus à partir du même échantillon dans la chambre d'échantillon utilisée pour ladite immuno-PCR ou sur un ou des acides nucléiques obtenus à partir d'un échantillon dans un chambre d'échantillon différente de la chambre d'échantillon utilisée pour ladite immuno-PCR ; et/ou lesdits ADN signal et le ou les acides nucléiques différents d'un ADN signal étant amplifiés de manière séquentielle dans le même canal ou la même chambre à température régulée.

13. Système permettant de réaliser une immuno-PCR pour détecter et/ou quantifier un ou plusieurs analytes cibles, et/ou pour détecter et/ou pour quantifier un acide nucléique, ledit système comprenant :
une enceinte conçue pour contenir un ou plusieurs modules de traitement d'échantillons, chaque module de traitement d'échantillons étant conçu pour contenir une cartouche amovible selon l'une quelconque des revendications 1 à 6, ledit système étant conçu pour faire fonctionner les modules de traitement d'échantillons pour réaliser une immuno-PCR afin de déterminer la présence et/ou la quantité d'un ou plusieurs analytes cibles et/ou pour déterminer le taux d'une ou plusieurs séquences d'ADN cibles au sein d'une cartouche d'échantillon amovible correspondante, ledit traitement sur un échantillon au sein de la cartouche d'échantillon amovible correspondante réalisant un procédé selon l'une quelconque des revendications 7 à 12.

14. Kit permettant de réaliser une immuno-PCR pour détecter et/ou pour quantifier un ou plusieurs analytes cibles, et/ou pour détecter et/ou pour quantifier un acide nucléique, ledit kit comprenant :
un récipient contenant une cartouche selon l'une quelconque des revendications 1 à 6.

15. Kit selon la revendication 14, ledit kit comprenant en outre un récipient contenant un ou plusieurs réactifs pour préparer un échantillon pour une immuno-PCR.
